# EUROPEAN PATENT APPLICATION

(11) **EP 4 310 099 A1**
(43) Date of publication of application: **24.01.2024**
(21) Application number: 22771559.6
(22) Date of filing: 18.03.2022
(51) Int. Cl.: C07K 16/12, A61K 38/10, A61K 39/00, A61K 39/085, A61K 39/395, A61P 1/16, A61P 7/00, A61P 11/00, A61P 13/12, A61P 37/04, A61P 43/00, C07K 7/06, C07K 7/08, C07K 16/40, C12N 15/13, C12N 15/31, C12P 21/08, G01N 33/53, G01N 33/68

(54) **PHARMACEUTICAL COMPOSITION FOR AMELIORATING ACUTE LUNG INJURY AND ACUTE WORSENING OF PULMONARY FIBROSIS**

(30) Priority: 19.03.2021 JP 2021046301; 22.10.2021 JP 2021173381
(71) Applicant: Mie University, Tsu-shi, Mie 514-8507 (JP); THE BOARD OF TRUSTEES OF THE UNIVERSITY OF ILLINOIS, Urbana, IL 61801 (US)
(72) Inventor: GABAZZA, Esteban, Tsu-shi, Mie 514-8507 (JP); GABAZZA, Corina, Tsu-shi, Mie 514-8507 (JP); CANN, Isaac, Urbana-champaign, Illinois 61801 (US)
(74) Representative: Mewburn Ellis LLP
(86) International application number: PCT/JP2022/012823
(87) International publication number: WO 2022/196816

(57) **Abstract**

Provided are a monoclonal antibody molecule or a derivative thereof against corisin and a pharmaceutical composition containing the same for ameliorating acute lung injury and acute exacerbation of pulmonary fibrosis. The pharmaceutical composition of the present invention contains a monoclonal antibody molecule or a derivative thereof against corisin, which is a peptide conserved in diverse Staphylococci, such as Staphylococcus nepalensis.

## Description

### [Technical Field]

The present patent application claims priority and benefits under the Paris Convention based on Japanese Patent Application No. 2021-046301 (filed on March 19, 2021) and Japanese Patent Application No. 2021-173381 (filed on October 22, 2021), and the entire contents of the foregoing applications are incorporated in the present specification by reference.

The present invention relates to a pharmaceutical composition for ameliorating acute exacerbation of pulmonary fibrosis and a method therefor, and more specifically relates to a pharmaceutical composition for ameliorating acute exacerbation of pulmonary fibrosis and a method therefor, the pharmaceutical composition containing an inhibitor of a peptide derived from the lung microbiome.

### [Background Art]

Idiopathic pulmonary fibrosis (IPF) is a chronic and incurable disease of unknown etiology. Increased apoptosis of alveolar epithelial cells and subsequent aberrant lung tissue repair are central to the progression of IPF. When on two types of antifibrotic drugs, pirfenidone and nintedanib, which are currently used in the treatment of IPF, patients have a life expectancy of only about two to three years after diagnosis (Non-Patent Literature 1). These antifibrotic drugs only slightly reduce loss of lung function and do not improve patient survival or quality of life (Non-Patent Literature 2 to 4). Recent epidemiological studies suggest that globally there are more than five million IPF patients and that the number of cases is further growing worldwide (Non-Patent Literature 5). Damage to alveolar epithelial cells and aberrant tissue repair caused by increased secretion of pulmonary fibrogenic factors such as transforming growth factor-β1 (Transforming Growth Factor-β1) and excessive recruitment of myofibroblasts and deposition of extracellular matrix in the lung play central roles in the disease pathogenesis of IPF (Non-Patent Literature 1). This progression ultimately culminates in lung tissue scarring, lung structural destruction, and respiratory failure (Non-Patent Literature 5). The natural history of IPF is unpredictable and variable (Non-Patent Literature 6), and the disease may progress slowly, rapidly, or with sudden clinical deterioration referred to as acute exacerbation (AE) (Non-Patent Literature 7, 8). The most frequent cause of death in IPF is AE, occurring in approximately 46% of cases (Non-Patent Literature 9, 10). AE predicts high mortality (of up to 50%) during onset and a short-term survival rate after acute onset; patients live only three to four months after the diagnosis of AE (Non-Patent Literature 8, 9). Mortality increases by up to 90% in cases requiring mechanical ventilation (Non-Patent Literature 9). Infection and diagnostic or surgical procedures can induce AE, although the precise mechanism is unknown (Non-Patent Literature 8). No effective therapy is currently available for AE (Non-Patent Literature 3).

A growing amount of evidence suggests a causative role of the intrapulmonary microbiome (lung microbiome) in idiopathic pulmonary fibrosis (IPF). Dysbiotic or abundant lung microbiome is associated with persistent altered expression of genes involved in host defense, decreased immune response, lung epithelial cell damage, abnormal fibroblast responsiveness, the severity of lung function abnormality, deterioration of chest radiograph findings, disease progression, and decreased survivability in IPF patients (Non-Patent Literature 11 to 16). Alteration and bacterial burden of the lung microbiome become even worse in IPF accompanied by acute exacerbation (Non-Patent Literature 17, 18). However, the mechanisms associated with the lung microbiome accompanied by pulmonary fibrosis remain unclear. The inventors of the present invention recently identified a pro-apoptotic peptide termed corisin in the lung microbiome that may explain the pathogenic role in pulmonary fibrosis (Non-Patent Literature 19). Corisin is a peptide conserved in diverse Staphylococci and strains of different pathogenic bacteria (Non-Patent Literature 19).

The amino acid sequence of corisin corresponds to a bacterial transglycosylase fragment (Non-Patent Literature 19). Intrapulmonary administration of corisin or corisin-harboring bacterium causes apoptosis of alveolar epithelial cells and acute exacerbation (AE) in mice having established pulmonary fibrosis (Non-Patent Literature 19). Also, IPF patients with slowly progressive disease exhibit increased lung levels of corisin compared to a healthy population, and patients with AE show strikingly much-elevated lung corisin levels compared to stable patients (Non-Patent Literature 19).

Therefore, the inventors of the present invention hypothesized that treatment using an antibody against corisin may directly inhibit the AE of pulmonary fibrosis (AE-IPF) by blocking the apoptotic activity of corisin. They conducted various experiments, developed an antibody against corisin, and proved that it may be utilized for Western blotting of corisin in lung tissue (Patent Literature 1).

### [Citation List]

### [Patent Literature]

[Patent Literature 1] WO2021/126957

### [Non-Patent Literature]

[Non-Patent Literature 1] 1 King, T. E., Jr., Pardo, A. & Selman, M. Idiopathic pulmonary fibrosis. Lancet 378, 1949-1961, doi: 10.1016/S0140-6736 (I 1)60052-4 (2011).
[Non-Patent Literature 2] 2 Richeldi, L. et al. Efficacy and safety of nintedanib in idiopathic pulmonary fibrosis. N Engl J Med370, 2071-2082, doi: 10.1056/NEJMoal402584 (2014).
[Non-Patent Literature 3] King, T. E., Jr., Noble, P. W. & Bradford, W. Z. Treatments for idiopathic pulmonary fibrosis. N Engl J Med 371, 783-784, doi: 10.1056/NEJMcl407776 (2014).
[Non-Patent Literature 4] King, T. E., Jr. et al. A phase 3 trial of pirfenidone in patients with idiopathic pulmonary fibrosis. N Engl J Med 370, 2083-2092, doi: 10.1056/NEJMoal402582 (2014).
[Non-Patent Literature 5] 5 Lederer, D. J. & Martinez, F. J. Idiopathic Pulmonary Fibrosis. N Engl J Med 378, 1811-1823, doi: 10.1056/NEJMral705751 (2018).
[Non-Patent Literature 6] 6 Sgalla, G., Biffi, A. & Richeldi, L. Idiopathic pulmonary fibrosis: Diagnosis, epidemiology and natural history. Respirology 21, 427-437, doi: 10.1111/resp.12683 (2016).
[Non-Patent Literature 7] Kreuter, M. et al. Acute exacerbation of idiopathic pulmonary fibrosis: international survey and call for harmonisation. Eur Respir J 55, doi: 10.1183/13993003.01760-2019(2020).
[Non-Patent Literature 8] Collard, H. R. et al. Acute Exacerbation of Idiopathic Pulmonary Fibrosis. An International Working Group Report. Am J Respir Crit Care Med 194, 265-275, doi: 10.1164/rccm.201604-0801CI (2016).
[Non-Patent Literature 9] Natsuizaka, M. et al. Epidemiologic survey of Japanese patients with idiopathic pulmonary fibrosis and investigation of ethnic differences. Am J Respir Crit Care Med 190, 773-779, doi: 10.1164/rccm.201403-05660C (2014).
[Non-Patent Literature 10] Ley, B., Collard, H. R. & King, T. E., Jr. Clinical course and prediction of survival in idiopathic pulmonary fibrosis. Am J Respir Crit Care Med 183, 431-440, doi: 10.1164/rccm.201006-0894CI (2011).
[Non-Patent Literature 11] Dickson, R. P. et al. Radiographic Honeycombing and Altered Lung Microbiota in Patients with Idiopathic Pulmonary Fibrosis. Am J Respir Crit Care Med200, 1544-1547, doi: 10.1164/rccm.201903-0680LE (2019).
[Non-Patent Literature 12] Han, M. K. et al. Lung microbiome and disease progression in idiopathic pulmonary fibrosis: an analysis of the COMET study. Lancet Respir Med 2, 548-556, doi: 10.1016/S2213-2600(14)70069-4 (2014).
[Non-Patent Literature 13] Huang, Y. et al. Microbes Are Associated with Host Innate Immune Response in Idiopathic Pulmonary Fibrosis. Am J Respir Crit Care Med 196, 208-219, doi: 10.1164/rccm.201607-15250C (2017).
[Non-Patent Literature 14] Molyneaux, P. L. et al. Host-Microbial Interactions in Idiopathic Pulmonary Fibrosis. Am J Respir Crit Care Med 195, 1640-1650, doi:10.1164/rccm.201607-14080C (2017).
[Non-Patent Literature 15] Takahashi, Y. et al. Impaired diversity of the lung microbiome predicts progression of idiopathic pulmonary fibrosis. Respir Resl9, 34, doi:10.1186/s1293 1-018-0736-9 (2018).
[Non-Patent Literature 16] Wang, J. et al. Lung microbiome and host immune tone in subjects with idiopathic pulmonary fibrosis treated with inhaled interferon-gamma. ERJ Open Res 3, doi: 10.1183/23120541.00008-2017 (2017).
[Non-Patent Literature 17] Molyneaux, P. L. et al. Changes in the respiratory microbiome during acute exacerbations of idiopathic pulmonary fibrosis. Respir Res 18, 29, doi: 10.1186/s12931-017-0511-3 (2017).
[Non-Patent Literature 18] Weng, D. et al. The Role of Infection in Acute Exacerbation of Idiopathic Pulmonary Fibrosis. Mediators Inflamm 2019, 5160694, doi: 10.1155/2019/5160694 (2019).
[Non-Patent Literature 19] D'Alessandro-Gabazza, C. N. et al. A Staphylococcus pro-apoptotic peptide induces acute exacerbation of pulmonary fibrosis. Nat Commun 11, 1539, doi: 10.1038/s41467-020-15344-3 (2020).

### Summary of Invention]

### [Problem to Be Solved by the Invention]

In a further study, the inventors of the present invention demonstrated the presence and apoptotic activities of corisin-like peptides derived from known pathogenic bacteria other than Staphylococcus nepalensis (Staphylococcus nepalensis) strain CNDG, thus suggesting a wider distribution of this toxic peptide. In addition, several monoclonal antibodies were developed and characterized to identify monoclonal antibody A21, which has the capacity to neutralize the toxic activity of corisin and related peptides.

### [Means for Solving the Problem]

By harnessing this knowledge, the inventors tested the efficacy of the neutralizing monoclonal antibody on two different pulmonary fibrosis models and demonstrated that this antibody ameliorates pulmonary fibrosis by significantly inhibiting acute exacerbation in a human transforming growth factor-β1 transgenic mouse model and acute lung injury in a bleomycin model, thereby completing the present invention.

Namely, the present invention includes the following aspects.

### <Monoclonal antibody molecule>

[1] An isolated monoclonal antibody molecule or a derivative thereof that specifically binds to corisin.
[2] The monoclonal antibody molecule or a derivative thereof according to [1], wherein corisin is IVMPESSGNPNAVNPAGYR (SEQ ID NO: 1).
[3] The monoclonal antibody molecule or a derivative thereof according to [1] or [2], wherein PESSGNP (SEQ ID NO: 68) or NPAGY (SEQ ID NO: 69) is an epitope.

### <Identification of monoclonal antibody molecule by CDR: Series A>

The monoclonal antibody molecule or a derivative thereof according to [1] or [2], having:
(IA)
   (i) an H chain variable region including an H chain variable region CDR1 amino acid sequence of SEQ ID NO: 37, an H chain variable region CDR2 amino acid sequence of SEQ ID NO: 38, and an H chain variable region CDR3 amino acid sequence of SEQ ID NO: 39; and
   (ii) an L chain variable region including an L chain variable region CDR1 amino acid sequence of SEQ ID NO: 41, an L chain variable region CDR2 amino acid sequence of SEQ ID NO: 42, and an L chain variable region CDR3 amino acid sequence of SEQ ID NO: 43;
(IIA)
   (i) an H chain variable region including an H chain variable region CDR1 amino acid sequence of SEQ ID NO: 70, an H chain variable region CDR2 amino acid sequence of SEQ ID NO: 49, and an H chain variable region CDR3 amino acid sequence of SEQ ID NO: 50; and
   (ii) an L chain variable region including an L chain variable region CDR1 amino acid sequence of SEQ ID NO: 52, an L chain variable region CDR2 amino acid sequence of SEQ ID NO: 53, and an L chain variable region CDR3 amino acid sequence of SEQ ID NO: 54;
Or
(IVA)
   (i) an H chain variable region including an H chain variable region CDR1 amino acid sequence of SEQ ID NO: 72, an H chain variable region CDR2 amino acid sequence of SEQ ID NO: 73, and an H chain variable region CDR3 amino acid sequence of SEQ ID NO: 74; and
   (ii) an L chain variable region including an L chain variable region CDR1 amino acid sequence of SEQ ID NO: 76, an L chain variable region CDR2 amino acid sequence of SEQ ID NO: 77, and an L chain variable region CDR3 amino acid sequence of SEQ ID NO: 78.

### <Identification of monoclonal antibody molecule by H chain variable region and mutant type: Series B>

The monoclonal antibody molecule or a derivative thereof according to any one of [1] to [3], having:
(IB) an H chain variable region including an H chain variable region amino acid sequence of SEQ ID NO: 36 or an amino acid sequence that is at least 80% identical thereto, or an H chain variable region including at least one framework (FW) region including an amino acid sequence having a substitution, deletion, insertion, or addition of one or several amino acid residues in comparison to the H chain variable region amino acid sequence of SEQ ID NO: 36;
(IIB) an H chain variable region including an H chain variable region amino acid sequence of SEQ ID NO: 48 or an amino acid sequence that is at least 80% identical thereto, or an H chain variable region including at least one framework (FW) region including an amino acid sequence having a substitution, deletion, insertion, or addition of one or several amino acid residues in comparison to the H chain variable region amino acid sequence of SEQ ID NO: 48; or
(IVB) an H chain variable region including an H chain variable region amino acid sequence of SEQ ID NO: 71 or an amino acid sequence that is at least 80% identical thereto, or an H chain variable region including at least one framework (FW) region including an amino acid sequence having a substitution, deletion, insertion, or addition of one or several amino acid residues in comparison to the H chain variable region amino acid sequence of SEQ ID NO: 71.

### <Identification of monoclonal antibody molecule by L chain variable region and mutant type: Series C>

The monoclonal antibody molecule or a derivative thereof according to any one of [1] to [3], having:
(IC) an L chain variable region including an L chain variable region amino acid sequence of SEQ ID NO: 40 or an amino acid sequence that is at least 80% identical thereto, or an L chain variable region including at least one framework region including an amino acid sequence having a substitution, deletion, insertion, or addition of one or several amino acid residues in comparison to the L chain variable region amino acid sequence of SEQ ID NO: 40;
(IIC) an L chain variable region including an L chain variable region amino acid sequence of SEQ ID NO: 51 or an amino acid sequence that is at least 80% identical thereto, or an L chain variable region including at least one framework region including an amino acid sequence having a substitution, deletion, insertion, or addition of one or several amino acid residues in comparison to the L chain variable region amino acid sequence of SEQ ID NO: 51;
(IIIC) an L chain variable region including an L chain variable region amino acid sequence of SEQ ID NO: 44 or an amino acid sequence that is at least 80% identical thereto, or an L chain variable region including at least one framework region including an amino acid sequence having a substitution, deletion, insertion, or addition of one or several amino acid residues in comparison to the L chain variable region amino acid sequence of SEQ ID NO: 44; or
(IVC) an L chain variable region including an L chain variable region amino acid sequence of SEQ ID NO: 75 or an amino acid sequence that is at least 80% identical thereto, or an L chain variable region including at least one framework region including an amino acid sequence having a substitution, deletion, insertion, or addition of one or several amino acid residues in comparison to the L chain variable region amino acid sequence of SEQ ID NO: 75.

The monoclonal antibody molecule or a derivative thereof according to any one of [1] to [3], having any H chain variable region according to [5] and any L chain variable region according to [6].

### <Identification of combination of H chain and L chain variable regions in monoclonal antibody molecule>

The monoclonal antibody molecule or a derivative thereof according to [7], having:
the H chain variable region of (IB) according to [5] and the L chain variable region of (IC) according to [6];
the H chain variable region of (IIB) according to [5] and the L chain variable region of (IIC) according to [6]; or the H chain variable region of (IVB) according to [5] and the L chain variable region of (IVC) according to [6].

### <Identification of monoclonal antibody molecule by H chain and L chain variable regions>

The monoclonal antibody molecule or a derivative thereof according to [8], wherein:
the H chain variable region of (IB) according to [5] is the H chain variable region amino acid sequence of SEQ ID NO: 36, and the L chain variable region of (IC) according to [6] is the L chain variable region amino acid sequence of SEQ ID NO: 40; or
the H chain variable region of (IIB) according to [5] is the H chain variable region amino acid sequence of SEQ ID NO: 48, and the L chain variable region of (IIC) according to [6] is the L chain variable region amino acid sequence of SEQ ID NO: 51; or
the H chain variable region of (IVB) according to [5] is the H chain variable region amino acid sequence of SEQ ID NO: 71, and the L chain variable region of (IVC) according to [6] is the L chain variable region amino acid sequence of SEQ ID NO: 75.

### <Monoclonal antibody molecule mutant type 1>

The monoclonal antibody molecule or a derivative thereof according to [8], wherein: the H chain variable region of (IB) according to [5] is an H chain variable region including an amino acid sequence that is at least 80% identical to the H chain variable region amino acid sequence of SEQ ID NO: 36, and the L chain variable region of (IC) according to [6] is an L chain variable region including an amino acid sequence that is at least 80% identical to the L chain variable region amino acid sequence of SEQ ID NO: 40; or
the H chain variable region of (IIB) according to [5] is an H chain variable region including an amino acid sequence that is at least 80% identical to the H chain variable region amino acid sequence of SEQ ID NO: 48, and the L chain variable region of (IIC) according to [6] is an L chain variable region including an amino acid sequence that is at least 80% identical to the L chain variable region amino acid sequence of SEQ ID NO: 51; or
the H chain variable region of (IVB) according to [5] is an H chain variable region including an amino acid sequence that is at least 80% identical to the H chain variable region amino acid sequence of SEQ ID NO: 71, and the L chain variable region of (IVC) according to [6] is an H chain variable region including an amino acid sequence that is at least 80% identical to the H chain variable region amino acid sequence of SEQ ID NO: 75.

### <Monoclonal antibody molecule mutant type 2>

The monoclonal antibody molecule or a derivative thereof according to [8], wherein: the H chain variable region of (IB) according to [5] is an H chain variable region including at least one framework (FW) region including an amino acid sequence having a substitution, deletion, insertion, or addition of one or several amino acid residues in comparison to the H chain variable region amino acid sequence of SEQ ID NO: 36, and the L chain variable region of (IC) according to [6] is an L chain variable region including at least one framework (FW) region including an amino acid sequence having a substitution, deletion, insertion, or addition of one or several amino acid residues in comparison to the L chain variable region amino acid sequence of SEQ ID NO: 40; or
the H chain variable region of (IIB) according to [5] is an H chain variable region including at least one framework (FW) region including an amino acid sequence having a substitution, deletion, insertion, or addition of one or several amino acid residues in comparison to the H chain variable region amino acid sequence of SEQ ID NO: 48, and the L chain variable region of (IIC) according to [6] is an L chain variable region including at least one framework (FW) region including an amino acid sequence having a substitution, deletion, insertion, or addition of one or several amino acid residues in comparison to the L chain variable region amino acid sequence of SEQ ID NO: 51; or
the H chain variable region of (IVB) according to [5] is an H chain variable region including at least one framework (FW) region including an amino acid sequence having a substitution, deletion, insertion, or addition of one or several amino acid residues in comparison to the H chain variable region amino acid sequence of SEQ ID NO: 71, and the L chain variable region of (IVC) according to [6] is an L chain variable region including at least one framework (FW) region including an amino acid sequence having a substitution, deletion, insertion, or addition of one or several amino acid residues in comparison to the L chain variable region amino acid sequence of SEQ ID NO: 75.

A derivative of the antibody molecule according to any of [1] to [11], wherein the derivative is Fab, F(ab')₂, Fv, or a single chain Fv fragment (scFv).

The antibody molecule or a derivative thereof according to any one of [1] to [12], wherein the antibody molecule or a derivative thereof neutralizes and/or inhibits corisin-induced apoptosis.

### <Pharmaceutical composition>

A pharmaceutical composition for treating or preventing acute lung injury or fibrosis, wherein the pharmaceutical composition contains an effective dose of the monoclonal antibody molecule or a derivative thereof according to any of [1] to [13].

The pharmaceutical composition according to [14], wherein the acute lung injury is fatal pneumonia that causes acute respiratory distress syndrome (ARDS), reduces lung function, and requires respiratory care.

The pharmaceutical composition according to [14], wherein the fibrosis is selected from a group composed of idiopathic pulmonary fibrosis (IPF), liver cirrhosis, renal fibrosis, cystic fibrosis, myelofibrosis, and fibrous disease of the breast.

### <Vaccine>

A vaccine composition for preventing acute lung injury or fibrosis, having as an active ingredient a corisin mutant having one or several mutations in the sequence of corisin having the amino acid sequence: IVMPESSGNPNAVNPAGYR (SEQ ID NO: 1), wherein the mutant does not exhibit apoptotic activity.

### <Biomarker>

Use of corisin as a biomarker to determine the stage of progression of acute lung injury or fibrosis.

### [Effect of the Invention]

The inventors of the present application further investigated the impact of the anticorisin monoclonal antibody in a general model of acute lung injury, and obtained results on the potential of corisin to impact such diseases. The present invention further elucidates the role of corisin in the pathogenesis and progression of pulmonary fibrosis and possibly other acute lung injury-related pulmonary diseases, and is believed to provide a novel approach to treating this incurable disease; namely, a new medicine, therapeutic method, and the like.

### [Brief Description of Drawings]

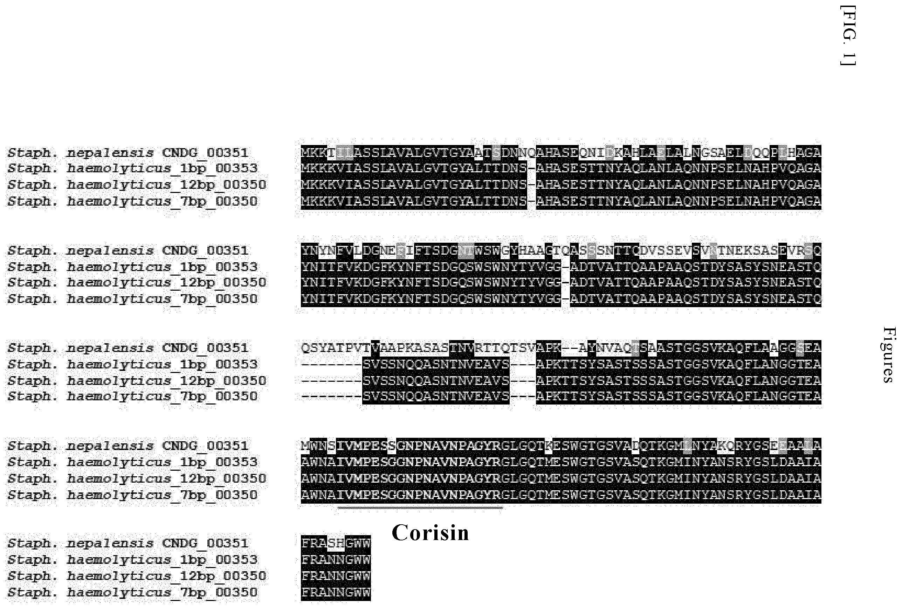

[FIG. 1] FIG. 1 is an alignment showing a putative transglycosylase containing the corisin peptide, derived from Staphylococcus nepalensis (Staphylococcus nepalensis) strain CNDG (CNDG), and the homology of three strains of Staphylococcus haemolyticus isolated from the same lung fiber tissue of mice. The corisin peptide is shown with a bar in the fourth column. Outside the corisin peptide sequence, conserved amino acids are shaded in black and similar amino acids are shaded in gray. Staphylococcus haemolyticus is Staphylococcus haemolyticus, and 1bp_00353, 12bp_00350, and 7bp_00350 are S. haemolyticus strain 1 protein number 353, S. haemolyticus strain 12 protein number 350, and S. haemolyticus strain 7 protein number 350, respectively. The protein from S. nepalensis strain CNDG is encoded at position 351 of the open reading frame (ORF) in the genome, counting from the gene encoding DnaA (ORF00001). Amino acids having similar properties are grouped into LIMV, AG, YWF, DEQN, KRH, and ST. The alignment was performed using multiple sequence comparisons by Log-Expectation (MUSCLE (https://www.ebi.ac.uk/Tools/msa/muscle/)).

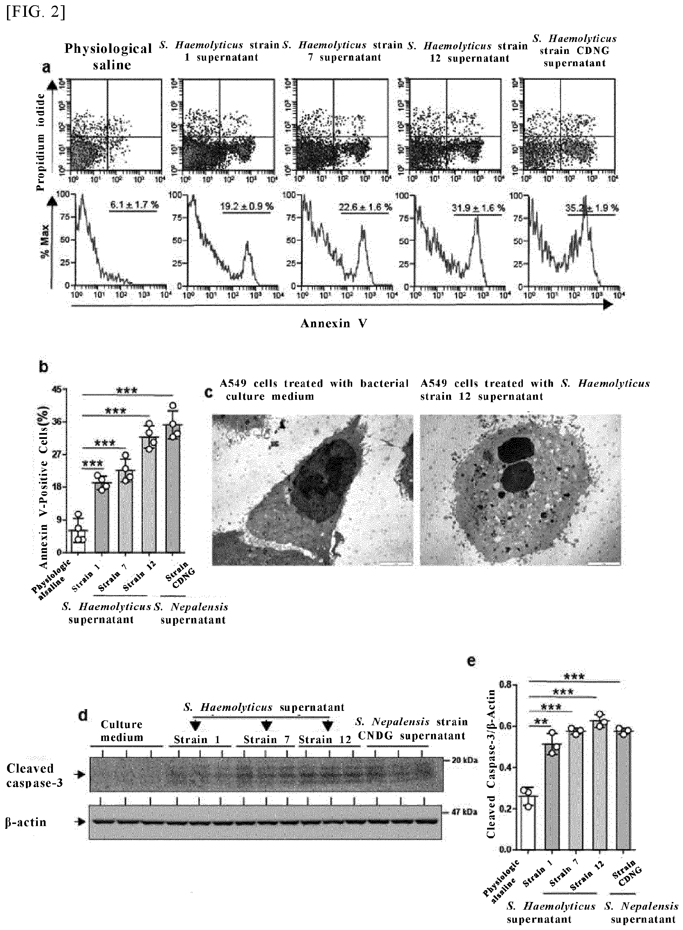

[FIG. 2] FIG. 2 shows that each culture supernatant from the three strains of Staphylococcus haemolyticus induces apoptosis of alveolar epithelial cells. FIG. 2a: Shows flow cytometry results of A549 alveolar epithelial cells when cultured for 48 hours in the presence of a culture supernatant (1/10 dilution) from Staphylococcus haemolyticus strains 1, 7, and 12. Fluorescein-labeled annexin V and propidium iodide staining are used as indicators. A549 cells treated with the culture supernatant of Staphylococcus nepalensis strain CNDG served as a positive control, and cells treated with physiological saline served as a negative control. FIG. 2b: Graph showing the percentage of apoptotic cells determined and quantified by flow cytometry. N = 4 in each group. Data are expressed as mean±SD. Statistical analysis was performed using Student's t-test. ***p <0.0001. FIG. 2c: Shows the results of A549 cells being cultured for 48 hours in the presence of the culture supernatant of S. haemolyticus and evaluated using a transmission electron microscope. FIG. 2c: Shows the results of A549 cells being cultured for 48 hours in the presence of the culture supernatant of S. haemolyticus and evaluated using a transmission electron microscope. FIG. 2e: A graph showing the results of caspase-3 cleavage in A549 cells cultured in the presence of the culture supernatant of the three S. haemolyticus strains 1, 7, and 12 and S. nepalensis. Data are expressed as mean±SD. Statistical analysis was performed using an unpaired t-test. **p <0.001, ***p <0.0001.

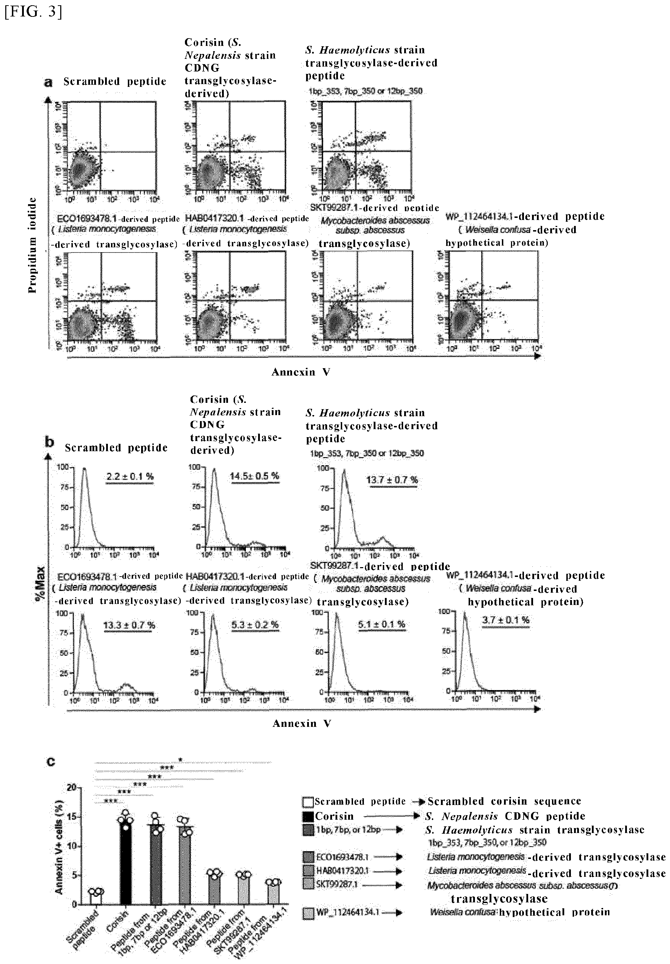

[FIG. 3] FIG. 3 shows that synthetic corisin-like peptides of transglycosylases from several pathogens induce apoptosis in alveolar epithelial cells. FIGS. 3a, b: Flow cytometry examination results showing the results of evaluating apoptosis after culturing A549 alveolar epithelial cells in the presence of a 10 µM corisin-like peptide. A549 cells treated with a synthetic corisin sequence or a scrambled peptide served as positive and negative controls, respectively. FIG. 3b, c: Graph showing the percentage of apoptotic cells determined and quantified by flow cytometry. N = 4 in each group. Data are expressed as mean±SD. Statistical analysis was performed using Student's t-test. *p <0.05; ***p <0.0001.

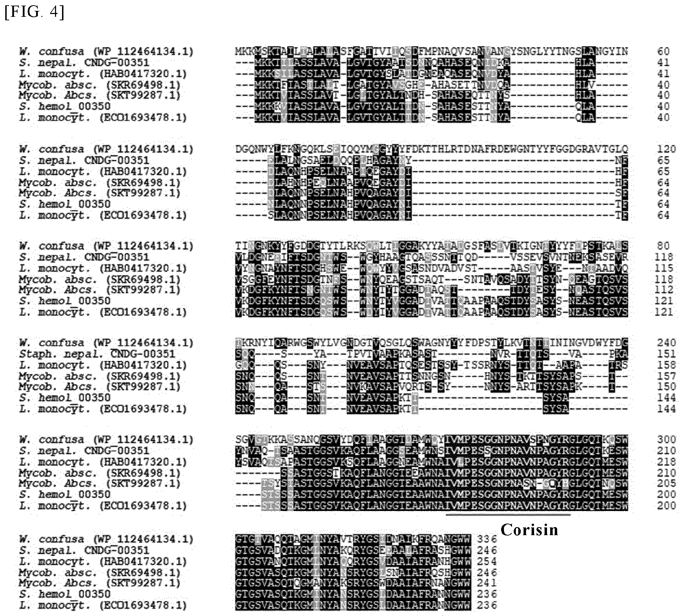

[FIG. 4] FIG. 4 illustrates an alignment of corisin-containing putative transglycosylases from various bacterial pathogens. Organism names are abbreviated as Wconf (Weissella confusa, GenBank protein accession no. WP_112464134.1), CNDG-00351 (S. nepalensis strain CNDG protein 00351), L. monocyt-2 (Listeria monocytogenes (Listeria monocytogenes), GenBank protein accession no. HAB0417320.1), Myabs-2 (Mycobacteroides abscessus (Mycobacteroides abscessus), GenBank protein accession no. SKR69498.1), Myabs-1 (Mycobacteroides abscessus, GenBank protein accession no. SKT99287.1), Shem_00350 (Staphylococcus hemolyticus 12b protein 00350), and Lmonocyt-1 (L. monocytogenes-GenBank protein accession no. ECO1693478.1). The corisin peptide is indicated in bold, and the portion where the amino acids are conserved is labeled as corisin (fifth section). Outside the corisin peptide sequence, conserved amino acids are shaded in black and similar amino acids are shaded in gray. Amino acids having similar characteristics are grouped as LIMV, AG, YWF, DEQN, KRH, and ST. S, Staphylococci. The alignment was performed using multiple sequence comparisons by Log-Expectation (MUSCLE (https://www.ebi.ac.uk/Tools/msa/muscle/)).

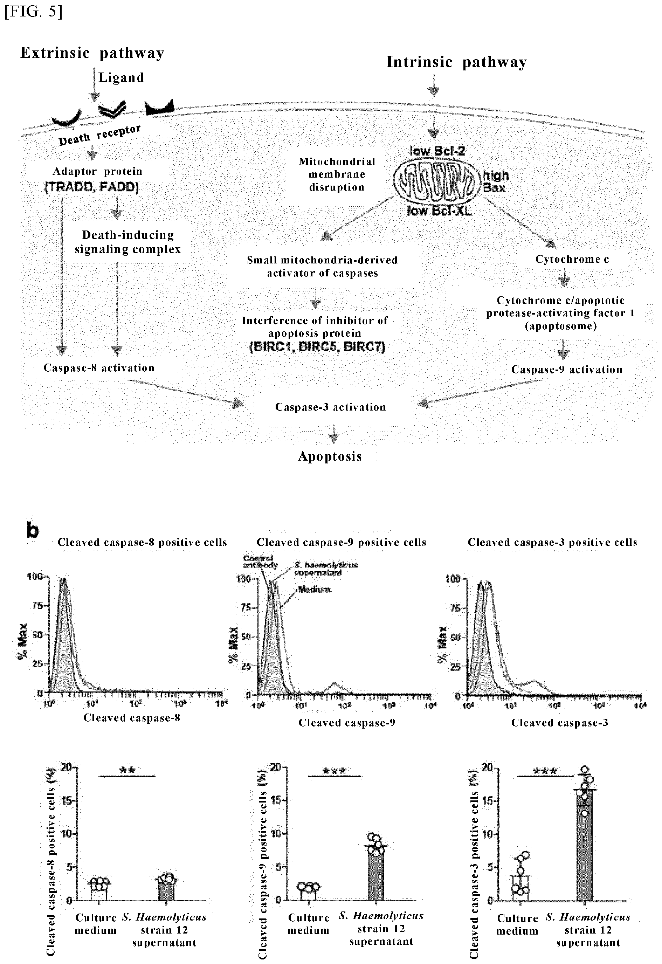

[FIG. 5] FIG. 5 illustrates the apoptotic pathways induced by S. haemolyticus culture supernatant. FIG. 5a: Apoptosis occurs by extrinsic and intrinsic pathways. The extrinsic pathway is initiated by ligands binding to membrane-bound death receptors, resulting in activation of intracellular signaling and cleavage of procaspase-8 to caspase-8. The intrinsic pathway is regulated by mitochondria. In the presence of stimuli, perturbation of the mitochondrial membrane increases its permeability and causes the release of suppressors of baculoviral inhibitor of apoptosis repeat-containing (BIRC) proteins and pro-apoptotic factors, and this contributes to apoptosome formation cleaving procaspase-9 into caspase-9. Both caspase-8 and caspase-9 can activate the apoptotic effector caspase-3. FIG. 5b: A549 alveolar epithelial cells were cultured for 24 hours in the presence of the culture supernatant (1/10 dilution) of S. haemolyticus strain 12 and the results of flow cytometry analysis of the percentage of cells positive for cleaved caspase-8, cleaved caspase-9, and cleaved caspase-3 are shown. The bottom part includes graphs showing the percentage of positive cells compared to the control. N = 6 in each group. Data are expressed as mean±SD. Statistical analysis was performed using Student's t-test. **p <0.01; ***p <0.0001.

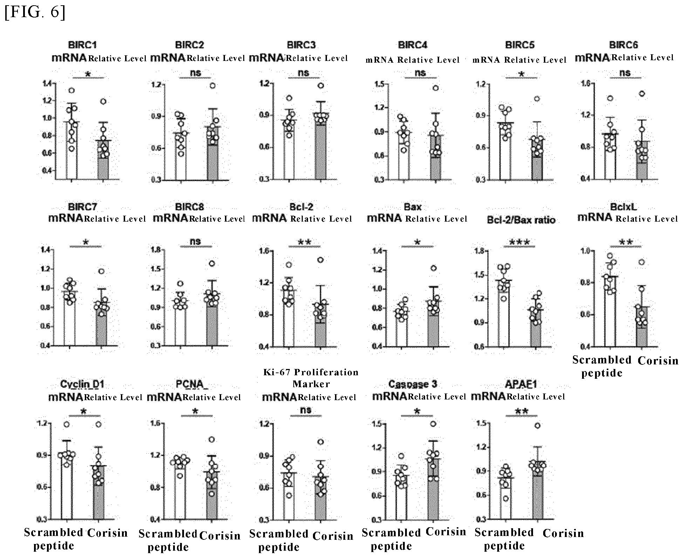

[FIG. 6] FIG. 6 includes graphs showing that corisin alters the mRNA expression of pro-apoptotic factors and anti-apoptotic factors. N = 6 in each group. Data are expressed as mean±SD. Statistical analysis was performed using an unpaired t-test. *p <0.05; **p <0.01; ***p <0.001.

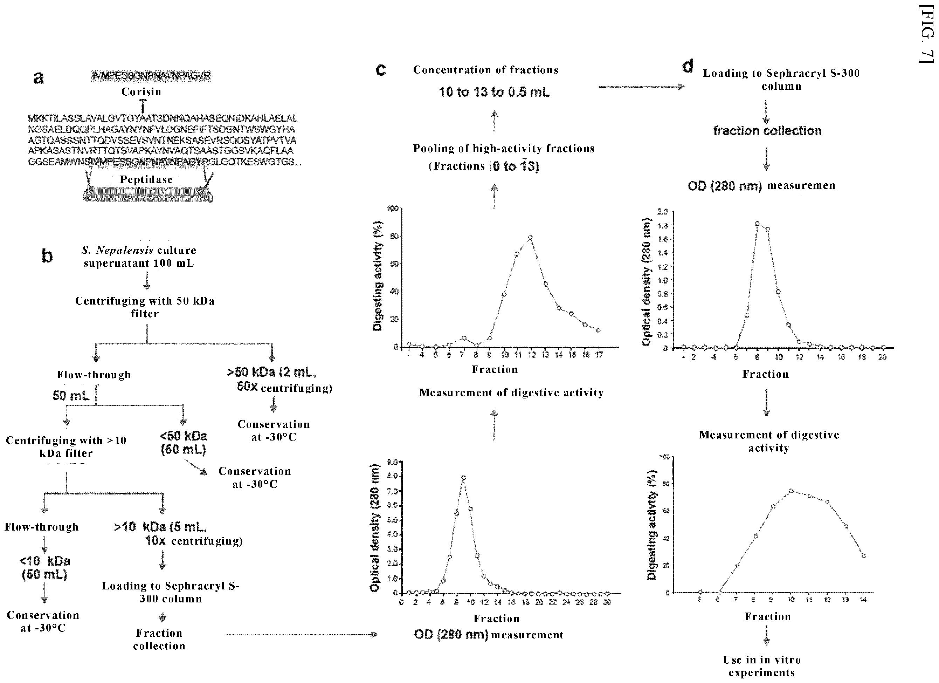

[FIG. 7] FIG. 7 shows the degradation of corisin-containing transglycosylase by the culture supernatant of Staphylococcus nepalensis strain CNDG and preparation of peptidase fractions. FIG. 7a: Shows the amino acid sequences of the transglycosylase and corisin moieties of Staphylococcus nepalensis strain CNDG. FIG. 7b: Illustrates the procedure for concentrating the Staphylococcus nepalensis culture supernatant into defined fractions. FIG. 7c: Illustrates the data and procedure for obtaining fractions having high digestive activity. FIG. 7d: Illustrates the data and procedure for obtaining fractions having high digestive activity against recombinant transglycosylase 351 for fractions with high absorbance at OD (280 nm).

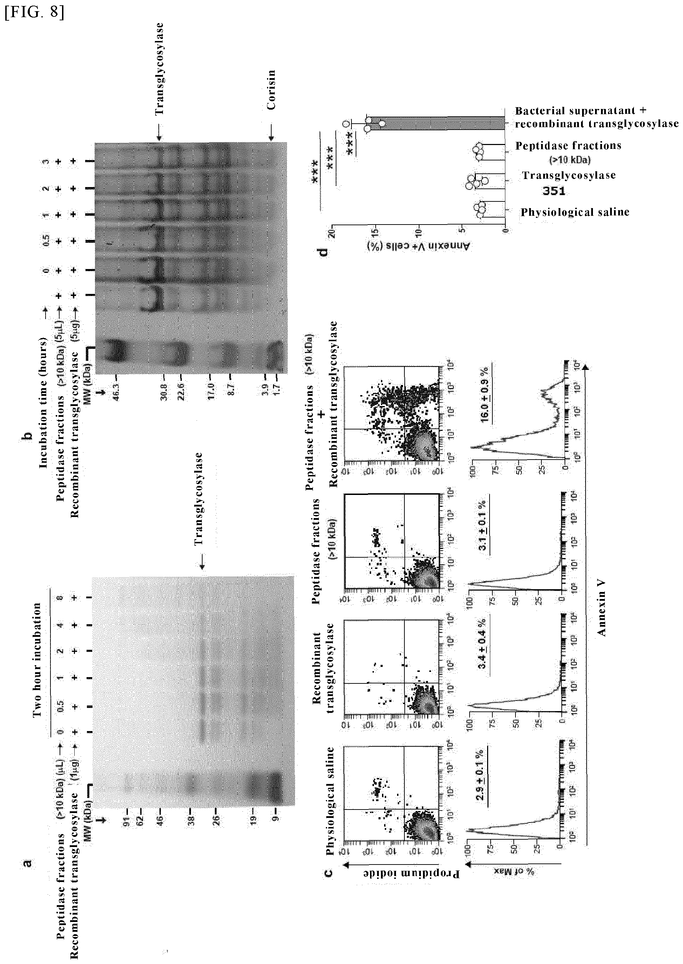

[FIG. 8] FIG. 8 illustrates that bacterial secretion products degrade transglycosylases to induce apoptosis of lung epithelial cells. FIG. 8a: Photograph showing the result of subjecting recombinant transglycosylase to sodium dodecyl sulfate-polyacrylamide gel following a two-hour incubation in the presence of varying amounts of peptidase fractions prepared from the Staphylococcus nepalensis culture supernatant. FIG. 8b: Photograph showing the result of subjecting recombinant transglycosylase to sodium dodecyl sulfate-polyacrylamide gel after incubation in the presence of fixed amounts of peptidase fractions at varying time intervals. FIG. 8c: Shows the flow cytometry analysis results of evaluating apoptosis of A549 alveolar epithelial cells after treatment using the indicated reaction mixture. The dots in the upper part of c , excluding the third quadrant, are apoptotic cells, and the bumps on the right side of the X-axis in the lower part of c are apoptotic cells. FIG. 8d: Graph showing the percentage of apoptotic cells compared to the control. N = 4 in each group. Data are mean±SD statistical analysis by one-way analysis of variance with Tukey's test. ***p <0.0001.

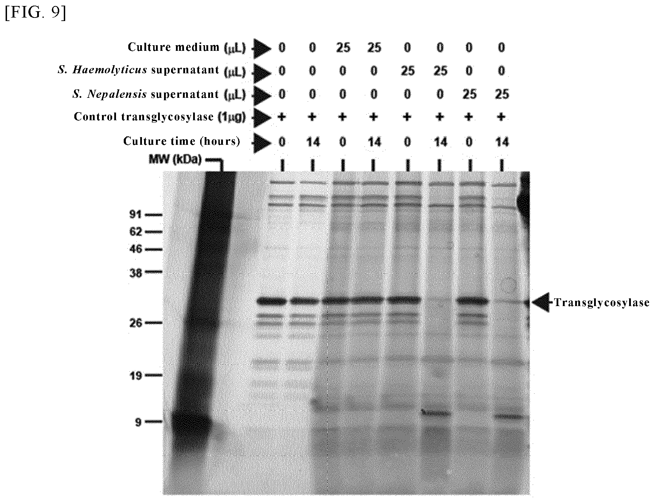

[FIG. 9] FIG. 9 is a photograph of sodium dodecyl sulfate-polyacrylamide gel showing that the Staphylococcus haemolyticus culture supernatant degrades the recombinant transglycosylase. The supernatant from Staphylococcus nepalensis served as the control.

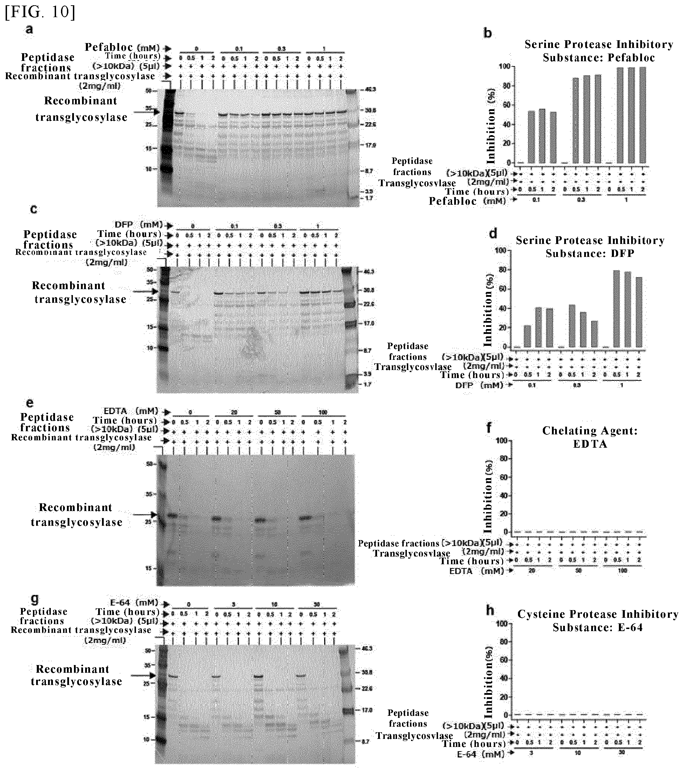

[FIG. 10] FIG. 10 illustrates that the putative serine protease derived from bacteria cleaves transglycosylases. FIG. 10a, b: A photograph and graph showing the effect of the serine protease inhibitor Pefabloc SC (a, b) on transglycosylase degradation. FIG. 10c, d: A photograph and graph showing the effect of diisopropyl fluorophosphate (DFP) on transglycosylase degradation. FIG. 10e, f: A photograph and graph showing the effect of the chelating agent ethylenediaminetetraacetic acid (EDTA) on transglycosylase degradation. FIG. 10g, h: A photograph and graph showing the effect of the cysteine protease inhibitor E-64 on transglycosylase degradation.

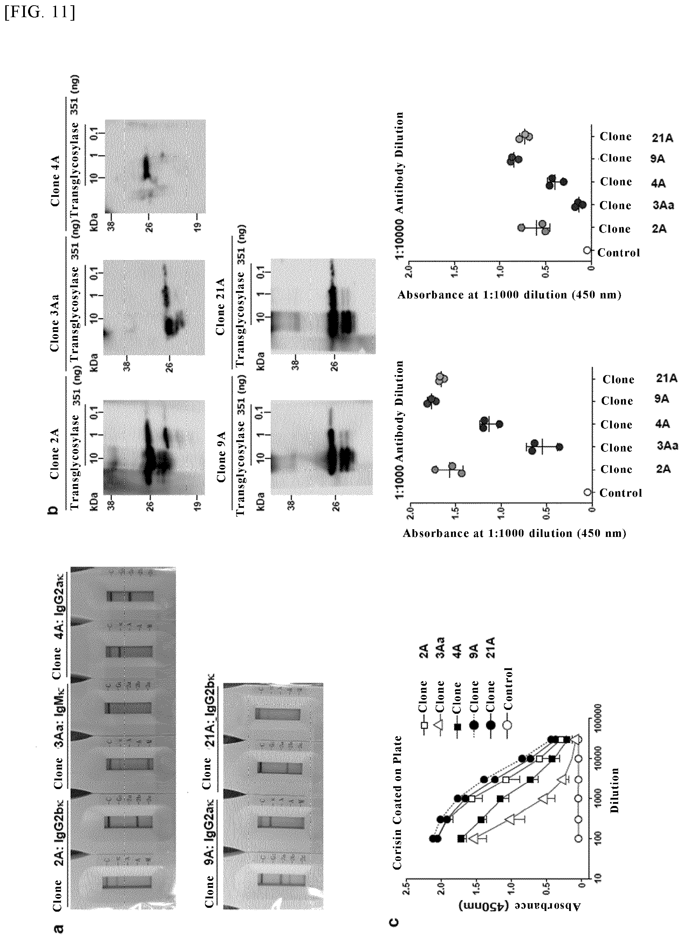

[FIG. 11] FIG. 11 shows that different classes of the anticorisin monoclonal antibody bind to the corisin peptides and corisin sequences on transglycosylase 351. FIG. 11a: Illustrates the isotype of each monoclonal antibody. FIG. 11b: Western blotting results showing the binding properties of each monoclonal antibody to transglycosylase 351. FIG. 11c: A graph evaluating the binding of each clone of the anticorisin monoclonal antibody. Data are expressed as mean±SD. The small band below the 26 kDa molecular weight marker is a degradation product of transglycosylase 351, which is known to degrade during purification of recombinant proteins.

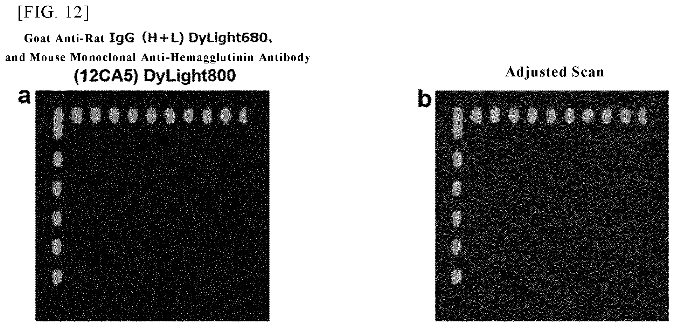

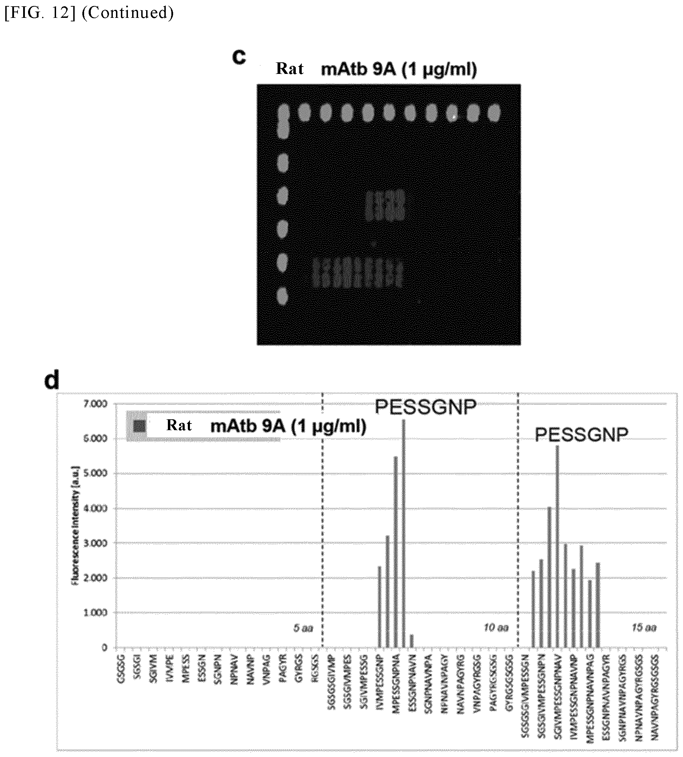

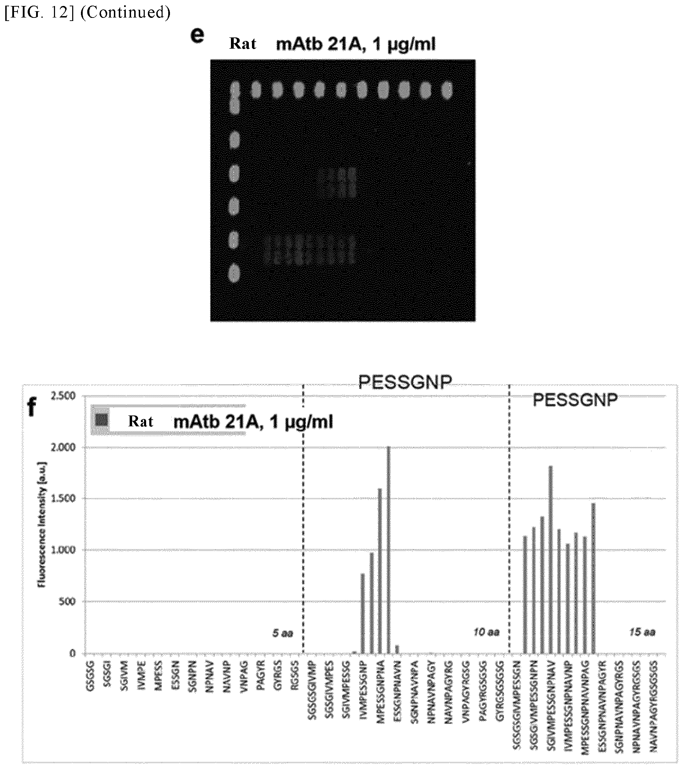

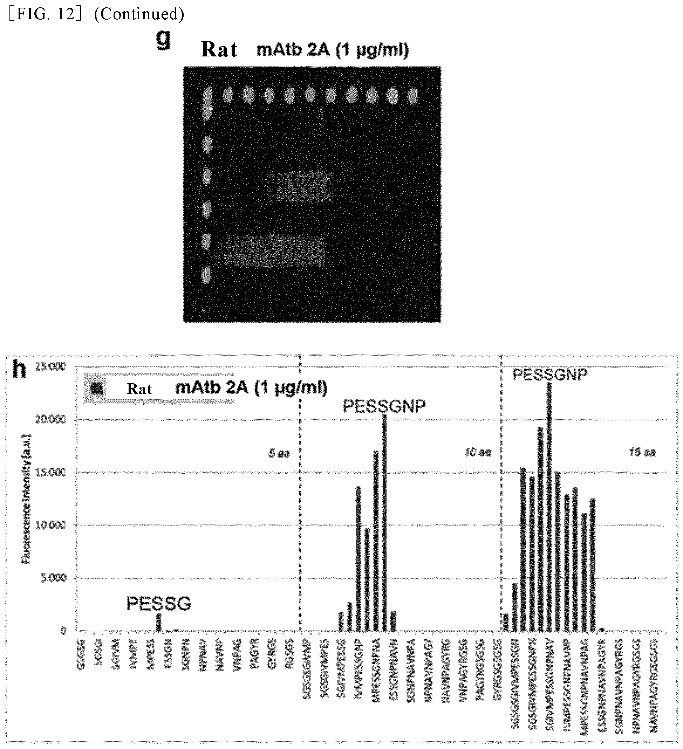

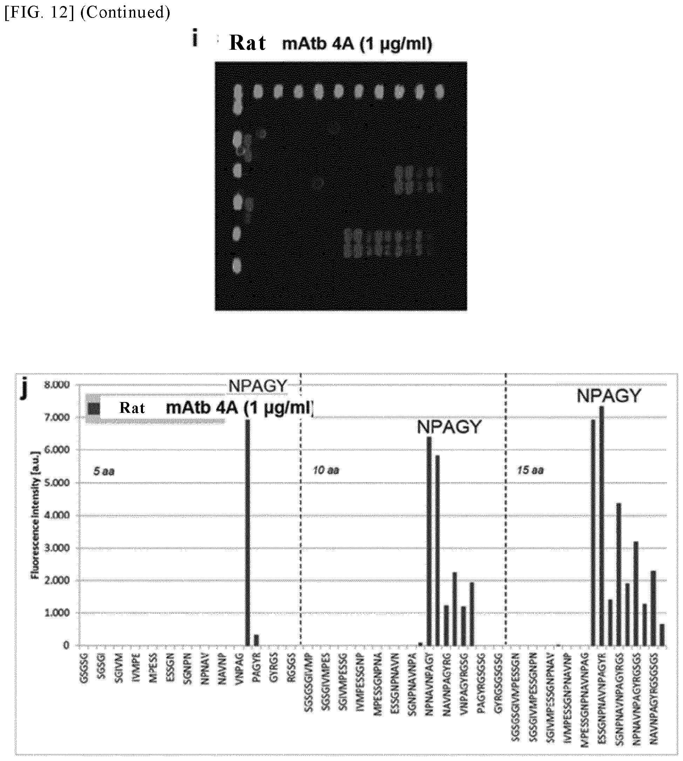

[FIG. 12] FIG. 12 illustrates that the rat anticorisin monoclonal antibody binds to the corisin consensus motifs PESSGNP or NPAGY. FIG. 12a, b: Shows the results of staining of peptide microarray copies verifying overall peptide microarray integrity and assay quality. FIG. 12c, d: Illustrates the scan intensity of rat monoclonal (mAtb) antibody 9A. FIG. 12e, f: Illustrates the scan intensity of rat monoclonal (mAtb) antibody 21A. FIG. 12g, h: Illustrates the scan intensity of rat monoclonal (mAtb) antibody 2A. FIG. 12i, j: Illustrates the scan intensity of rat monoclonal (mAtb) antibody 4A.

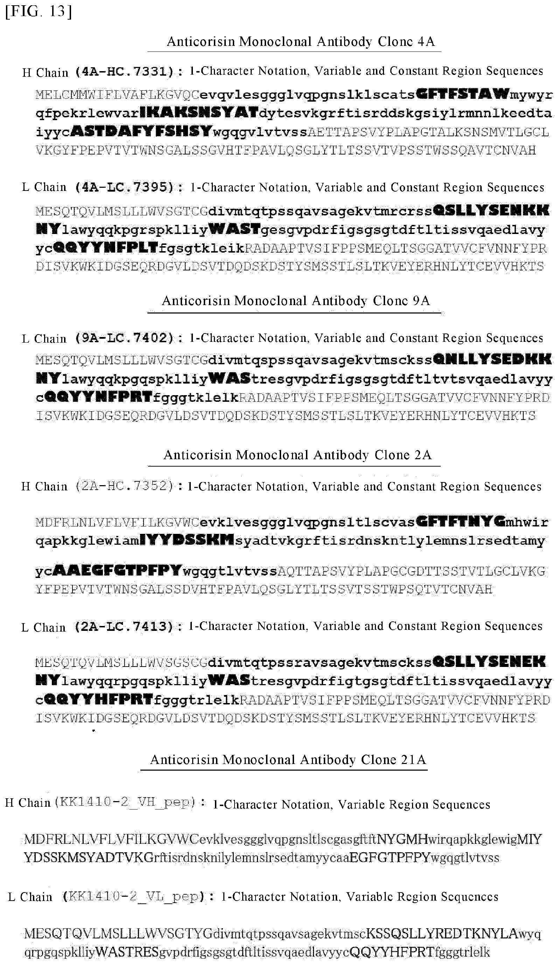

[FIG. 13] FIG. 13: Illustrates the sequencing of variable frameworks and complementarity determining regions of anticorisin monoclonal antibody clones. Lower case letters indicate the framework regions (FR1, FR2, FR3, FR4) and bold upper case letters indicate the CDR regions (CDR1, CDR2, CDR3) of the antibody variable regions.

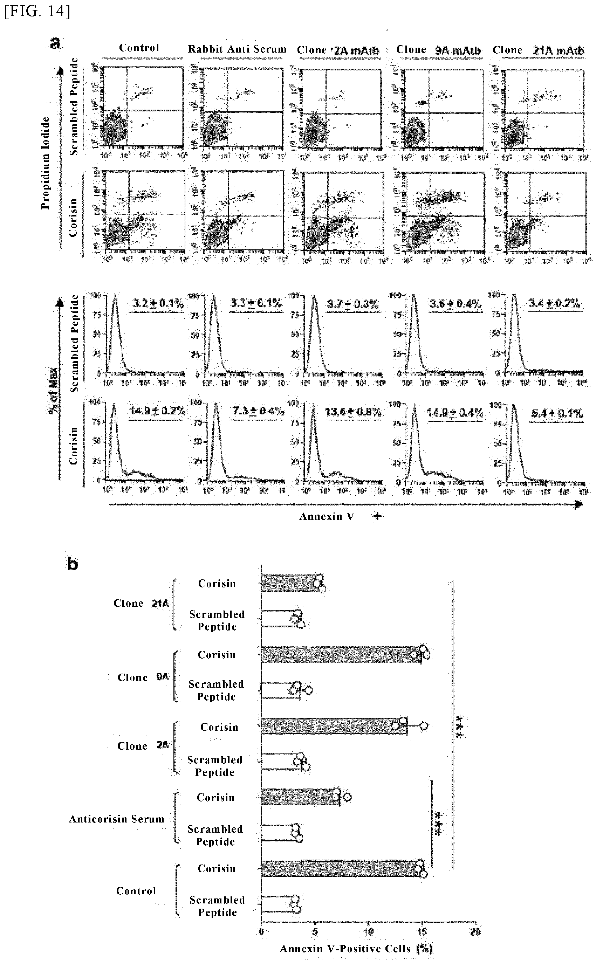

[FIG. 14] FIG. 14 includes various graphs showing that the anticorisin monoclonal antibody clone 21A has potent neutralizing activity against corisin-induced apoptosis of human alveolar epithelial cells. FIG. 14a: Results of flow cytometry analysis showing the inhibitory effect of the anticorisin monoclonal antibody on the pro-apoptotic activity of corisin on cultured alveolar epithelial cells. The dots in the upper part of a, excluding the third quadrant portion, are apoptotic cells. The bumps on the right side of the X-axis in the lower part of a indicate apoptotic cells. FIG. 14b: Graph showing the percentage of apoptotic cells determined and quantified by flow cytometry compared to the control. N = 3 in each group. Data are expressed as mean±SD. Statistical analysis was performed using Student's t-test. ***p <0.0001.

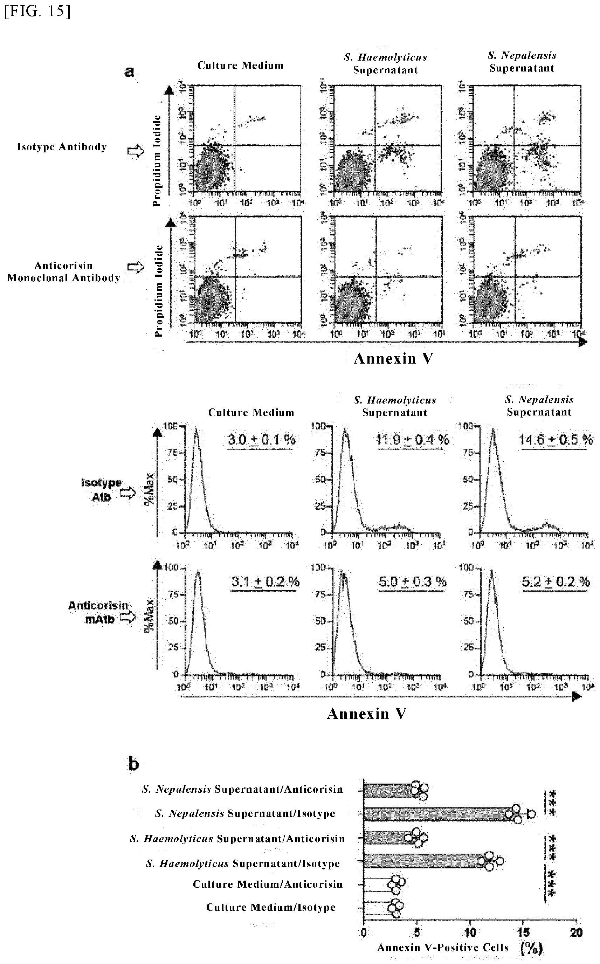

[FIG. 15] FIG. 15 illustrates that the anticorisin monoclonal antibody clone 21A neutralizes the apoptotic activity of the culture supernatant from Staphylococcus haemolyticus against human alveolar epithelial cells. FIG. 15a: Results of flow cytometry analysis showing the inhibitory effect of the anticorisin monoclonal antibody clone 21A on the pro-apoptotic activity of corisin derived from Staphylococcus haemolyticus. The dots in the upper part of a, excluding the third quadrant portion, are apoptotic cells. The bumps on the right side of the X-axis in the lower part of a indicate apoptotic cells. FIG. 15b: Graph showing the percentage of apoptotic cells determined and quantified by flow cytometry compared to the control. N = 4 in each group. Data are expressed as mean±SD. Statistical analysis was performed using one-way analysis of variance with Tukey's test. ***p <0.0001.

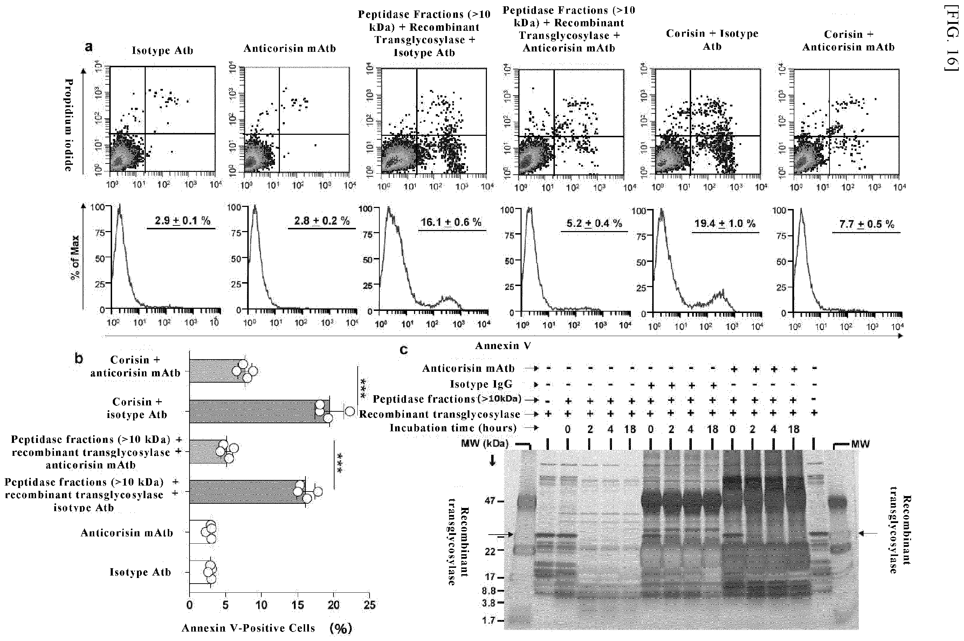

[FIG. 16] FIG. 16 illustrates that the anticorisin neutralizing monoclonal antibody inhibits lung cell apoptosis induced by transglycosylase degradation products. FIG. 16a: Results of flow cytometry analysis showing the inhibitory effect of the anticorisin neutralizing monoclonal antibody on lung cell apoptosis induced by transglycosylase degradation products. The dots in the upper part of a, excluding the third quadrant portion, are apoptotic cells. The bumps on the right side of the X-axis in the lower part of a indicate apoptotic cells. FIG. 16b: Graph showing the percentage of apoptotic cells determined by flow cytometry compared to the control. N = 4 in each group. Data are expressed as mean±SD. Statistical analysis was performed using one-way analysis of variance with Tukey's test. ***p <0.0001. FIG. 16c: Shows the results of subjecting the reaction mixture to sodium dodecyl sulfate-polyacrylamide gel electrophoresis and staining with silver stain.

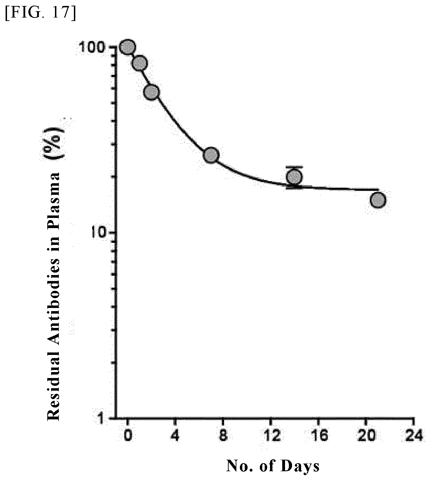

[FIG. 17] FIG. 17 is a graph illustrating the average half-life period of the anticorisin neutralizing antibody in plasma.

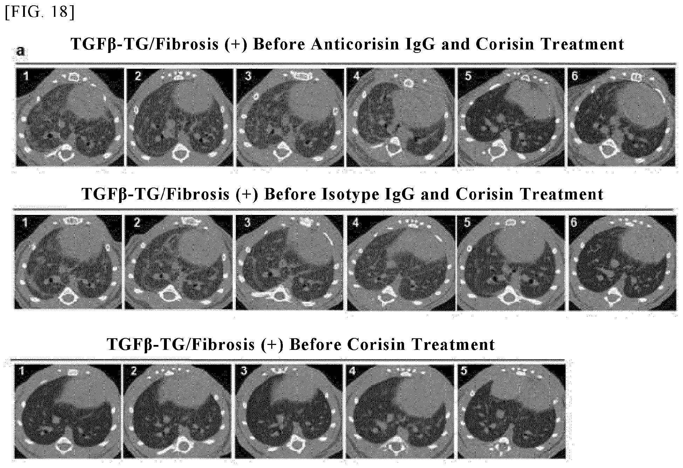

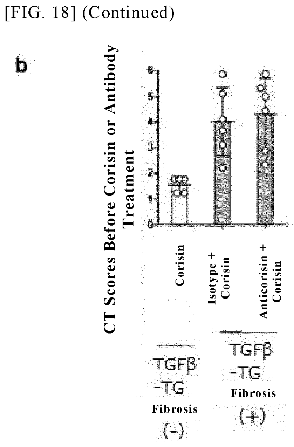

[FIG. 18] FIG. 18 shows matched scores for pulmonary fibrosis prior to intratracheal instillation of corisin between TGFβ1 TG mice that were administered the anticorisin antibody and TGFβ1 TG mice that were administered the isotype antibody. FIG. 18a: Results of computed tomography (CT) performed before starting treatment using the anticorisin monoclonal antibody (mAtb) or the isotype antibody (Atb). N = 6 in the TGFβ1-TG/fibrosis (+)/anticorisin antibody/corisin and TGFβ1-TG/fibrosis (+)/isotype antibody/corisin groups, and n = 5 in the TGFβ1-T/fibrosis (-)/corisin group. FIG. 18b: Graph showing the average scores recorded by eight experts as grades of pulmonary fibrosis in CT. Data are expressed as mean±SD. Statistical analysis was performed using one-way analysis of variance with Tukey's test. TGFβ1: transforming growth factor β1, TG: transgenic.

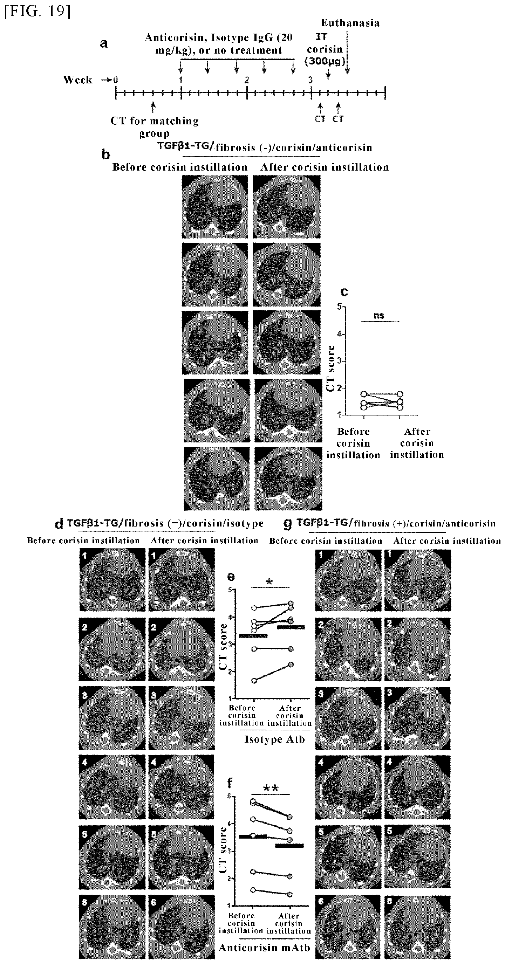

[FIG. 19] FIG. 19 shows that the monoclonal anticorisin antibody prevents exacerbation of lung CT findings in TGFβ1 TG mice. FIG. 19a: Illustrates the schedule for obtaining CT findings in TGFβ1 TG mice with pulmonary fibrosis. The gradings of the CT findings for pulmonary fibrosis were blinded and scored by experts. FIG. 19b, c: CT findings before and after intratracheal instillation of corisin in the TGFβ1-TG/fibrosis (-)/corisin group and a graph showing their scores. Statistical analysis was performed using paired Student's t-test. ns, not significant. FIG. 19d, e: CT findings before and after intratracheal instillation of corisin in the TGFβ1-TG/fibrosis (+)/corisin/isotype group and a graph showing their scores. Bars indicate average scores. Statistical analysis was performed by paired t-test. *p <0.05. TGFβ1: transforming growth factor β1, TG: transgenic. FIG. 19f, g: CT findings before and after intratracheal instillation of corisin in the TGFβ1-TG/fibrosis (+)/corisin/anticorisin group and a graph showing their scores. Bars indicate average scores. Statistical analysis was performed by paired t-test. **p <0.01.

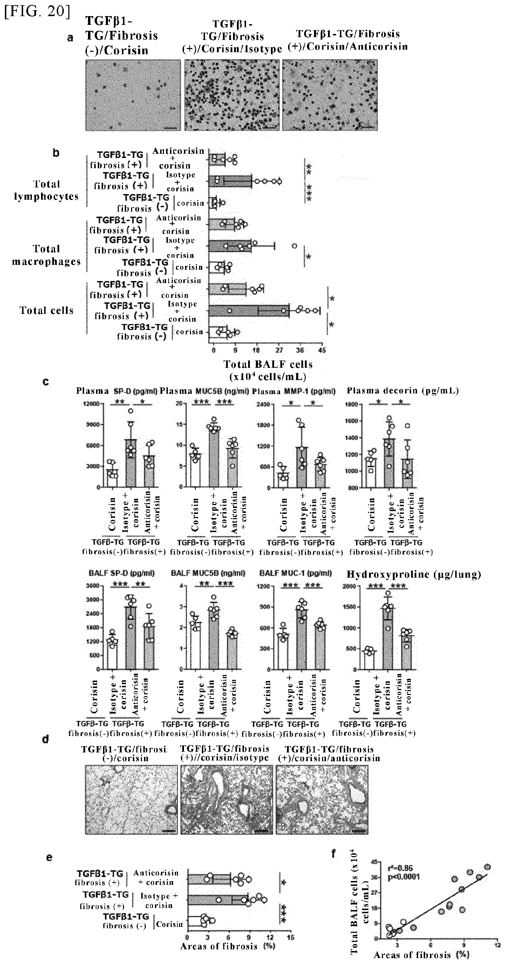

[FIG. 20] FIG. 20 shows that the monoclonal anticorisin antibody suppresses acute exacerbation (AE) of pulmonary fibrosis in TGFβ1 TG mice. FIG. 20a: Shows Giemsa staining results of cell fraction numbers. The scale bar indicates 50 µm. FIG. 20b: Graph showing the total number of BALF cells and neutrophils. Data are mean±SD. Statistical analysis was performed by one-way analysis of variance with Neuman-Keuls test. *p <0.05; **p <0.01; ***p <0.0001. FIG. 20c: Graphs showing the results of measuring the levels of surfactant protein D (SP-D), MUC5B protein, matrix metalloproteinase-1 (MMP-1), decorin, and MUC-1 using a commercially available enzyme immunoassay kit. Hydroxyproline levels were measured using a commercially available colorimetric kit in accordance with the manufacturer's instructions. Data are mean±SD. Statistical analysis was performed by one-way analysis of variance with Neuman-Keuls test. *p <0.05; **p <0.01; ***p <0.0001. FIG. 20d, e: Photographs and a graph showing collagen deposition in the lung tissue of each group by Masson's staining. The scale bar indicates 200 µm. Data are mean±SD. Statistical analysis was performed by one-way analysis of variance with Neuman-Keuls test. *p <0.05; ***p <0.0001. FIG. 20f: Graph showing a correlation between the fibrosis area and total number of inflammatory cells as described in FIG. 20b. Statistical analysis using Spearman's correlation coefficient.

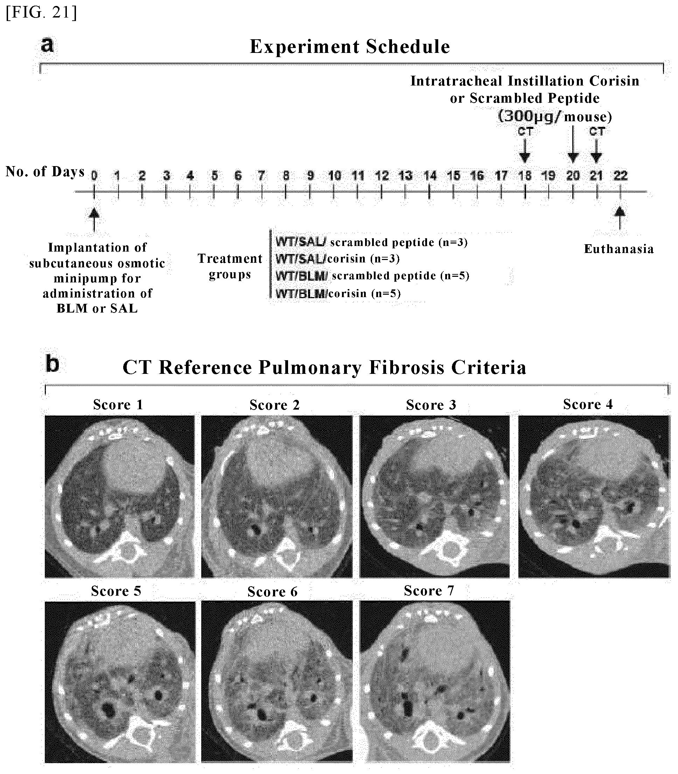

[FIG. 21] FIG. 21 shows the experiment schedule for induction of acute exacerbation in mice with bleomycin-induced pulmonary fibrosis and the CT criteria for pulmonary fibrosis. FIG. 21a: Shows the experiment schedule for induction of acute exacerbation in mice with bleomycin-induced pulmonary fibrosis. FIG. 21b: Shows the CT criteria for pulmonary fibrosis. The following CT scoring system was used to determine the grade of pulmonary fibrosis. Score 1: Normal findings. Score 2: Slightly increased lung density. Score 3: Speckled ground-glass opacity and septal thickening. Score 4: Moderate ground-glass opacity and septal thickening. Score 5: Subpleural thickening, diffuse ground-glass opacity, and reticular septal thickening. Score 6: Subpleural thickening, diffuse ground-glass opacity, reticular septal thickening, and traction bronchiectasis. Score 7: Subpleural thickening, severe ground-glass opacity, reticular septal thickening, and diffuse traction bronchiectasis.

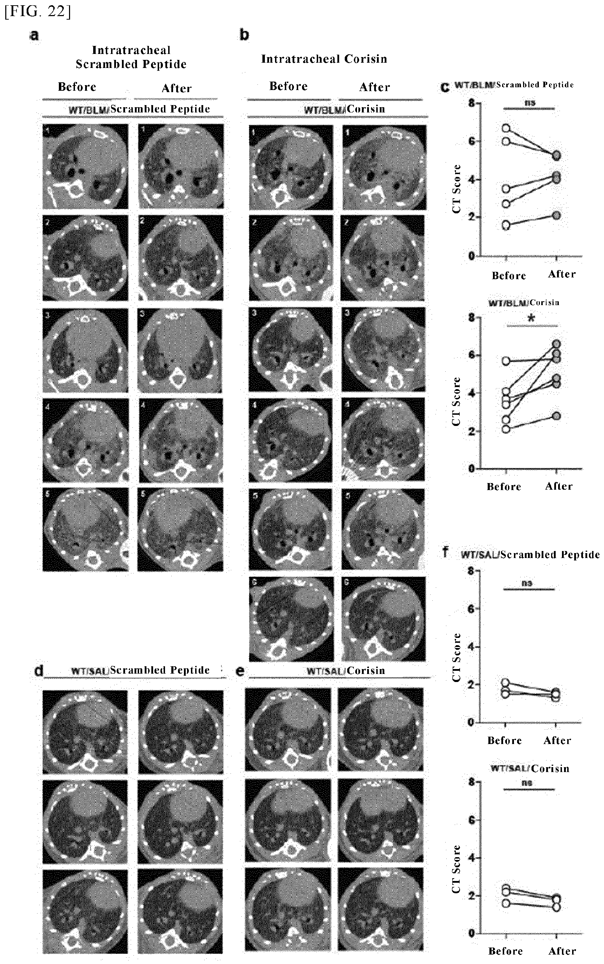

[FIG. 22] FIG. 22 shows a deterioration of lung CT findings after intratracheal instillation of corisin in mice with bleomycin-induced pulmonary fibrosis. FIG. 22a, b, c: Lung CT findings after intratracheal instillation of corisin in mice with bleomycin-induced pulmonary fibrosis and graphs showing their scores. Number of mice in WT/BLM/scrambled peptide: n = 5, and WT/BLM/corisin group: n = 6. Statistical analysis by Student's paired t-test. *p <0.05; ns, not significant. FIG. 22d, e, f: Lung CT findings after intratracheal instillation of corisin in mice with pulmonary fibrosis that were administered physiological saline and graphs showing their scores. Number of mice: n = 3 in the WT/SAL/scrambled peptide and WT/SAL/corisin groups. Statistical analysis by paired t-test. ns, not significant.

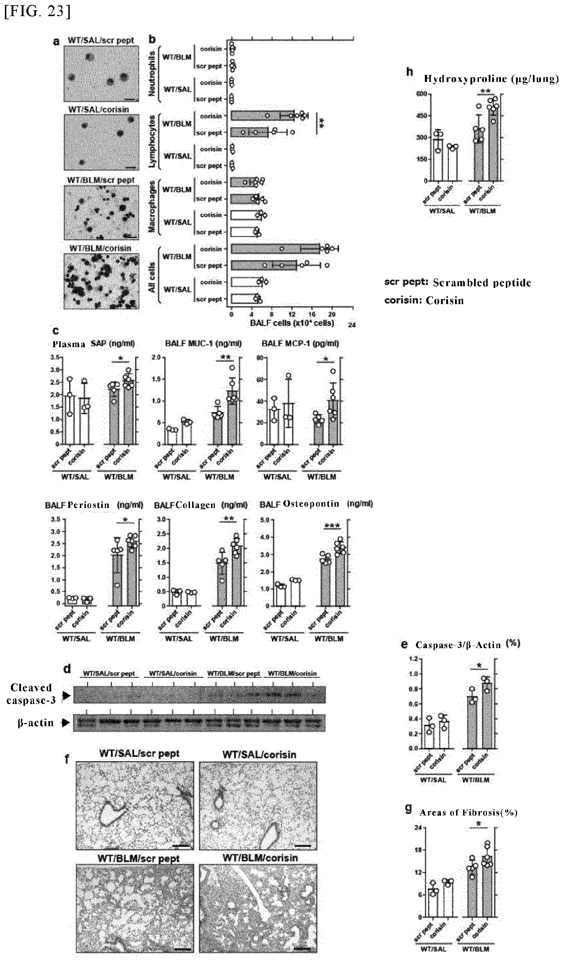

[FIG. 23] FIG. 23 shows acute exacerbation of pulmonary fibrosis after intratracheal instillation of corisin in mice with bleomycin-induced pulmonary fibrosis. FIG. 23a: Shows representative photomicrographs of BALF cell staining for each treatment group in mice with BLM-induced pulmonary fibrosis that were administered corisin. FIG. 23b: Graph showing the number of stained BALF cells in mice with BLM-induced pulmonary fibrosis that were administered corisin in each treatment group. The scale bar indicates 20 µm. Data are mean±SD statistical analysis by a t-test. *p <0.05; *p <0.01. Number of mice in the WT/SAL/scrambled peptide and WT/SAL/corisin groups: n = 3, and WT/BLM/scrambled peptide and WT/BLM/corisin groups: n = 5. FIG. 23c: Graphs showing the levels of serum amyloid P component (SAP), MUC-1, monocyte chemoattractant protein-1 (MCP-1), periostin, collagen I, and osteopontin in mice with BLM-induced pulmonary fibrosis that were administered corisin. Data are mean±SD statistical analysis by unpaired t-test. *p <0.05; *p <0.01; ***p <0.001. FIG. 23d: Shows the results of evaluation and quantification by Western blotting of caspase-3 cleavage in mice with BLM-induced pulmonary fibrosis that were administered corisin. FIG. 23e: Shows a graph of the ratio of cleaved caspase-3 to β-actin in mice with BLM-induced pulmonary fibrosis that were administered corisin. Bars indicate mean±SD statistical analysis by unpaired t-test. *p <0.05. FIG. 23f: Shows the results of quantifying collagen deposition. FIG. 23g: Shows a graph of the results of quantifying collagen deposition. Data are mean±SD statistical analysis by unpaired t-test. *p <0.05. FIG. 23h: Shows a graph depicting the measurement results of hydroxyproline content in lung tissue. Bars indicate mean±SD statistical analysis by unpaired t-test. *p <0.05; **p <0.001.

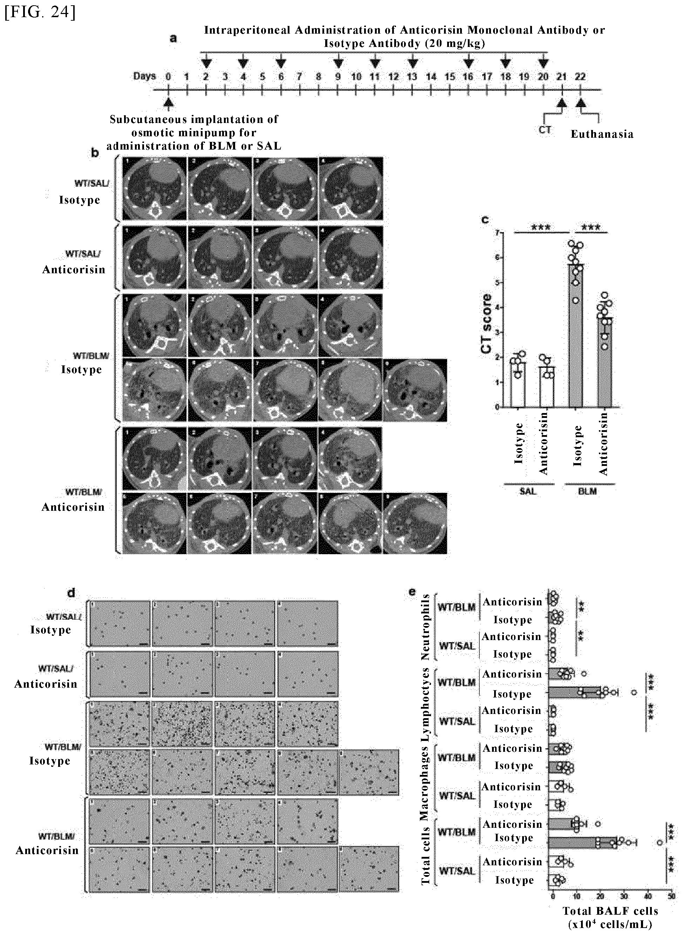

[FIG. 24] FIG. 24 shows improved lung CT findings and reduced lung inflammatory cell infiltration in mice with bleomycin-induced pulmonary fibrosis that were treated with the anticorisin monoclonal antibody. FIG. 24a: Shows the schedule for investigating the suppressive effect of the anticorisin monoclonal antibody on pulmonary fibrosis. FIG. 24b, c: b shows lung CT findings in mice with bleomycin-induced pulmonary fibrosis that were treated with the anticorisin monoclonal antibody. c is a graph of CT scores reflecting the results of b. Number of mice in the WT/SAL/isotype and WT/SAL/anticorisin groups: n = 4, and WT/BLM/anticorisin and WT/BLM/isotype groups: n = 9. Data are mean±SD statistical analysis by one-way analysis of variance with Tukey's test. ***p <0.001. FIG. 24d, e: d shows photographs of cells in the BALF of mice with bleomycin-induced pulmonary fibrosis that were treated with the anticorisin monoclonal antibody. e shows the total number of BALF cells and the number of fractional cells in mice with bleomycin-induced pulmonary fibrosis that were treated with the anticorisin monoclonal antibody. Number of mice in the WT/SAL/isotype and WT/SAL/anticorisin groups: n = 4, and WT/BLM/anticorisin and WT/BLM/isotype groups: n = 9. The scale bar indicates 50 µm. Data are mean±SD statistical analysis by one-way analysis of variance with Tukey's test. **p <0.01, ***p <0.0001.

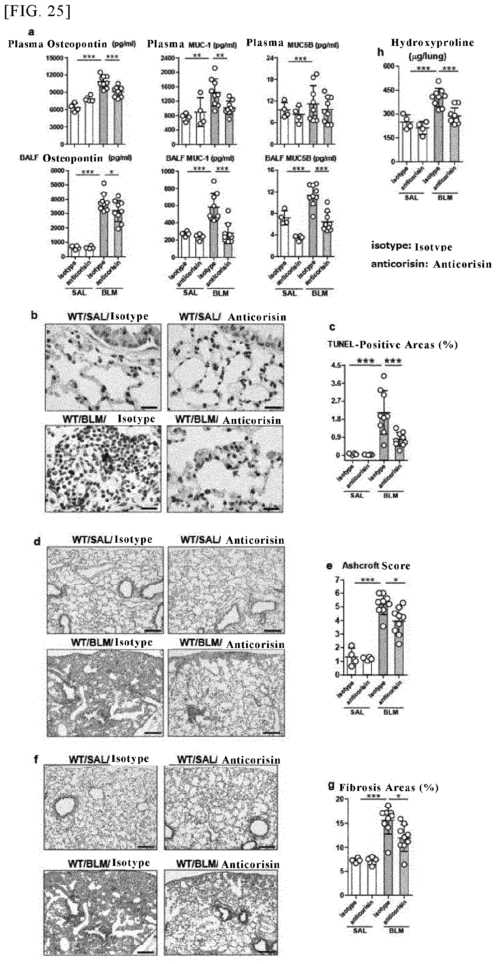

[FIG. 25] FIG. 25 shows amelioration of acute tissue injury, parenchymal cell apoptosis, and tissue fibrosis in the lungs of mice with pulmonary fibrosis that were treated with the anticorisin monoclonal antibody. FIG. 25a: Graphs showing levels of osteopontin, MUC-1, and MUC5B in the lungs of mice with pulmonary fibrosis that were treated with the anticorisin monoclonal antibody. N = 4 in the WT/SAL/isotype and WT/SAL/anticorisin groups, and n = 9 in the WT/BLM/isotype and WT/BLM/anticorisin groups. Bars indicate mean±SD statistical analysis by one-way analysis of variance with Tukey's test. *p <0.05, **p <0.01, ***p <0.0001. FIG. 25b: Photographs showing DNA fragmentation in the lungs of mice with pulmonary fibrosis mice that were treated with the anticorisin monoclonal antibody. Arrows indicate apoptotic cells. FIG. 25c: Graph quantifying apoptotic cells in the lungs of mice with pulmonary fibrosis that were treated with the anticorisin monoclonal antibody. The scale bar indicates 20 µm. N = 4 in the WT/SAL/isotype and WT/SAL/anticorisin groups, and n = 9 in the WT/BLM/isotype and WT/BLM/anticorisin groups. Bars indicate mean±SD statistical analysis by one-way analysis of variance with Tukey's test. ***p <0.0001. FIG. 25d, e: Shows the results of seven experts using Ashcroft's score to blindly score the grade of pulmonary fibrosis in the lungs of mice with pulmonary fibrosis that were treated with the anticorisin monoclonal antibody. The scale bar indicates 200 µm. N = 4 in the WT/SAL/isotype and WT/SAL/anticorisin groups, and n = 9 in the WT/BLM/isotype and WT/BLM/anticorisin groups. Bars indicate mean±SD statistical analysis by analysis of variance with Tukey's test. *p <0.05, ***p <0.0001. FIG. 25f, g: f includes photographs of Masson's trichrome staining for collagen deposition in the lungs, and g is a graph of having quantified the same using WindRoof image software. N = 4 in the WT/SAL/isotype and WT/SAL/anticorisin groups, and n = 9 in the WT/BLM/isotype and WT/BLM/anticorisin groups. Bars indicate mean±SD statistical analysis by one-way analysis of variance with Tukey's test. *p <0.05, ***p <0.0001. FIG. 25h: Graph showing hydroxyproline content in lung tissue, measured by colorimetry. N = 4 in the WT/SAL/isotype and WT/SAL/anticorisin groups, and n = 9 in the WT/BLM/isotype and WT/BLM/anticorisin groups. Bars indicate mean±SD statistical analysis by one-way analysis of variance with Tukey's test. ***p <0.0001.

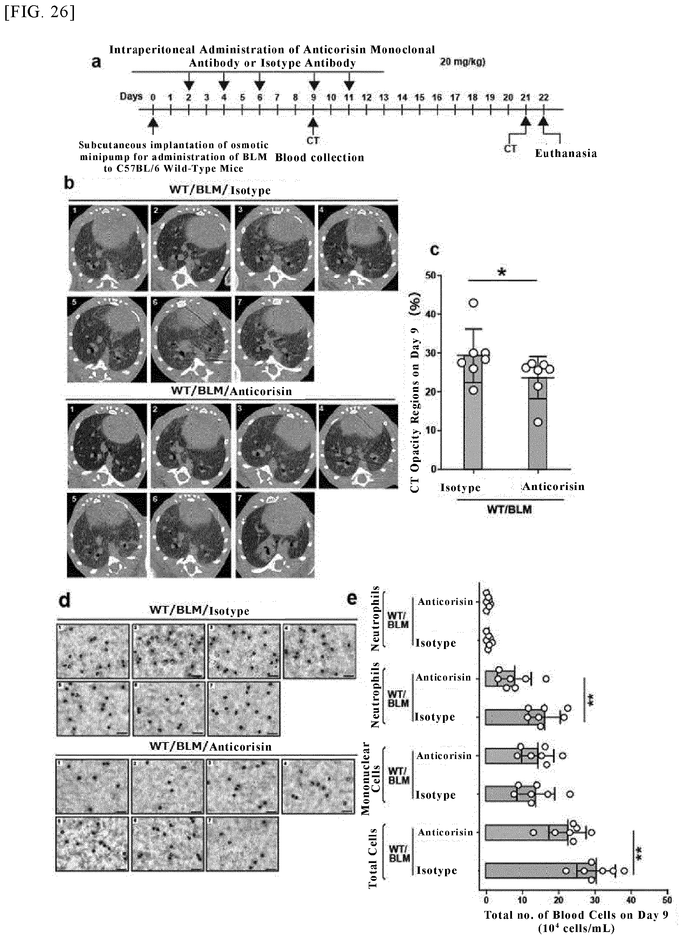

[FIG. 26] FIG. 26 shows lung CT abnormalities and reduction in lung inflammatory cells on day 9 in mice with bleomycin-induced pulmonary fibrosis that were treated with the anticorisin monoclonal antibody during the acute phase of the disease. FIG. 26a: Shows the schedule for examining the amelioration effect of the anticorisin monoclonal antibody on BLM-induced lung injury and pulmonary fibrosis when treated in the acute phase. FIG. 26b, c: b includes photographs showing CT lung opacity in BLM-induced lung injury and pulmonary fibrosis using the anticorisin monoclonal antibody for treatment in the acute phase, and c is a graph of CT scores reflecting the results of b. N = 7 in mice treated with the anticorisin monoclonal antibody (WT/BLM/anticorisin) or isotype antibody (WT/BLM/isotype). Data are mean±SD. Statistical analysis was performed by an unpaired t-test. *p <0.05. FIG. 26d, e: d includes photographs showing blood cells in BLM-induced lung injury and pulmonary fibrosis using the anticorisin monoclonal antibody for treatment in the acute phase, and e is includes graphs showing the total number of cells thereof and the number of cell fractions. N = 7 in mice treated with the anticorisin monoclonal antibody (WT/BLM/anticorisin) or isotype antibody (WT/BLM/isotype). The scale bar indicates 20 µm. Data are mean±SD statistical analysis by unpaired t-test. **p <0.01.

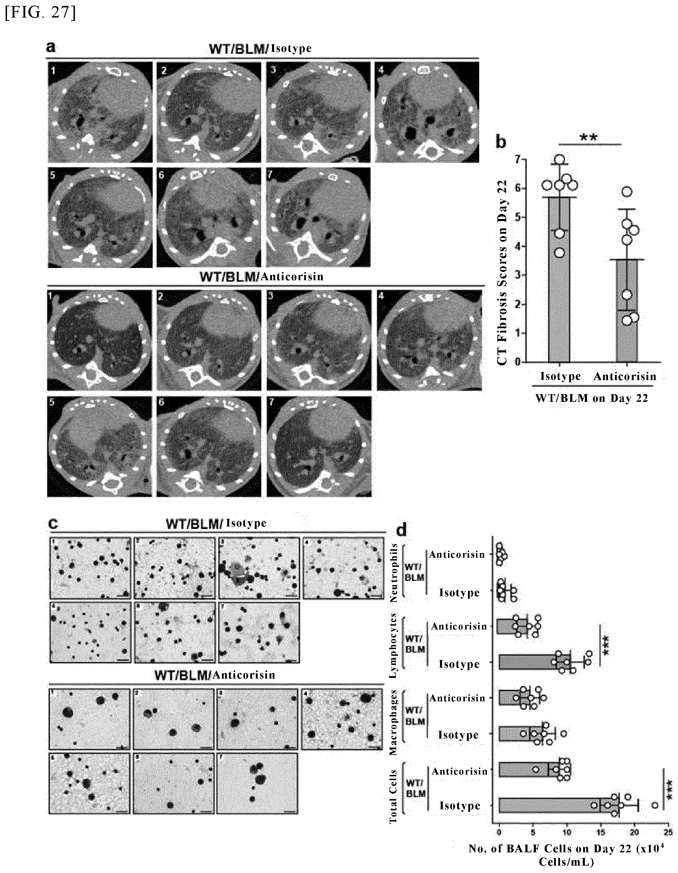

[FIG. 27] FIG. 27 shows lung CT abnormalities and reduction in lung inflammatory cells on day 22 in mice with bleomycin-induced pulmonary fibrosis that were treated with the anticorisin monoclonal antibody during the acute phase of the disease. FIG. 27a, b: a includes photographs showing CT images taken on day 21 following BLM pump infusion (chronic phase) in mice with bleomycin-induced pulmonary fibrosis that were treated with the anticorisin monoclonal antibody during the acute phase of the disease, and b is a graph of CT fibrosis scores on day 22. N = 7 in both treatment groups. Data are mean±SD. Statistical analysis was performed by an unpaired t-test. **p <0.01. FIG. 27c, d: c includes photographs showing cell fractions after Giemsa staining in bronchoalveolar lavage fluid (BALF) at day 22 after BLM pump in mice with bleomycin-induced pulmonary fibrosis that were treated with the anticorisin monoclonal antibody in the acute phase of the disease, and d is a graph illustrating the total number of cells thereof. N = 7 in both treatment groups. The scale bar indicates 20 µm. Data are mean±SD statistical analysis by unpaired t-test. ***p <0.0001.

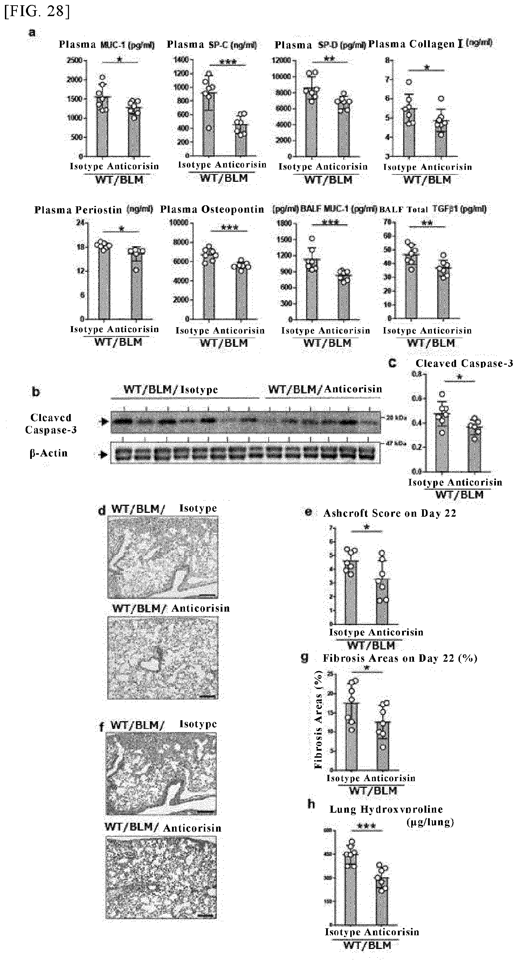

[FIG. 28] FIG. 28 shows an amelioration in lung injury, lung parenchymal apoptosis, and collagen deposition on day 22 in mice with bleomycin-induced pulmonary fibrosis that were treated with the anticorisin monoclonal antibody during the acute phase of the disease. FIG. 28a: Graphs showing levels of MUC-1, surfactant protein C (SP-C), SP-D, collagen I, periostin, osteopontin, and total TGFβ1 in BALF and plasma collected at day 22 after BLM pump infusion. Data are mean±SD. Statistical analysis was performed by an unpaired t-test. *p <0.05, **p <0.01, ***p <0.001. FIG. 28b, c: b shows the results of Western blotting of cleaved caspase-3 and β-actin, and c shows a graph of the cleaved caspase-3 to β-actin ratio, which is the result of b. N = 7 in the WT/BLM/isotype group, and n = 6 in the WT/BLM/anticorisin group. Data are mean±SD. Statistical analysis was performed by an unpaired t-test. *p <0.05. FIG. 28d, e: Seven experts blindly scored the grade of pulmonary fibrosis using Ashcroft's score. The scale bar indicates 200 µm. N = 7 in both the WT/BLM/isotype and WT/BLML/anticorisin groups. Bars indicate mean±SD statistical analysis performed by an unpaired t-test. *p <0.05. FIG. 28f, g: f includes photographs of Masson's trichrome staining for collagen deposition in the lungs, and g is a graph of having quantified the same using WindRoof image software. FIG. 28h: Graph showing hydroxyproline content in the lungs. N = 7 in both the WT/BLM/isotype and WT/BLML/anticorisin groups. Bars indicate mean±SD statistical analysis performed by an unpaired t-test. *p <0.05; ***p <0.0001.

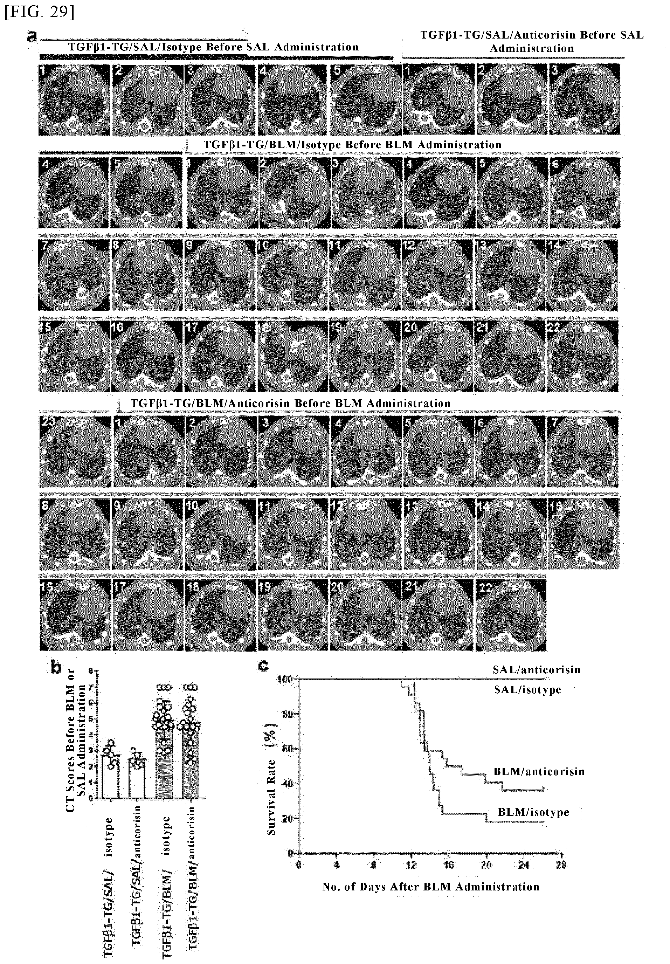

[FIG. 29] FIG. 29 shows that the anticorisin monoclonal antibody prolongs survival in mice with acute exacerbation of pulmonary fibrosis. FIG. 29a, b: a shows computed tomography (CT) results in mice with acute exacerbation of pulmonary fibrosis and b is a graph of CT scores reflecting the results of b. N = 22 in both the WT/BLM/isotype and WT/BLM/anticorisin groups, and n = 5 in both the WT/SAL/isotype and WT/SAL/anticorisin groups. Bars indicate mean±SD statistical analysis was performed by one-way analysis of variance with Tukey's test. *p <0.05. FIG. 29c: Graph showing the results of monitoring survival rates. Statistical analysis was performed by the log-rank test. p = 0.001.

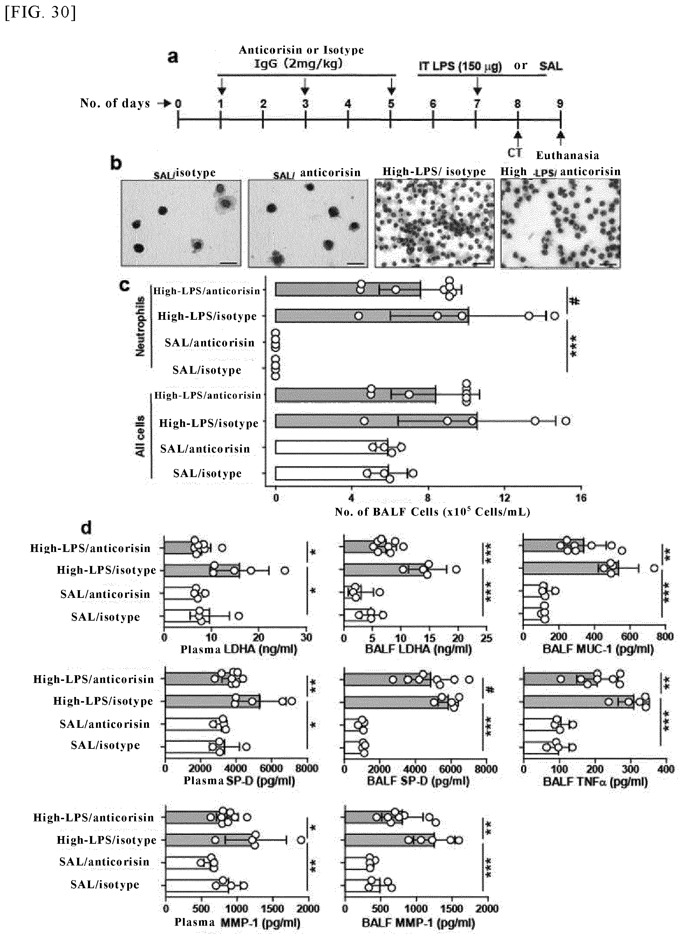

[FIG. 30] FIG. 30 shows that the anticorisin monoclonal antibody attenuates severe lipopolysaccharide-induced acute lung injury. FIG. 30a: Illustrates the schedule of examining that the anticorisin monoclonal antibody attenuates severe lipopolysaccharide-induced acute lung injury. FIG. 30b: Shows representative photomicrographs of BALF cell staining for each treatment group. FIG. 30c: Graph showing cell counts in BALF for each treatment group. The scale bar indicates 20 µm. N = 4 in the SAL/isotype and SAL/anticorisin groups, n = 5 in the LPS/isotype group, and n = 8 in the LPS/anticorisin group. Data are mean±SD statistical analysis by one-way analysis of variance with Neuman-Keuls test. #p = 0.1; ***p <0.0001. FIG. 30d: Graphs showing levels of lactate dehydrogenase A (LDHA), surfactant protein D (SP-D), matrix metalloproteinase-1 (MMP-1), MUC-1, and tumor necrosis factor α (TNFα) in mice with severe lipopolysaccharide-induced acute lung injury. N = 4 in the SAL/isotype and SAL/anticorisin groups, n = 5 in the LPS/isotype group, and n = 8 in the LPS/anticorisin group. Data are mean±SD statistical analysis by analysis of variance with the Newman-Keuls method. #p = 0.1, *p <0.05, **p <0.01, ***p <0.001.

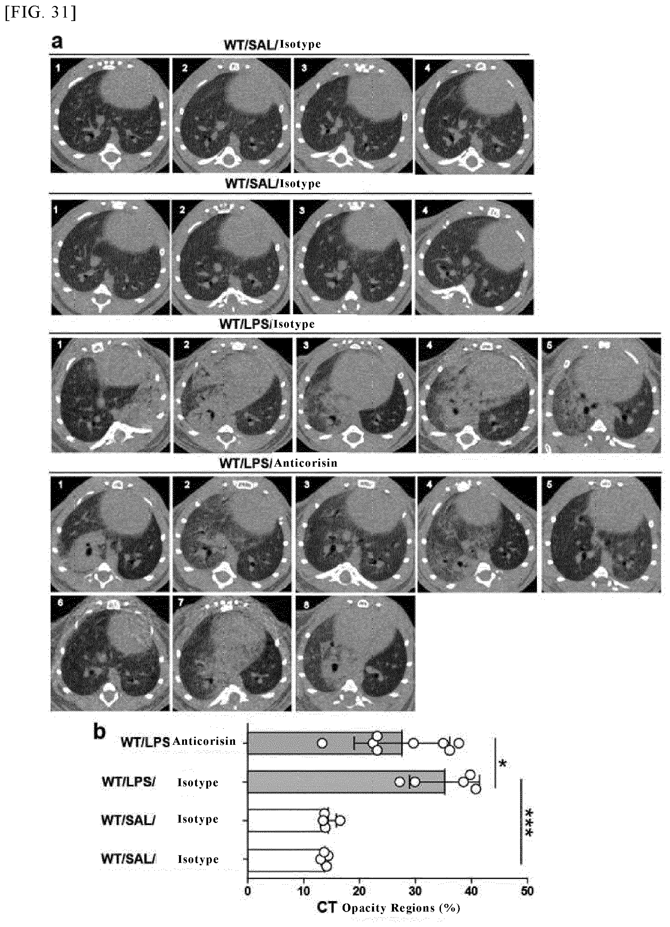

[FIG. 31] FIG. 31 shows that treatment using the anticorisin monoclonal antibody improves CT findings in mice with severe lipopolysaccharide-induced acute lung injury. FIG. 31a: Shows results of computed tomography (CT) performed on day 1 after intratracheal instillation of lipopolysaccharide (LPS). N = 4 in the WT/SAL/isotype and WT/SAL/anticorisin groups, n = 5 in the WT/LPS/isotype group, and n = 8 in the WT/LPS/anticorisin group. FIG. 31b: Graph showing CT opacity from the apical to basal areas of the lungs quantified using WindRoof image software. Data are statistical analysis by one-way analysis of variance with Neuman-Keuls test, which is mean±SD. *p <0.05, ***p <0.001.

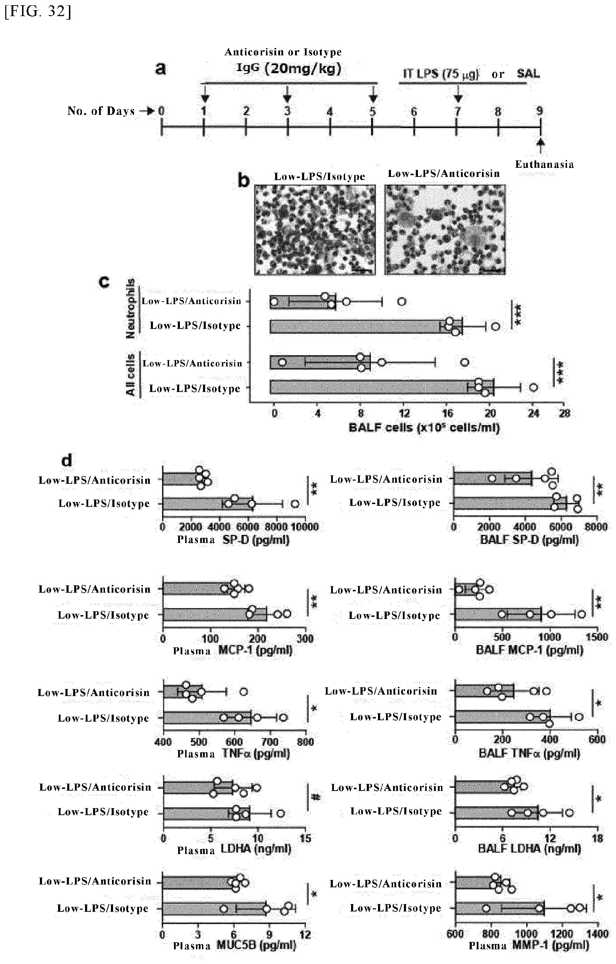

[FIG. 32] FIG. 32 shows that the anticorisin monoclonal antibody attenuates moderate lipopolysaccharide-induced acute lung injury. FIG. 32a: Illustrates the schedule of examining whether the anticorisin monoclonal attenuates moderate lipopolysaccharide-induced acute lung injury. FIG. 32b: Shows representative photomicrographs of BALF cell staining for each treatment group. FIG. 32c: Graph showing stained BALF cell counts for each treatment group. The scale bar indicates 20 µm. N = 4 in the LPS/isotype, and n = 5 in the LPS/anticorisin group. Data are mean±SD statistical analysis from unpaired tests. ***p <0.001. FIG. 32d: Graphs showing BALF and level plasma in plasma of surfactant protein D (SP-D), monocyte chemoattractant protein-1 (MCP-1), TNFα, lactate dehydrogenase A (LDHA), MUC-5B, and matrix metalloproteinase-1 (MMP-1) in mice with moderate lipopolysaccharide-induced acute lung injury that were administered the anticorisin monoclonal. N = 4 in the LPS/isotype group, and n = 5 in the LPS/isotype group. Data are mean±SD statistical analysis by analysis of variance with the Newman-Keuls method. *p <0.05, **p <0.01, ***p <0.001.

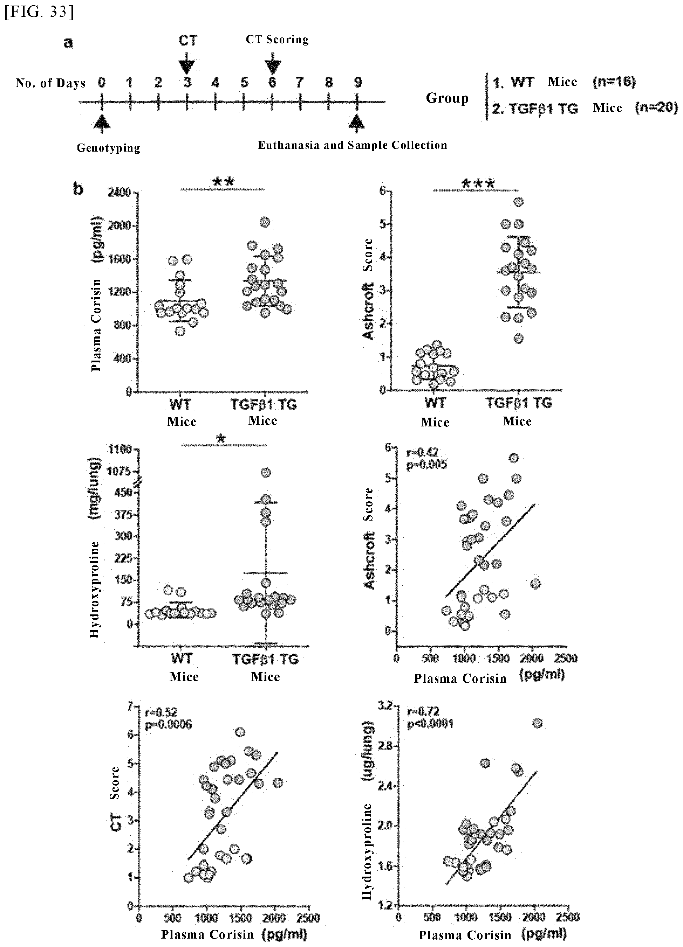

[FIG. 33] FIG. 33 shows that elevated circulating levels of corisin correlate with markers of pulmonary fibrosis. FIG. 33a: Illustrates the schedule for measuring plasma levels of corisin in TGFβ1 transgenic (TG) and wild-type (WT) mice and evaluating correlation with fibrosis markers. N = 16 in the WT mice group, and n = 20 in the TGFβ1 TG mice group. FIG. 33b: Graphs showing Ashcroft scores for pulmonary fibrosis and measurements of plasma corisin and lung hydroxyproline in TGFβ1 TG and WT mice. Statistical differences between groups were analyzed by unpaired t-tests and strength of correlation by Pearson product-moment correlation. *p <0.05, **p <0.01 ***p <0.001.

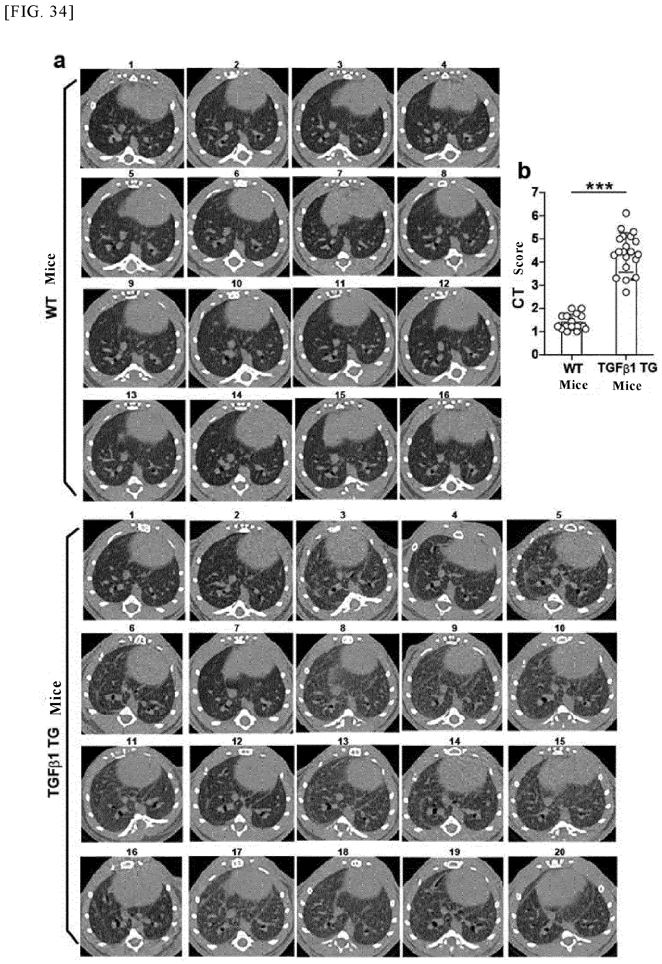

[FIG. 34] FIG. 34 shows increased CT scores for pulmonary fibrosis in TGFβ1 TG mice. FIG. 34a: Shows CT score results for pulmonary fibrosis in TGFβ1 TG mice. FIG. 34b: Graph showing CT opacity from the apical to basal areas of the lungs quantified using WindRoof image software. N = 16 in the WT mice group, and n = 20 in the TGFB 1 TGmice group. WT: wild type, TGFβ1 TG: transforming growth factor β1, CT: computed tomography. Data are mean±SD. Statistical analysis was performed using unpaired Student's t-test. ***p <0.001.

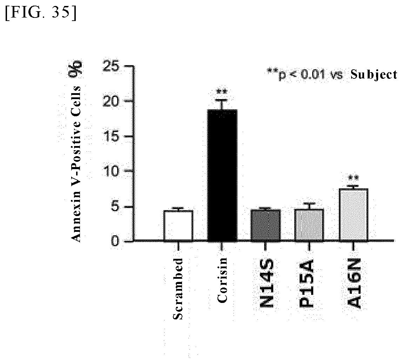

[FIG. 35] FIG. 35 is a graph showing apoptotic activity in corisin peptides with defined amino acid mutations.

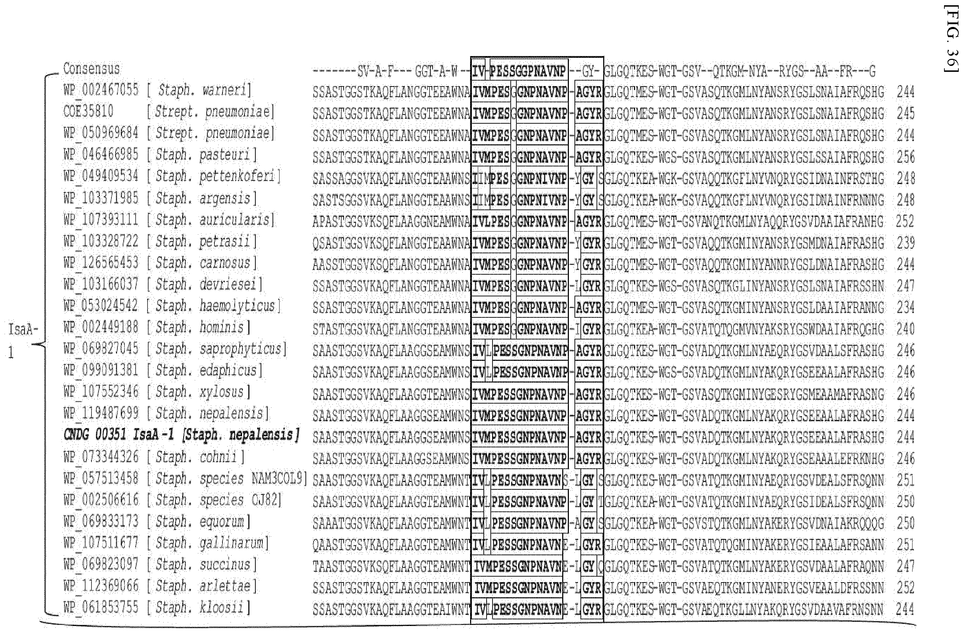

[FIG. 36] FIG. 36 shows a multiple sequence alignment of the conserved sequences of the pro-apoptotic segments of transglycosylases in several species of staphylococci and streptococci.

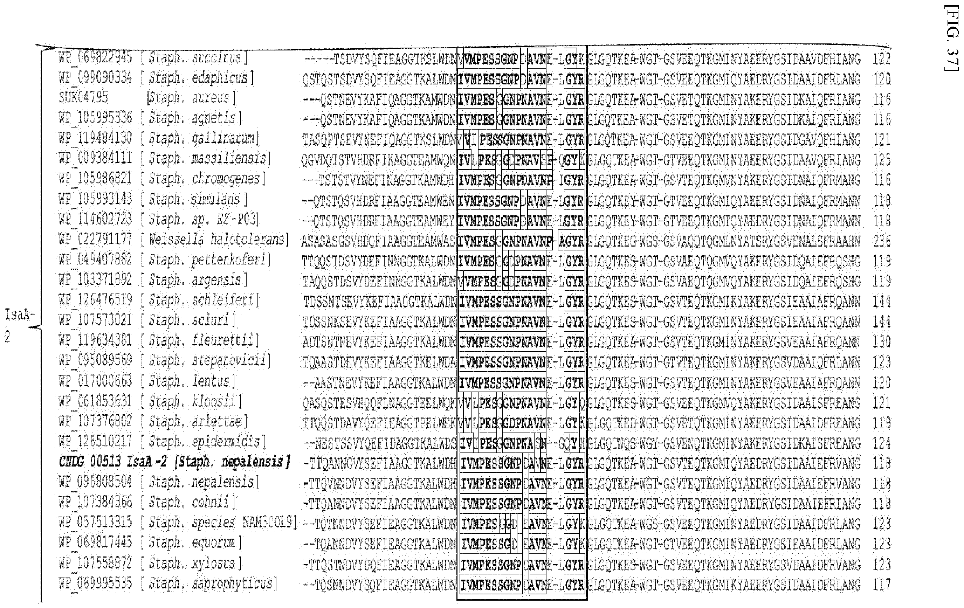

[FIG. 37] FIG. 37 shows a multiple sequence alignment of the conserved sequences of the pro-apoptotic segments of transglycosylases in several species of staphylococci and streptococci.

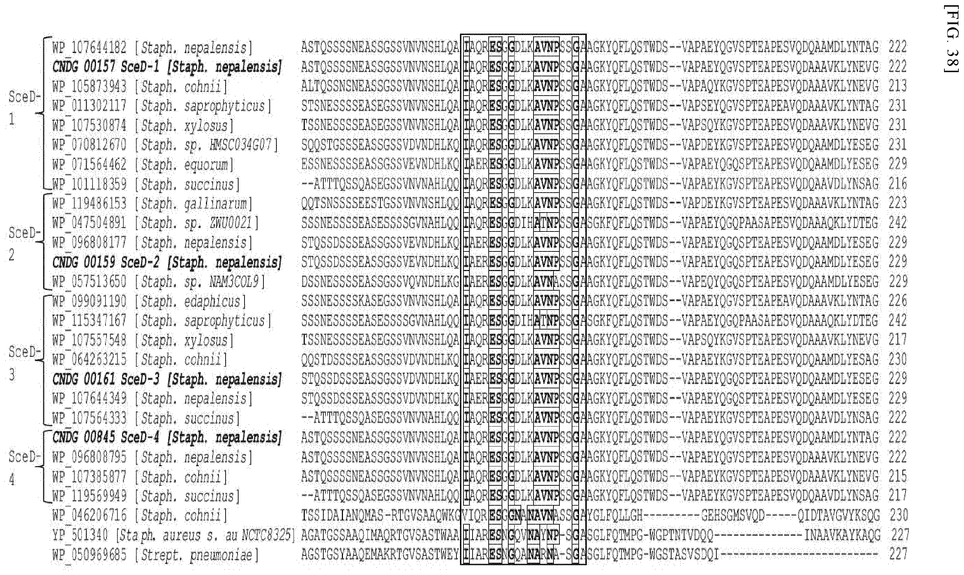

[FIG. 38] FIG. 38 shows a multiple sequence alignment of the conserved sequences of the pro-apoptotic segments of transglycosylases in several species of staphylococci and streptococci. Examples of the corisin shown in FIGS. 36, 37, and 38C may include, for example, amino acid sequence IVMPESGGNPNAVNPAGYR (SEQ ID NO: 58), IIMPESGGNPNIVNPYGYS (SEQ ID NO: 59), IVMPESGGNPNAVNPYGYR (SEQ ID NO: 60), IVLPESSGNPNAVNPAGYR (SEQ ID NO: 61), IVLPESSGNPNAVNELGYR (SEQ ID NO: 62), IVMPESGGNPNAVNELGYR (SEQ ID NO: 63), IVMPESSGNPNAVNELGYR (SEQ ID NO: 64), IVMPESSGNPDAVNELGYR (SEQ ID NO: 65), IAQRESGGDLKAVNPSSGA (SEQ ID NO: 66), or IAERESGGDLKAVNPSSGA (SEQ ID NO: 67), and these may be used in one or a plurality of aspects of the present invention.

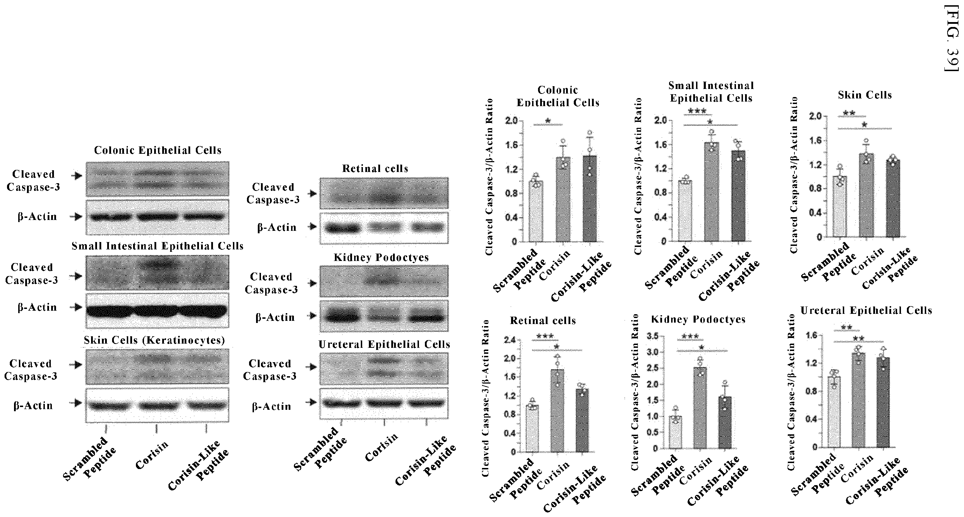

[FIG. 39] FIG. 39 shows photographs where colonic mucosal epithelial cells, small intestinal mucosal epithelial cells, skin keratinocytes, retinal epithelial cells, kidney podocytes, and ureteral epithelial cells were cultured together with corisin, a corisin-like peptide, or a scrambled peptide, wherein cleaved caspase-3 positive cells are shown; also included are graphs of the ratio of the caspase-3 positive cells to the β-actin positive cells serving as the control.

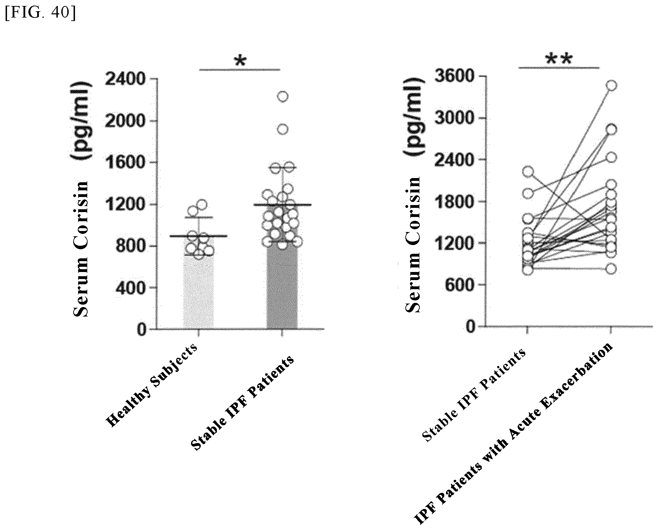

[FIG. 40] FIG. 40 includes graphs comparing corisin concentrations in serum in healthy subjects, stable sudden pulmonary fibrosis (IPF) patients, and sudden pulmonary fibrosis (IPF) patients with acute exacerbation. Corisin was measured by enzyme immunoassay using rabbit polyclonal anti-transglycosylase and antibody rat monoclonal anticorisin antibody. Healthy subjects, n = 8; IPF patients, n = 22; IPF patients with acute exacerbation, n = 22. Bars indicate mean±SD. Statistical analysis was performed by t-test. *p <0.05; **p <0.001

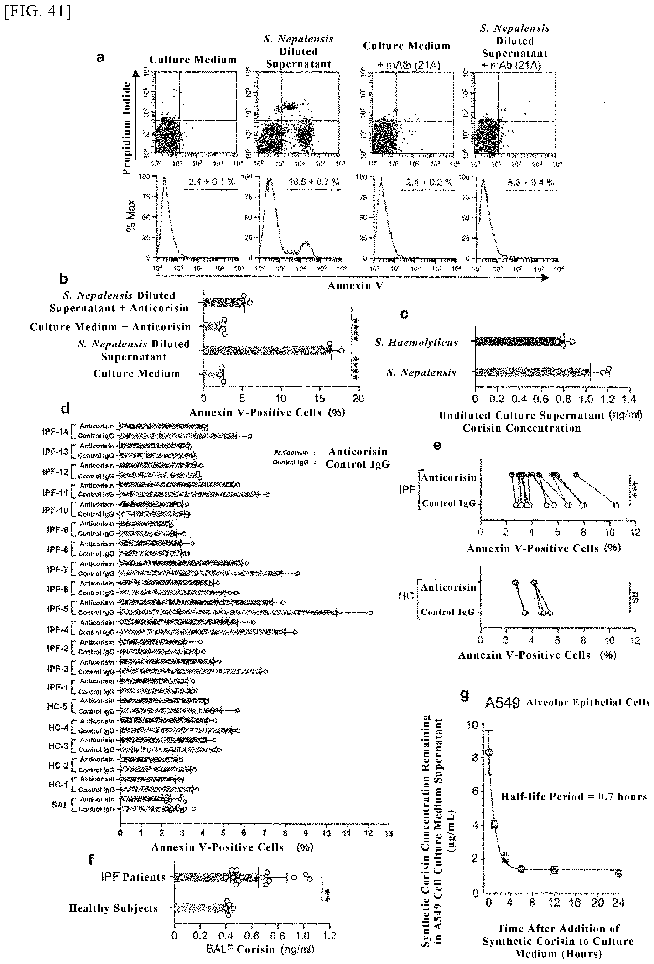

[FIG. 41] FIG. 41a and b: Flow cytometry examination results showing the results of culturing A549 alveolar epithelial cells in the presence of Staphylococcus nepalensis culture supernatant (1:10 dilution) and an anticorisin antibody and evaluation of apoptosis. Bars indicate mean±SD. Statistical analysis was performed using ANOVA method and Newman-Keuls test. ****p <0.0001. FIG. 41c: A graph measuring the concentration of native corisin in undiluted culture supernatants of Staphylococcus nepalensis and Staphylococcus haemolyticus. Bars indicate mean±SD. FIG. 41d: Flow cytometry examination results showing the results of culturing A549 alveolar epithelial cells in the presence of bronchoalveolar lavage fluid (BALF) (1:2 dilution) from healthy subjects (HC; n = 5 in the drawing) and patients with acute exacerbation of idiopathic pulmonary fibrosis (IPF; n = 14 in the drawing), the anticorisin neutralizing antibody (clone 21A) (anticorisin in the drawing) or control IgG (control IgG in the drawing) and evaluating apoptosis. Bars indicate mean±SD. FIG. 41e: Results of comparing the ratio of apoptotic cells induced by BALF in all the healthy subjects (n = 5) and IPF patients (n = 14) in FIG. 41d between the anticorisin antibody group and control IgG group. Bars indicate mean±SD. Statistical analysis was performed using the Wilcoxon test. ***p<0.001. ns, no significant difference. FIG. 41f: Graph showing concentrations of native corisin in undiluted BALF from healthy subjects (n = 5) and IPF patients (n = 14). Bars indicate mean±SD. **p <0.01. FIG. 41g: Graph of corisin concentration measurements taken after culturing A549 alveolar epithelial cells, adding synthetic corisin to the cell culture medium at a final concentration of 10 µg/mL, and collecting the culture medium after 0, 1, 3, 6, 12, and 24 hours. Shows in vitro half-life of results corisin.

### [Embodiments of the Invention]

### <Monoclonal antibody molecule>

One embodiment of the present invention relates to an isolated monoclonal antibody molecule or a derivative thereof that specifically binds to corisin.

In the present invention, corisin generally refers to a staphylococcal pro-apoptotic peptide that has been found to induce acute exacerbation of pulmonary fibrosis. Corisin may have one amino acid sequence such as, for example, IVMPESSGNPNAVNPAGYR (SEQ ID NO: 1), IVMPESGGNPNAVNPAGYR (SEQ ID NO: 58), IIMPESGGNPNIVNPYGYS (SEQ ID NO: 59), IVMPESGGNPNAVNPYGYR (SEQ ID NO: 60), IVLPESSGNPNAVNPAGYR (SEQ ID NO: 61), IVLPESSGNPNAVNELGYR (SEQ ID NO: 62), IVMPESGGNPNAVNELGYR (SEQ ID NO: 63), IVMPESSGNPNAVNELGYR (SEQ ID NO: 64), IVMPESSGNPDAVNELGYR (SEQ ID NO: 65), IAQRESGGDLKAVNPSSGA (SEQ ID NO: 66), or IAERESGGDLKAVNPSSGA (SEQ ID NO: 67). Preferably, corisin is a peptide having the amino acid sequence IVMPESSGNPNAVNPAGYR (SEQ ID NO: 1).

The sequence of SEQ ID NO: 1 may originate from Staphylococcus nepalensis. The origin of the sequences of other SEQ ID NOs are noted below with reference to FIGS. 36, 37, and 38:

| | |
|---|---|
| - SEQ ID NO: 58: wp022791177 (top row 236): one | |
| - SEQ ID NO: 59: wp049409534 | wp103371985 (top row 248): two |
| - SEQ ID NO: 60: wp103328722 | wp126565453 (top row 239, 244): two |
| - SEQ ID NO: 61: wp069827045 | wp099091381 (top row 246): two |
| - SEQ ID NO: 62: wp061853755 (top row 244): one | |
| - SEQ ID NO: 63: suk04795wp105995336 (top row 116): two | |
| - SEQ ID NO: 64: wp112369066 (top row 252): one | |
| - SEQ ID NO: 65: wp099090334 (top row 120): one | |
| - SEQ ID NO: 66: wp107644182 to wp119486153 (top row 222 to 223): 92 | |
| - SEQ ID NO: 66: wp096808177 to CNDG00159 (top row 229): two | |

Conservation of corisin was revealed by phylogenetic analysis. To reveal the evolutionary relationships of transglycosylases expressed by different bacteria, a phylogenetic tree was constructed according to a public database (www.ncbi.nlm.nih.gov/pubmed) based on the amino acid sequences of six transglycosylases identified in the genome of Staphylococcus nepalensis strain CNDG and their homologs. The topology of the phylogenetic tree indicates that a derivative of the transglycosylase close to the ancestral sequence split into two IsaA clusters (IsaA-1 and IsaA-2), and proteins known as SceD members likely evolved from IsaA-1 related sequences (SceD-1, SceD-2, SceD-3, SceD-4). Multiple alignments of the IsaA and SceD amino acid sequences generally revealed conservation of amino acid residues representing pro-apoptotic corisin, highlighting their functional significance (FIGS. 36, 37, and 38). The amino acid sequence identity of corisin homologous transglycosylases derived from Staphylococcus xylosus (Staphylococcus xylosus), Staphylococcus cohnii (Staphylococcus cohnii), and Staphylococcus nepalensis was 100%. Furthermore, these Staphylococci shared greater 98% or greater identity with the corresponding corisin regions of transglycosylases from other members of the IsaA-1 and IsaA-2 clusters and 60% identity with the corresponding regions of members of the SceD cluster (FIGS. 36, 37, and 38). Genomic constructs of gene clustering around transglycosylases (synteny) tended to be conserved in Staphylococcus cohnii and Staphylococcus nepalensis.

One embodiment of the present invention relates to a monoclonal antibody molecule or a derivative thereof of the present invention, wherein an amino acid having an amino acid sequence of PESSGNP (SEQ ID NO: 68) or NPAGY (SEQ ID NO: 69) is the epitope. In the present invention, an epitope having an amino acid sequence of PESSGNP (SEQ ID NO: 68) or NPAGY (SEQ ID NO: 69) was discovered (FIG. 12) by developing various anticorisin monoclonal antibodies having binding properties to a corisin molecule and performing epitope mapping for identifying the binding site thereof. It is conceivable that a monoclonal antibody molecule or a derivative thereof targeting this epitope has comparable binding properties to the applicable corisin and comparable functionality, for example, apoptosis-inhibiting and/or neutralizing activity. Accordingly, a preferred monoclonal antibody molecule or a derivative thereof of the present invention has neutralizing activity against corisin. This includes neutralizing antibodies.

One specific embodiment of the present invention relates to a series A monoclonal antibody molecule or a derivative thereof that identifies the monoclonal antibody of the present invention by the CDR, including:
(IA)
   (i) an H chain variable region including an H chain variable region CDR1 amino acid sequence of SEQ ID NO: 37, an H chain variable region CDR2 amino acid sequence of SEQ ID NO: 38, and an H chain variable region CDR3 amino acid sequence of SEQ ID NO: 39; and
   (ii) an L chain variable region including an L chain variable region CDR1 amino acid sequence of SEQ ID NO: 41, an L chain variable region CDR2 amino acid sequence of SEQ ID NO: 42, and an L chain variable region CDR3 amino acid sequence of SEQ ID NO: 43; (IIA);
   (i) an H chain variable region including an H chain variable region CDR1 amino acid sequence of SEQ ID NO: 70, an H chain variable region CDR2 amino acid sequence of SEQ ID NO: 49, and an H chain variable region CDR3 amino acid sequence of SEQ ID NO: 50; and
   (ii) an L chain variable region including an L chain variable region CDR1 amino acid sequence of SEQ ID NO: 52, an L chain variable region CDR2 amino acid sequence of SEQ ID NO: 53, and an L chain variable region CDR3 amino acid sequence of SEQ ID NO: 54;
   or
(IVA)
   (i) an H chain variable region including an H chain variable region CDR1 amino acid sequence of SEQ ID NO: 72, an H chain variable region CDR2 amino acid sequence of SEQ ID NO: 73, and an H chain variable region CDR3 amino acid sequence of SEQ ID NO: 74; and
   (ii) an L chain variable region including an L chain variable region CDR1 amino acid sequence of SEQ ID NO: 76, an L chain variable region CDR2 amino acid sequence of SEQ ID NO: 77, and an L chain variable region CDR3 amino acid sequence of SEQ ID NO: 78.

In the present invention, "monoclonal antibody molecule" means an immunoglobulin molecule having a tetrapeptide chain structure, wherein two identical H chains (heavy chains) and two identical L chains (light chains) are mutually bonded by disulfide bonds. The approximately 110 amino acid sequences flanking the N-termini of antibody H chains and L chains are highly variable and are known as variable regions (Fv regions). The amino acid sequence of the remaining portion near the C-terminus is relatively stable and is known as the constant region. The variable region has three hypervariable regions (HVR) and four relatively conserved framework regions (FR). Three hypervariable regions determine the specificity of an antibody and are also known as complementarity determining regions (CDRs). Each L chain variable region (LCVR) and each H chain variable region (HCVR) is composed of three CDR regions and four FR regions in the consecutive order of FR1, CDR1, FR2, CDR2, FR3, CDR3, and FR4 from the amino terminus to the carboxyl terminus. The three L chain CDRs refer to LCDR1, LCDR2, and LCDR3 and the three H chain CDRs refer to HCDR1, HCDR2, and HCDR3. Antibody molecules include non-human antibody molecules, humanized antibody molecules and human antibody molecules.

In the present invention, "isolated" means a specimen isolated from its original native environment. For example, a naturally occurring polynucleotide or polypeptide present in a living animal is not isolated, but the same polynucleotide or polypeptide that has been artificially separated from one portion or all of the coexisting specimens in the natural system is isolated.

In the present invention, "a derivative of the monoclonal antibody molecule" means a diabody and single chain molecule, as well as an "antigen-binding fragment". "Antigen-binding fragment" refers to Fab fragments, Fab' fragments, and F(ab')2 fragments having antigen-binding activity, and Fv fragments and scFv fragments binding to corisin, and may include one or more CDR regions in the antibodies defined by the present invention. Fv fragments are the smallest antibody fragments that include the H chain variable regions, the L chain variable regions, and all antigen-binding sites excluding the constant regions. Generally, Fv antibodies may further include a polypeptide linker between the H chain variable region and L chain variable region domains to form the necessary structure for antigen binding. Furthermore, different linkers are used to join the variable regions of two antibodies, forming polypeptides termed single-chain antibodies or single-chain Fv (scFv). In the present specification, the "antigen-binding site" of the present invention refers to a discontinuous three-dimensional site on an antigen that is recognized by an antibody or antigen-binding fragment of the present invention. In the present specification, "specifically recognizing corisin" means having the ability to bind to corisin, which is synonymous with "an antibody that binds to corisin" or "an antibody against corisin".

One further specific embodiment of the present invention relates to a series B monoclonal antibody molecule or a derivative thereof that identifies the monoclonal antibody of the present invention by H chain variable region and mutant type, including:
(IB) an H chain variable region including an H chain variable region amino acid sequence of SEQ ID NO: 36 or an amino acid sequence that is at least 80% identical thereto, or an H chain variable region including at least one framework (FW) region including an amino acid sequence having a substitution, deletion, insertion, or addition of one or several amino acid residues in comparison to the H chain variable region amino acid sequence of SEQ ID NO: 36;
(IIB) an H chain variable region including an H chain variable region amino acid sequence of SEQ ID NO: 48 or an amino acid sequence that is at least 80% identical thereto, or an H chain variable region including at least one framework (FW) region including an amino acid sequence having a substitution, deletion, insertion, or addition of one or several amino acid residues in comparison to the H chain variable region amino acid sequence of SEQ ID NO: 48; or
(IVB) an H chain variable region including an H chain variable region amino acid sequence of SEQ ID NO: 71 or an amino acid sequence that is at least 80% identical thereto, or an H chain variable region including at least one framework (FW) region including an amino acid sequence having a substitution, deletion, insertion, or addition of one or several amino acid residues in comparison to the H chain variable region amino acid sequence of SEQ ID NO: 71.

In the present invention, a mutated monoclonal antibody molecule or a derivative thereof has binding properties and functions equivalent to those of the original monoclonal antibody molecule without mutation.

One further specific embodiment of the present invention relates to a series C monoclonal antibody molecule or a derivative thereof that identifies the monoclonal antibody of the present invention by L chain variable region and mutant type, including:
(IC) an L chain variable region including an L chain variable region amino acid sequence of SEQ ID NO: 40 or an amino acid sequence that is at least 80% identical thereto, or an L chain variable region including at least one framework region including an amino acid sequence having a substitution, deletion, insertion, or addition of one or several amino acid residues in comparison to the L chain variable region amino acid sequence of SEQ ID NO: 40;
(IIC) an L chain variable region including an L chain variable region amino acid sequence of SEQ ID NO: 51 or an amino acid sequence that is at least 80% identical thereto, or an L chain variable region including at least one framework region including an amino acid sequence having a substitution, deletion, insertion, or addition of one or several amino acid residues in comparison to the L chain variable region amino acid sequence of SEQ ID NO: 51;
(IIIC) an L chain variable region including an L chain variable region amino acid sequence of SEQ ID NO: 44 or an amino acid sequence that is at least 80% identical thereto, or an L chain variable region including at least one framework region including an amino acid sequence having a substitution, deletion, insertion, or addition of one or several amino acid residues in comparison to the L chain variable region amino acid sequence of SEQ ID NO: 44; or
(IVC) an L chain variable region including an L chain variable region amino acid sequence of SEQ ID NO: 75 or an amino acid sequence that is at least 80% identical thereto, or an L chain variable region including at least one framework region including an amino acid sequence having a substitution, deletion, insertion, or addition of one or several amino acid residues in comparison to the L chain variable region amino acid sequence of SEQ ID NO: 75.

In the present invention, a mutated monoclonal antibody molecule or a derivative thereof has binding properties and functions equivalent to those of the original monoclonal antibody molecule without mutation.

One further specific embodiment of the present invention relates to the monoclonal antibody molecule or a derivative thereof of the present invention, having:
any H chain variable region of the present invention and any L chain variable region of the present invention. The preferred combinations of the H chain and L chain variable regions are combinations of isolated monoclonal antibody clones 4A, 9A, 2A, and 21A, respectively. Specifically, the combinations are as follows.

Anticorisin monoclonal antibody clone 4A: Monoclonal antibody (IA), (IB), (IC) related:
H chain (4A-HC.7331): SEQ ID NO: 36 
evqvlesggglvqpgnslklscatsGFTFSTAWmywyrqfpekrlewvarIKAKSNSYATdytesvkgr

ftisrddskgsiylrmnnlkeedtaiyycASTDAFYFSHSYwgqgvlvtvss,
 and
L chain (4A-LC.7395): SEQ ID NO: 40

Anticorisin monoclonal antibody clone 9A: Monoclonal antibody (IIIC) related:
L chain (9A-LC.7402): SEQ ID NO: 44

Anticorisin monoclonal antibody clone 2A: Monoclonal antibody (IIA), (IIB), (IIC) related:
H chain (2A-HC.7352): SEQ ID NO: 48 
evklvesggglvqpgnsltlscvasGFTFTNYGmhwirqapkkglewiamIYYDSSKMsyadtvkgrfti
srdnskntlylemnslrsedtamyycAAEGFGTPFPYwgqgtlvtvss,
 and
L chain (2A-LC.7413): SEQ ID NO: 51

Anticorisin monoclonal antibody clone 21A: Monoclonal antibody (IVA), (IVB), (IVC) related:
H chain (KK1410-2_VH_pep): SEQ ID NO: 71 evklvesggglvqpgnsltlscgasgftftNYGMHwirqapkkglewigMIYYDSSKMSYADTVKGr ftisrdnsknilylemnslrsedtamyycaaEGFGTPFPYwgqgtlvtvss, and
L chain (KK1410-2_VL_pep): SEQ ID NO: 75

As noted above, the monoclonal antibody molecule of the present invention may have any H chain variable region and/or any L chain variable region. In (IB), (IIB), (IVB), (IC), (IIC), (IIIC), and (IVC), 80% is preferably 85%, more preferably 90%, even more preferably 95%. H chain variable regions and L chain variable regions are mutants from which more conserved regions named "framework regions" (FR or FW) can be subdivided into interspersed regions of high frequency variability named "complementarity determining regions" (CDR).

In some embodiments, the L chain or H chain variable framework of the monoclonal antibody molecule against corisin may include an L chain or H chain variable framework containing at least 80%, 85%, 87%, 90%, 93%, 95%, 97%, or 99% of L chain or H chain variable framework residues derived from the human consensus sequence.

One further specific embodiment of the present invention relates to the mutant monoclonal antibody molecule or a derivative thereof of the present invention, having any mutant H chain variable region of the present invention and any mutant L chain variable region of the present invention. The preferred combinations of the mutant H chain and mutant L chain variable regions are combinations of isolated monoclonal antibody clones 4A, 9A, 2A, and 21A, respectively. Specifically, the combinations are as follows.

Anticorisin monoclonal antibody clone 4A: Monoclonal antibody (IA), (IB), (IC) related:
H chain: SEQ ID NO: 36
an H chain variable region including an amino acid sequence that is at least 80% identical to the H chain variable region amino acid sequence of evqvlesggglvqpgnslklscatsGFTF S T A WmywyrqfpekrlewvarIKAKSNSY A Tdytesvkgrftis rddskgsiylrmnnlkeedtaiyycASTDAFYFSHSYwgqgvlvtvss, and
L chain: SEQ ID NO: 40
an L chain variable region including an amino acid sequence that is at least 80% identical to the L chain variable region amino acid sequence of di vmtqtpssq avsagekvtmrcrssQ SLL YSENKKNYlawyq q kpgrspklli y WAS Tgesgvpdrfigsgs gtdftltissvqaedlavyycQQYYNFPLTfgsgtkleik

Anticorisin monoclonal antibody clone 2A: Monoclonal antibody (IIA), (IIB), (IIC) related:
H chain: SEQ ID NO: 48
an H chain variable region including an amino acid sequence that is at least 80% identical to the H chain variable region amino acid sequence of ev kl ves gggl vqp gn sl tl scvasG FTF TNY Gmhwi rq ap kkgl ewi amIYYD S SKMsy adtv kgrfti srd nskntlylemnslrsedtamyycAAEGFGTPFPYwgqgtlvtvss, and
L chain: SEQ ID NO: 51
an L chain variable region including an amino acid sequence that is at least 80% identical to the L chain variable region amino acid sequence of divmtqtpssravsagekvtmsckssQSLLYSENEKNYlawyqqrpgqspklliyWAStresgvpdrfigtgsg tdftltissvqaedlavyycQQYYHFPRTfgggtrlelk

Anticorisin monoclonal antibody clone 21A: Monoclonal antibody (IVA), (IVB), (IVC) related:
H chain: SEQ ID NO: 71
an H chain variable region including an amino acid sequence that is at least 80% identical to the H chain variable region amino acid sequence of evkl vesgggl vqpgnsl tl scgasgftftNYGMHwirq apkkglewigMIYYD S SKMS Y ADTVKGrfti s rdnsknilylemnslrsedtamyycaaEGFGTPFPYwgqgtlvtvss, and
L chain: SEQ ID NO: 75
an H chain variable region including an amino acid sequence that is at least 80% identical to the H chain variable region amino acid sequence of divmtqtpssqavsagekvtmscKSSQSLLYREDTKNYLAwyqqrpgqspklliyWASTRESgvpdrfi gs gsgtdftl ti ssvq aedl avyy cQ Q YYHFPR Tfgggtrl elk.

One further specific embodiment of the present invention relates to the mutant monoclonal antibody molecule or a derivative thereof of the present invention, having any mutant H chain variable region of the present invention and any mutant L chain variable region of the present invention. The preferred combinations of the mutant H chain and mutant L chain variable regions are combinations of isolated monoclonal antibody clones 4A, 9A, 2A, and 21A, respectively. Specifically, the combinations are as follows.

Anticorisin monoclonal antibody clone 4A: Monoclonal antibody (IA), (IB), (IC) related:
H chain: SEQ ID NO: 36
an H chain variable region including at least one framework (FW) region including an amino acid sequence having a substitution, deletion, insertion, or addition of one or several amino acid residues in comparison to the H chain variable region amino acid sequence of evqvlesggglvqpgnslklscatsGFTFSTAWmywyrqfpekrlewvarIKAKSNSYATdytesvkgrftis rddskgsiylrmnnlkeedtaiyycASTDAFYFSHSYwgqgvlvtvss, and
L chain: SEQ ID NO: 40
an L chain variable region including at least one framework (FW) region including an amino acid sequence having a substitution, deletion, insertion, or addition of one or several amino acid residues in comparison to the L chain variable region amino acid sequence of di vmtqtpssq avsagekvtmrcrssQ SLL YSENKKNYlawyq q kpgrspklli y WAS Tgesgvpdrfigsgs gtdftltissvqaedlavyycQQYYNFPLTfgsgtkleik

Anticorisin monoclonal antibody clone 2A: Monoclonal antibody (IIA), (IIB), (IIC) related:
H chain: SEQ ID NO: 48
an H chain variable region including at least one framework (FW) region including an amino acid sequence having a substitution, deletion, insertion, or addition of one or several amino acid residues in comparison to the H chain variable region amino acid sequence of ev kl ves gggl vqp gn sl tl scvasG FTF TNY Gmhwi rq ap kkgl ewi amIYYD S SKMsy adtv kgrfti srd nskntlylemnslrsedtamyycAAEGFGTPFPYwgqgtlvtvss, and
L chain: SEQ ID NO: 51
an L chain variable region including at least one framework (FW) region including an amino acid sequence having a substitution, deletion, insertion, or addition of one or several amino acid residues in comparison to the L chain variable region amino acid sequence of divmtqtpssravsagekvtmsckssQSLLYSENEKNYlawyqqrpgqspklliyWAStresgvpdrfigtgsg tdftltissvqaedlavyycQQYYHFPRTfgggtrlelk

Anticorisin monoclonal antibody clone 21A: Monoclonal antibody (IVA), (IVB), (IVC) related:
H chain: SEQ ID NO: 71
an H chain variable region including at least one framework (FW) region including an amino acid sequence having a substitution, deletion, insertion, or addition of one or several amino acid residues in comparison to the H chain variable region amino acid sequence of evkl vesgggl vqpgnsl tl scgasgftftNYGMHwirq apkkglewigMIYYD S SKMS Y ADTVKGrfti s rdnsknilylemnslrsedtamyycaaEGFGTPFPYwgqgtlvtvss, and
L chain: SEQ ID NO: 75
an L chain variable region including at least one framework (FW) region including an amino acid sequence having a substitution, deletion, insertion, or addition of one or several amino acid residues in comparison to the L chain variable region amino acid sequence of divmtqtpssqavsagekvtmscKSSQSLLYREDTKNYLAwyqqrpgqspklliyWASTRESgvpdrfi gs gsgtdftl ti ssvq aedl avyy cQ Q YYHFPR Tfgggtrl elk.

The monoclonal antibody molecule of the present invention may be an antibody produced by hybridomas or a genetically engineered antibody produced by transformed cells transformed by an expression vector containing antibody genes. The monoclonal antibody molecule of the present invention, the monoclonal antibody molecule of the present invention having any H chain variable region of the present invention and any L chain variable region of the present invention, and a mutant type monoclonal antibody molecule may be prepared according to a known method in the field of the art. For example, a nucleic acid molecule encoding an antibody molecule of the present invention is incorporated into a vector that may be introduced into a host cell. Since the antibody molecule of the present invention always contains at least two polypeptide chains, the nucleic acids encoding those chains may be present either in a single vector or in two or more vectors. When two or more vectors are used, these vectors may be introduced together into the host cell. Vectors and host cells containing nucleic acids encoding such antibody molecules may be cultured under conditions that allow protein expression. The expressed antibody molecules may then be obtained from the culture medium in which the cells were cultured or from the cells themselves, and purified by any of a number of suitable means known in the field of the art.

In the present invention, corisin-induced apoptosis occurs through activation of the extrinsic or intrinsic pathway (Reference Literature 25). The extrinsic pathway is mediated by membrane-bound death receptors that transmit an intracellular death signal through their intracellular death domain after ligand binding and procaspase-8 cleavage to caspase-8 (FIG. 5a) (Reference Literature 25). The intrinsic pathway is regulated by mitochondria. Pro-apoptotic stimuli cause mitochondrial perturbations that increase mitochondrial membrane permeability, causing the release of suppressors of baculoviral inhibitor of apoptosis repeat-containing (BIRC) proteins and pro-apoptotic factors, and these contribute to apoptosis formation and cleave procaspase-9 to caspase-9 (Reference Literature 26, 27). The cleaved forms of caspase-8 and caspase-9 activate caspase-3, an executing factor of apoptosis (FIG. 5a). Using these series of apoptotic pathways, apoptotic activity may be examined by various well-known methods. The antibody molecule or a derivative thereof of the present invention is preferably a corisin-neutralizing antibody molecule or a derivative thereof, and more preferably an antibody molecule or a derivative thereof that neutralizes and/or inhibits corisin-induced apoptosis.

### <Pharmaceutical composition>

One embodiment of the present invention relates to a pharmaceutical composition for treating or preventing acute lung injury or fibrosis, wherein the pharmaceutical composition contains an effective dose of the monoclonal antibody molecule or a derivative thereof of the present invention.

In the present invention, the acute lung injury is fatal pneumonia that causes acute respiratory distress syndrome (ARDS), reduces lung function, and requires respiratory care. Significant human lung injuries including COVID-19 and severe acute respiratory syndrome (SARS) are characterized by acute lung injury with sequelae of pulmonary fibrosis (with sequelae of pulmonary fibrosis).

In the present invention, fibrosis includes idiopathic pulmonary fibrosis (IPF), liver cirrhosis, renal fibrosis, cystic fibrosis, myelofibrosis and/or fibrous disease of the breast, and the like. In particular, idiopathic pulmonary fibrosis is an incurable disease of unknown etiology. Increased apoptosis of alveolar epithelial cells and subsequent aberrant lung tissue repair are central to its progression. The inventors of the present invention discovered the present invention by identifying a peptide, corisin, that induces apoptosis of alveolar epithelial cells and an acute exacerbation of pulmonary fibrosis. The monoclonal antibody molecule or a derivative thereof of the present invention may be a neutralizing antibody, for example, an antibody that blocks or inhibits the negative effects of corisin in the lungs or other tissues of patients suffering from fibrosis.

In the present invention, "treat" means a method or process with the purpose of (1) delaying or preventing the onset of a disease or condition; (2) slowing or stopping the progression, exacerbation, or worsening of the symptoms of a disease or condition; (3) causing remission of the symptoms of a disease or condition; or (4) curing a disease or condition. Treatment may be administered prior to the onset of a disease or condition as a prophylactic measure, or alternatively treatment may be administered after the onset of a disease.

In the present invention, "prevent" means preventing the onset of inflammatory and/or fibrotic conditions.

In the present invention, "pharmaceutical composition" generally means a pharmaceutical preparation for dealing with, treating, or preventing a disease or pathological condition, or for examination and diagnosis.

"Subject" or "patient" as used in the present specification means an animal which is subject to treatment, observation, or testing. By way of example only, the subject may be a mammal, including humans. An antibody against corisin, preferably for humans, may be provided alone or as a pharmaceutical composition. To achieve this purpose, a compound may be manufactured into various pharmaceutical dosage forms according to the prophylactic or therapeutic purpose. Examples of pharmaceutical dosage forms are oral formulations, injections, suppositories, ointments, salves, and the like. Such dosage forms are well known to persons having ordinary skill in the art and may be formulated by a conventional method.

In the present invention, the "pharmaceutical composition" contains one or more antibodies against corisin, including one or more other chemical ingredients such as a stabilizer, diluent, dispersing agent, suspending agent, thickener and/or excipient, and the like. The pharmaceutical composition facilitates administration of antibodies against corisin to an organism. Several techniques of administering antibodies against corisin are known, for example, oral administration, or parenteral administration such as topical administration, intravenous administration, aerosol administration, eye drops, and the like.

The term "diluent" refers to a compound used to dilute the desired pharmaceutical preparation (for example, an antibody against corisin) before transport. Diluents may also be used for stabilizers as they can provide a more stable environment. Salts dissolved in buffers capable of controlling or maintaining pH are useful as diluents, and these include phosphate-buffered saline.

"Effective dose" or "therapeutic effective dose" means a sufficient amount of the antibody against the corisin of the present invention administered to relieve one or more degrees of symptoms of the disease, disorder, or condition to be treated. As a result, the signs, symptoms, or causes of disease may be attenuated and/or relieved. For example, the "effective dose" in therapeutic applications is the amount of antibodies against corisin which is necessary to provide a desired pharmacological effect, therapeutic improvement, or clinically significant reduction of disease symptoms without excessive side effects. The "effective dose" may vary for each subject based on the metabolism of antibodies against corisin, genetics, combinations, or age, weight, general condition, condition being treated, or severity of the condition being treated in the subject, and differences in the decision of the prescribing physician.

"Effective dose" includes a "prophylactic effective dose", and this means the amount of the antibody against corisin of the present invention that is applied to a patient to relieve one or more degrees of symptoms of a disease, condition, or disorder to be treated. In such prophylactic applications, such amounts may vary with the subject's health condition, weight, and the like.

The amount of antibody against corisin incorporated into each unit dosage form may vary depending on the subject's health condition or the type of formulation. Preferred amounts per unit dosage form are approximately 1 to 1,000 mg for oral administration and approximately 0.1 to 500 mg for injection. The daily dose varies according to the patient's symptoms, weight, age, sex, and other factors, and the usual range per day for adults is approximately 0.1 to 5,000 mg, and preferably approximately 1 to 1,000 mg. The formulation is preferably administered in a single dose or in two to four divided doses.

For formulating solid formulations for oral administration, excipients and, if necessary, binders, disintegrants, lubricants, colorants, corrective agents, flavoring agents, and the like are added to the anti-PD-1 formulation, and the formulation is formulated into tablets, coated tablets, granules, powders, capsules, or the like by conventional methods. Such additives are well known in the art, and useful examples include excipients such as lactose, sucrose, sodium chloride, starch, calcium carbonate, kaolin, microcrystalline cellulose, silicic acid, and the like, water, ethanol propanol, simple syrups, glucose solutions, binders such as starch solutions, gelatin solutions, carboxymethylcellulose, hydroxypropyl cellulose, hydroxypropyl starch, methylcellulose, ethylcellulose, shellac, calcium phosphate, polyvinylpyrrolidone, and the like, disintegrating agents such as dry starch, sodium alginate, agar powder, sodium bicarbonate, calcium carbonate, sodium lauryl sulfate, monoglyceride stearate, lactose, and the like, lubricating agents such as purified talc, stearates, sodium borate, polyethylene glycol, and the like, and corrective agents such as sucrose, orange peel, citric acid, tartaric acid, and the like.

To formulate liquid formulations for oral administration, corrective agents, buffering agents, stabilizers, flavoring agents, and the like are added to the compound,
and the mixture may be formulated into oral liquid formulations, syrups, elixirs, or the like by conventional methods. Examples of useful corrective agents are described above. Examples of buffering agents include sodium citrate and the like. Examples of stabilizers include tragacanth, gum arabic, gelatin, and the like. As a non-limiting embodiment, a syrup may be prepared by a conventional method using ingredients in the ratios indicated below.

Injections are prepared by conventional methods as subcutaneous, intramuscular, or intravenous injections by adding pH adjusters, buffering agents, stabilizers, isotonic agents, local anesthetics, and the like to antibodies against corisin. Examples of pH adjusters and buffering agents include sodium citrate, sodium acetate, sodium phosphate, and the like. Examples of stabilizers include sodium pyrosulfite, EDTA, thioglycolate, and the like. Examples of local anesthetics include procalin hydrochloride, lidocaine hydrochloride, and the like. Examples of isotonic agents include sodium chloride, glucose, and the like. As a non-limiting embodiment, an injection may be prepared by a conventional method using ingredients in the ratios indicated below.

Another aspect of the present embodiment of the present invention relates to a method for treating or preventing acute lung injury or fibrosis, wherein the method includes administering the monoclonal antibody molecule or a derivative thereof of the present invention to a subject in need of such treatment or the like, and preferably includes administering an effective dose of the monoclonal antibody molecule or a derivative thereof of the present invention to such a subject. Preferably, in the method of the present invention, the acute lung injury is fatal pneumonia that causes acute respiratory distress syndrome (ARDS), reduces pulmonary function, and necessitates respiratory care, or the fibrosis is selected from a group composed of idiopathic pulmonary fibrosis (IPF), liver cirrhosis, renal fibrosis, cystic fibrosis, myelofibrosis, and fibrous disease of the breast.

Moreover, another aspect of the present invention relates to the monoclonal antibody molecule or a derivative thereof of the present invention for treating or preventing acute lung injury or fibrosis. Preferably, in the monoclonal antibody molecule or a derivative thereof of the present invention, the acute lung injury is fatal pneumonia that causes acute respiratory distress syndrome (ARDS), reduces pulmonary function, and necessitates respiratory care, or the fibrosis is selected from a group composed of idiopathic pulmonary fibrosis (IPF), liver cirrhosis, renal fibrosis, cystic fibrosis, myelofibrosis, and fibrous disease of the breast.

Yet another aspect of the present invention relates to the monoclonal antibody molecule or a derivative thereof of the present invention for manufacturing a medicine for treating or preventing acute lung injury or fibrosis. Preferably, in the monoclonal antibody molecule or a derivative thereof of the present invention, the acute lung injury is fatal pneumonia that causes acute respiratory distress syndrome (ARDS), reduces pulmonary function, and necessitates respiratory care, or the fibrosis is selected from a group composed of idiopathic pulmonary fibrosis (IPF), liver cirrhosis, renal fibrosis, cystic fibrosis, myelofibrosis, and fibrous disease of the breast.

### <Vaccine>

Another embodiment of the present invention relates to a vaccine composition for preventing acute lung injury or fibrosis, having as an active ingredient a corisin mutant having one or several mutations in the sequence of corisin having the amino acid sequence: IVMPESSGNPNAVNPAGYR (SEQ ID NO: 1), wherein the mutant does not exhibit apoptotic activity. Preferred corisin mutants are corisin N14S: IVMPESSGNPNAVSPAGYR (SEQ ID NO: 55) and corisin P15A: IVMPESSGNPNAVNAAGYR (SEQ ID NO: 56).

In the present invention, "vaccine composition" means a biological formulation containing an antigen that induces a specific immune response (production of a specific antibody). In the present invention, the vaccine may contain a peptide that may be modified. The corisin mutant contained in the vaccine composition of the present invention has antigenicity and weakens the pro-apoptotic activity of corisin due to defined mutations or alterations to amino acid positions. That is, the peptide alters amino acids at different positions of the corisin peptide and does not induce apoptosis, but is sufficiently similar to corisin to induce an antibody that neutralizes the pro-apoptotic corisin peptide.

In the present invention, mutations include "substitution of an amino acid residue", "deletion of an amino acid residue", "insertion of an amino acid residue", or "addition of an amino acid residue". Examples of "substitution of an amino acid residue" include non-conservative amino acid substitution. "Non-conservative amino acid substitution" refers to substituting a defined amino acid with an amino acid including a side chain having different properties to the side chain of that amino acid. Specifically, in non-conservative amino acid substitution, a defined amino acid is substituted with another amino acid belonging to a different group to the defined amino acid. Groups of amino acids including side chains having different properties are well-known in the field. As an example of such, an amino acid of a different group to an amino acid group having the same properties, such as an amino acid having a basic side chain (for example, lysine, arginine, or histidine), an amino acid having an acidic side chain (for example, aspartic acid or glutamic acid), or an amino acid having a neutral side chain (for example, glycine, asparagine, glutamine, serine, threonine, tyrosine, cysteine, alanine, valine, leucine, isoleucine, proline, phenylalanine, methionine, or tryptophan), is selected.

Moreover, amino acids having neutral side chains may further be classified into amino acids having polar side chains (for example, asparagine, glutamine, serine, threonine, tyrosine, and cysteine) and amino acids having non-polar side chains (for example, glycine, alanine, valine, leucine, isoleucine, proline, phenylalanine, methionine, and tryptophan). In this case, too, an amino acid of a group having different properties is selected. Furthermore, in terms of the other group, for example, an amino acid having an aromatic side chain (for example, phenylalanine, tryptophan, or tyrosine), an amino acid having a side chain including a hydroxyl group (an alcoholic hydroxyl group or a phenolic hydroxyl group) (for example, serine, threonine, or tyrosine), or the like may also be used.

"Deletion of an amino acid residue" includes, for example, deletion of an arbitrary amino acid residue selected from among partial sequences of the corisin peptide. "Insertion of an amino acid residue" or "addition of an amino acid residue" involves, for example, inserting the amino acid residue into a partial sequence of the corisin peptide or the adding the amino acid residue to the N-terminus side or the C-terminus side of the partial sequence.

Another aspect of this embodiment of the present invention relates to a vaccine therapy for preventing acute lung injury or fibrosis, wherein the therapy includes administering a corisin mutant having one or several mutations in a sequence of corisin having an amino acid sequence: IVMPESSGNPNAVNPAGYR (SEQ ID NO: 1), the mutant not exhibiting apoptotic activity, to a subject in need of such treatment or the like; and preferably including administering an effective dose of the monoclonal antibody molecule or a derivative thereof of the present invention to such a subject. Preferred corisin mutants are corisin N14S: IVMPESSGNPNAVSPAGYR (SEQ ID NO: 55) and corisin P15A: IVMPESSGNPNAVNAAGYR (SEQ ID NO: 56).

Furthermore, another aspect of the present invention relates to a corisin mutant having one or several mutations in a sequence of corisin having the amino acid sequence: IVMPESSGNPNAVNPAGYR (SEQ ID NO: 1), wherein the mutant does not exhibit apoptotic activity, used in a vaccine therapy for preventing acute lung injury or fibrosis.

A further aspect of the present invention relates to a use of a corisin mutant having one or several mutations in a sequence of corisin having the amino acid sequence: IVMPESSGNPNAVNPAGYR (SEQ ID NO: 1), wherein the mutant does not exhibit apoptotic activity, for manufacturing a medicine used in a vaccine therapy for preventing acute lung injury or fibrosis.

### <Biomarker>

Another aspect of the present invention relates to a use of corisin as a biomarker for determining the stage of progression of acute lung injury or fibrosis. The use of corisin as a biomarker also encompasses evaluating or diagnosing a subject suspected of having or developing fibrosis. A biological sample is collected from a subject and an amount of corisin present in the biological sample is detected. The detected amount of corisin in the biological sample may be compared to a predetermined threshold of 1 or more. The predetermined threshold may be set based on, for example, the level of corisin normally present which is typical for healthy individuals.

The biological sample may be, for example, sputum, bronchial secretion, pleural effusion, bronchoalveolar lavage fluid (BALF), or tissue collected from the bronchi or lungs. The biological sample may be blood. Herein, corisin may have one amino acid sequence such as, for example, IVMPESSGNPNAVNPAGYR (SEQ ID NO: 1), IVMPESGGNPNAVNPAGYR (SEQ ID NO: 58), IIMPESGGNPNIVNPYGYS (SEQ ID NO: 59), IVMPESGGNPNAVNPYGYR (SEQ ID NO: 60), IVLPESSGNPNAVNPAGYR (SEQ ID NO: 61), IVLPESSGNPNAVNELGYR (SEQ ID NO: 62), IVMPESGGNPNAVNELGYR (SEQ ID NO: 63), IVMPESSGNPNAVNELGYR (SEQ ID NO: 64), IVMPESSGNPDAVNELGYR (SEQ ID NO: 65), IAQRESGGDLKAVNPSSGA (SEQ ID NO: 66), or IAERESGGDLKAVNPSSGA (SEQ ID NO: 67). Preferably, corisin is a peptide having the amino acid sequence IVMPESSGNPNAVNPAGYR (SEQ ID NO: 1).

Corisin may be detected by detection of corisin bound to an antibody which recognizes any of the amino acid sequences in mass spectrometry, Western blotting, or enzyme-linked immunosorbent assay (for example, for ELISA, for example, IVMPESSGNPNAVNPAGYR (SEQ ID NO: 1), IVMPESGGNPNAVNPAGYR (SEQ ID NO: 58), IIMPESGGNPNIVNPYGYS (SEQ ID NO: 59), IVMPESGGNPNAVNPYGYR (SEQ ID NO: 60), IVLPESSGNPNAVNPAGYR (SEQ ID NO: 61), IVLPESSGNPNAVNELGYR (SEQ ID NO: 62), IVMPESGGNPNAVNELGYR (SEQ ID NO: 63), IVMPESSGNPNAVNELGYR (SEQ ID NO: 64), IVMPESSGNPDAVNELGYR (SEQ ID NO: 65), IAQRESGGDLKAVNPSSGA (SEQ ID NO: 66), or IAERESGGDLKAVNPSSGA (SEQ ID NO: 67); for example, by binding a labeled antibody to an antibody which binds to a substrate, for example. Kits for implementing such methods may include such antibodies and one or more reagents for performing detection of corisin in a biological sample.

In such an aspect, the present invention provides a method for determining the stage of progression of fibrosis, the method including
(a10) a step of measuring an amount of corisin in a subject (test biomarker amount);
(b10) a step of comparing the test biomarker amount and a reference amount of corisin (control biomarker amount), and
(c10) determining that the stage of progression of acute lung injury or fibrosis of a subject is advanced when the test biomarker amount is greater than the control biomarker amount. The reference amount of corisin may be the amount of corisin in the same subject measured before step (a10), or may be the amount of corisin in myofibroblasts of a healthy subj ect.

The inventors of the present invention have found that corisin is a fibrosis-specific marker molecule that is associated with fibrotic diseases, particularly severe fibrotic diseases. The use of the methods, biomarkers and corisin of the present invention allows prediction of the stage of progression of acute lung injury or fibrosis and is beneficial for prevention or treatment of acute lung injury or fibrosis.

The correspondence between the three-letter notations and the one-letter notations for amino acids used in the present Specification is as follows.
Alanine: Ala: A
Arginine: Arg: R
Asparagine: Asn: N
Aspartic acid: Asp: D
Cysteine: Cys: C
Glutamine: Gln: Q
Glutamic acid: Glu: E
Glycine: Gly: G
Histidine: His: H
Isoleucine: Ile: I
Leucine: Leu: L
Lysine: Lys: K
Methionine: Met: M
Phenylalanine: Phe: F
Proline: Pro: P
Serine: Ser: S
Threonine: Thr: T
Tryptophan: Trp: W
Tyrosine: Tyr: Y
Valine: Val: V

Hereinafter, the present invention will be described in detail using reference examples and examples. These are merely examples, and it should be noted that various modifications to the present invention are possible within the scope of the invention stated in the Scope of Patent Claims, and that these are also included within the scope of the present invention.

### [Examples]

### Reference Result 1

### Broad Group of Pathogens having Pro-Apoptotic Corisin-Like Peptides

The inventors of the present invention have previously demonstrated that culture supernatant from a mixture of Staphylococcus nepalensis (Staphylococcus nepalensis) strain CNDG and Staphylococcus spp. (Staphylococcus spp.) proliferated in culture medium inoculated with pulmonary fibrosis specimens from terminally ill mice having pulmonary fibrosis induces apoptosis of alveolar epithelial cells (Non-Patent Literature 19). Furthermore, it was reported that a transglycosylase fragment referred to as corisin released by Staphylococcus nepalensis is responsible for lung epithelial cell apoptosis in established transgenic mice, leading to acute exacerbation of chronic pulmonary fibrosis (Non-Patent Literature 19). Since sequences of corisin or its derivatives are highly conserved among many members of the Staphylococcus family (Non-Patent Literature 19), other species of Staphylococcus, in addition to Staphylococcus nepalensis, also release corisin-like peptides from transglycosidases, and it was expected that this would induce apoptosis in lung epithelial cells.

In order to test this expectation, a mixture of Staphylococcus spp. proliferated in culture medium inoculated with pulmonary fibrosis specimens was streak plated, and individual bacteria colonies were isolated. The obtained 12 colonies were screened. Three colonies of strains 1, 7, and 12 secreted apoptotic factors, and no apoptotic activity was detected in the remaining isolates. These results indicate that the previously discovered culture of strain 6 (Non-Patent Literature 19) was a mixture of pro-apoptotic and non-apoptotic bacteria. Whole genome sequencing revealed that these three apoptotic bacterial isolates were distinct strains of Staphylococcus haemolyticus (Staphylococcus haemolyticus). Whole genome sequences of Staphylococcus haemolyticus strains 1, 7 and 12 have been deposited in the Genbank database with accession numbers allocated thereto. These genomic analyses demonstrated the presence of a corisin-like fragment conserved in strain 1 (transglycosylase_1bp_353), strain 7 (transglycosylase_7bp_350), and strain 12 (transglycosylase_12bp_350) (FIG. 1). The sequence of the corisin-like fragment was identical in the corresponding transglycosylase of each strain (Non-Patent Literature 19) (FIG. 1).

A549 alveolar epithelial cells were then cultured in the presence of a bacterial culture supernatant of each bacterial strain to evaluate apoptosis. The culture supernatant of each strain induced apoptosis of alveolar epithelial cells (FIG. 2a, b). Transmission electron microscopy confirmed an increase in apoptotic cells after treatment with culture supernatant of Staphylococcus haemolyticus strain 12 (FIG. 2c). A549 alveolar epithelial cells cultured in the presence of culture supernatants derived from each Staphylococcus haemolyticus strain also showed a significant increase in caspase-3 cleavage (FIG. 2d, e). A synthetic peptide of the Staphylococcus haemolyticus corisin-like sequence prepared by a manufacturer (ThermoFisher Scientific) differed only at a single position when compared to the published sequence (Non-Patent Literature 19), but this reproduced the pro-apoptotic effect of the supernatant of each strain (strains 1, 7, and 12) corresponding to A549 lung epithelial cells (FIG. 3a, b, c). Staphylococcus haemolyticus is a commensal bacterium, but it is also a well-known opportunistic and multi-drug resistant pathogen (Reference Literature 20). This bacterium grows more optimally under conditions of oxygen and salt (10% NaCl) (Reference Literature 21). Human infections caused by Staphylococcus haemolyticus include sepsis, peritonitis, endocarditis, meningitis, and wound, bone, and urinary tract infections. Interestingly, the corisin-like sequences of transglycosylase homologues derived from the Staphylococcus haemolyticus strain isolated herein are identical to the sequences observed in transglycosylases derived from Mycobacteroides abscessus subsp. abscessus (Genbank protein accession number SKR 69498.1) and Listeria monocytogenes (Genbank protein accession number ECO 1693478.1) strains (FIG. 4). Listeria monocytogenes is the most virulent foodborne pathogen (Reference Literature 22), and Mycobacteroides abscessus subsp. abscessus (Reference Literature 23) is a multi-drug resistant nontuberculous mycobacterium generally leading to chronic pulmonary infection. Homologous transglycosylases derived from other strains of Listeria monocytogenes and Mycobacteroides abscessus subsp. abscessus, and hypothetical proteins derived from Weissella confusa (Reference Literature 24), which is an opportunistic pathogen, also include corisin-like sequences having pro-apoptotic activity (FIG. 3a, b, c). These observations indicate that a wide range of pathogens carry derivatives of this toxic peptide.

### Reference Result 2

### Activation of Intracellular Apoptotic Pathways by Bacterial Culture Supernatant and Corisin

FIG. 5a shows the extrinsic and intrinsic pathways of apoptosis. A549 alveolar epithelial cells were cultured in the presence of (1/10 diluted) S. haemolyticus strain 12 culture supernatant for 24 hours. Percentages of cells positive for cleaved caspase-8, cleaved caspase-9, and cleaved caspase-3 were determined and quantified by flow cytometry. It was discovered that the S. haemolyticus culture supernatant significantly increased the number of alveolar epithelial cells having activated caspase-8 and activated caspase-3 compared to control stimulation (FIG. 5b).

Next, the effect of corisin on mRNA expression of markers for pro-apoptotic factors such as Bax and anti-apoptotic factors BIRC1, BIRC5, BIRC7, Bcl2, BclXL, cyclin D1, proliferating cell nuclear antigens, and proliferating Ki-67 were examined. A549 alveolar epithelial cells were cultured in the presence of 50 µM corisin for 24 hours, total mRNA was extracted, and cDNA was prepared and amplified by RT-PCR. As a result, it was also found that corisin significantly reduced mRNA expression of anti-apoptotic factors, and also significantly increased mRNA expression of pro-apoptotic factors such as Bax and apoptotic protease-activating factor 1 (APAF-1) compared to scrambled peptides (FIG. 6, Table 1). These results indicate that bacteria-derived peptides selectively activate the intrinsic pathway of apoptosis.

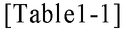

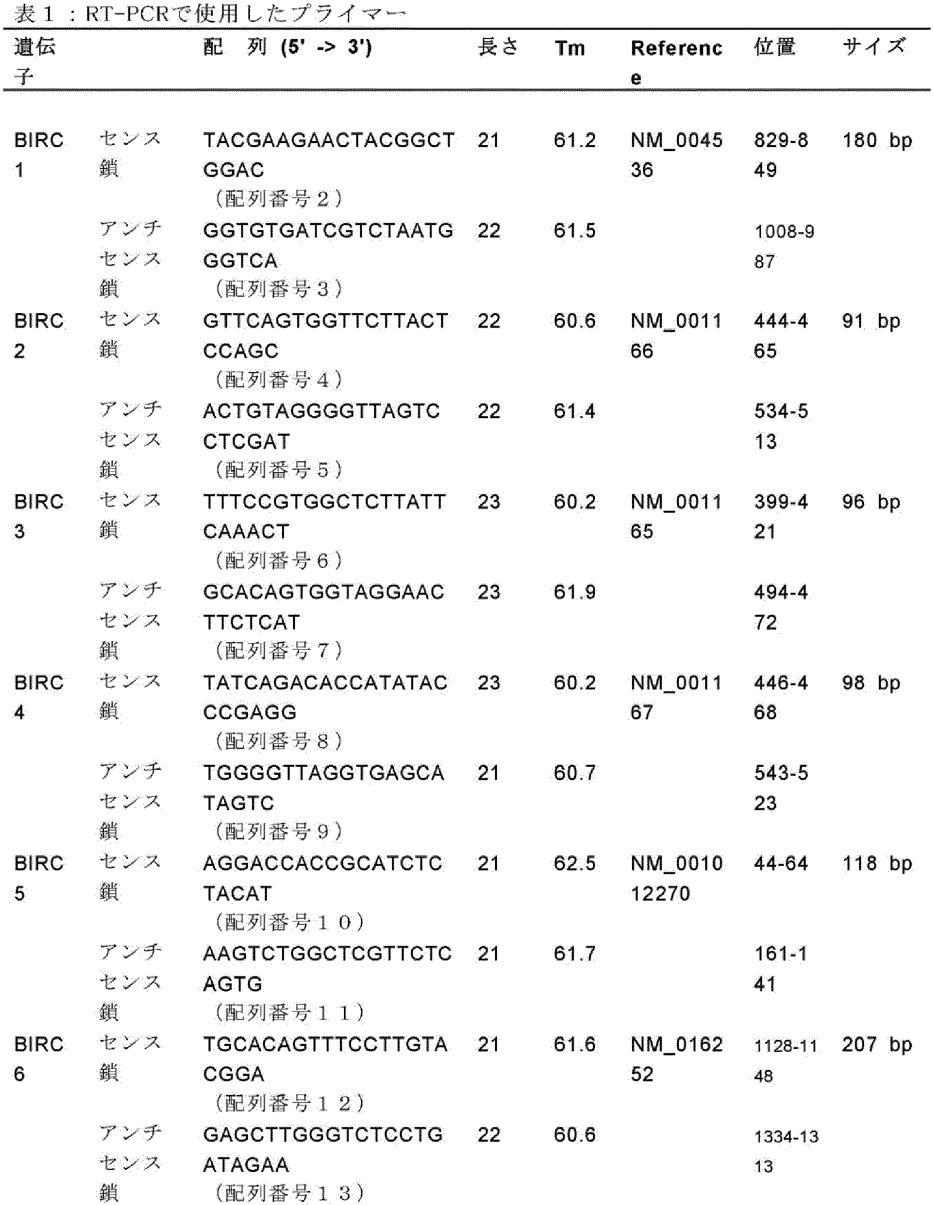

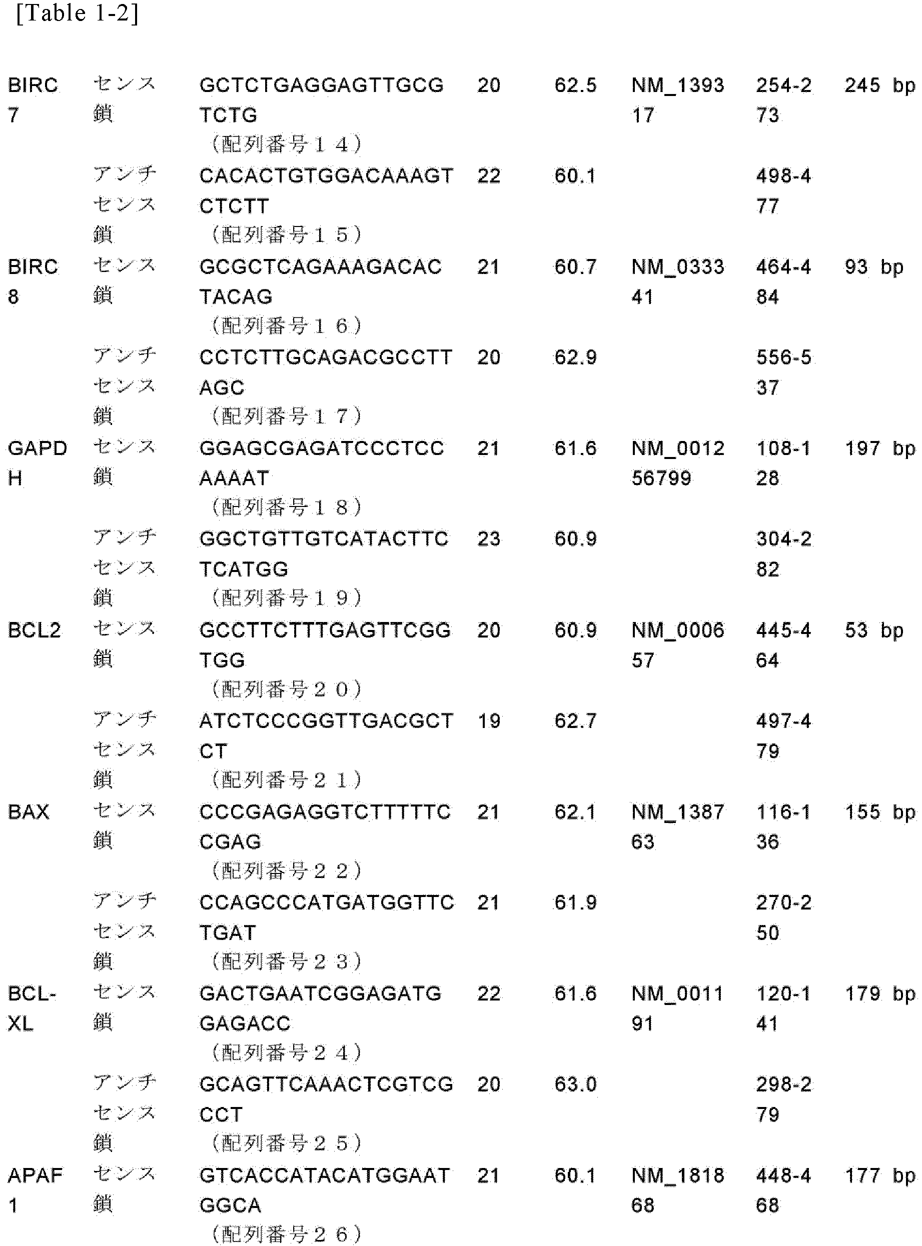

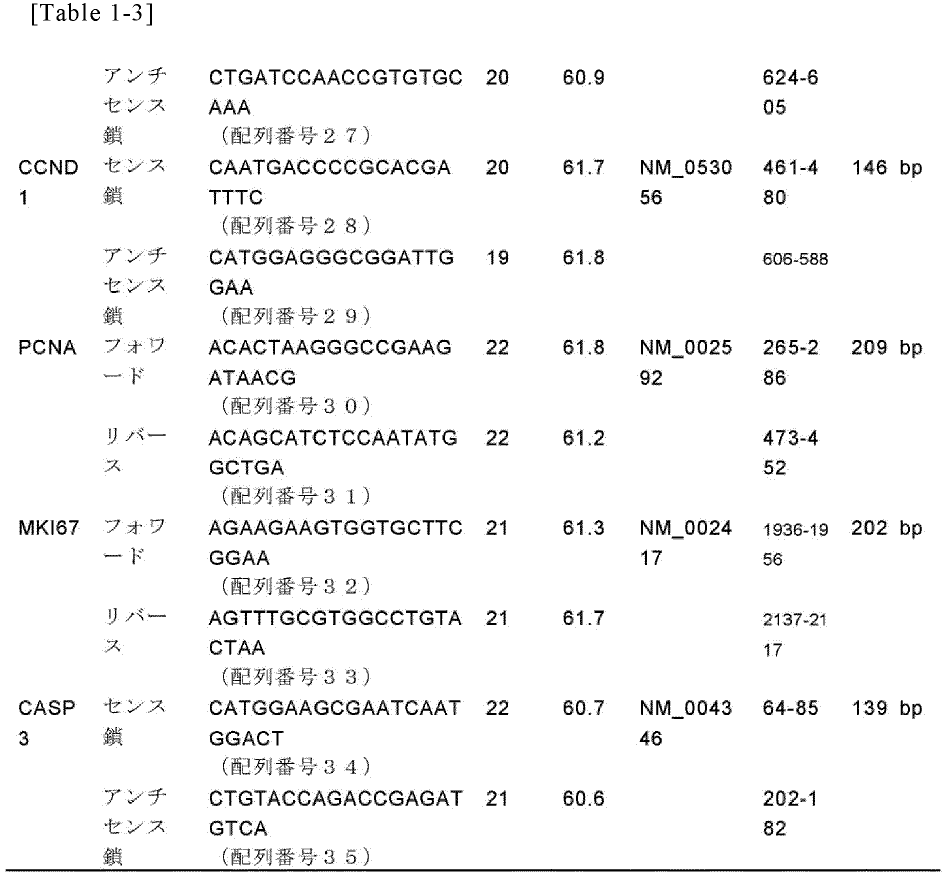

[In the table,
BIRC, baculoviral inhibitor of apoptosis repeat-containing (BIRC) protein;
GAPDH, glyceraldehyde 3-phosphate dehydrogenase;
Bcl-2, B-cell lymphoma 2;
Bax, Bcl-2-associated X protein;
BclXL: B-cell lymphoma-extra large;
APAF-1: apoptotic protease-activating factor 1;
CCND1, cyclin D1;
PCNA, proliferating cell nuclear antigen;
MPKi-67, proliferation marker Ki-67]

### Reference Result 3

### Ability to Degrade Corisin-Containing Transglycosylase in a Bacterial Secretion Factor and Ability to Induce Apoptosis of Lung Epithelial Cells

The inventors of the present invention have previously reported that corisin, which is an apoptotic peptide, may be cleaved from an S. nepalensis transglycosylase to induce AE of pulmonary fibrosis (Non-Patent Literature 19). The factor involved in this cleavage is unknown. Herein, it was predicted that corisin-shedding bacteria secrete a protease that cleaves apoptotic peptides from their respective transglycosylase (FIG. 7a). In order to verify this prediction, firstly, a peptide fraction was partially purified from the S. nepalensis CNDG strain culture supernatant. The S. nepalensis culture supernatant was successively concentrated using a >50 kDa filter, and then a flow-through (<50 kDa) fraction was concentrated using a >10 kDa filter (FIG. 7b). The concentrated fraction was loaded on a Sephacryl S-300 column, the product was separated by gel electrophoresis, and then the digestive activity of the collected fractions on a corisin-containing recombinant transglycosylase was measured (FIG. 7c). Fractions having high digestive activity were pooled and concentrated. Loading was performed a second time on a Sephacryl S-300 column, and fractions having high digestion activity were separated (FIG. 7d).

Next, a reaction mixture containing a digestion buffer, recombinant transglycosylase, and various amounts of a peptidase fraction (>10kDa) prepared from the Staphylococcus nepalensis culture supernatant was incubated for two hours at 37°C. Sodium dodecyl sulfate-polyacrylamide gel electrophoresis was then performed, followed by staining with silver stain. Furthermore, a reaction mixture containing a digestion buffer, recombinant transglycosylase, and a dose of the peptidase fraction (>10kDa) prepared from the Staphylococcus nepalensis culture supernatant was incubated for various time intervals at 37°C. Sodium dodecyl sulfate-polyacrylamide gel was performed, followed by staining with silver stain. As a result, the silver staining of the gel showed a dose- and time-dependent degradation of the recombinant transglycosylase (FIG. 8a, b). As another experiment, the degradation action of the Staphylococcus haemolyticus culture supernatant on transglycosylase was investigated. A reaction mixture containing a digestion buffer, recombinant transglycosylase, and the Staphylococcus haemolyticus strain 12 culture supernatant was incubated for 14 hours at 37°C. Sodium dodecyl sulfate-polyacrylamide gel electrophoresis was then performed, followed by staining with silver stain. As a result, the Staphylococcus haemolyticus culture supernatant was also shown to cleave the recombinant transglycosylase (FIG. 9). Next, A549 alveolar epithelial cells were cultured to a subconfluent. The cultured cells were treated with a degradation product prepared by incubating recombinant transglycosylase in Staphylococcus nepalensis culture supernatant for two hours. As a result, flow cytometry analysis performed 48 hours after cell culture revealed that apoptosis of the epithelial cells cultured in the presence of the degradation product increased significantly compared to the control (FIG. 8c, d). These results indicate that a bacterial secretion factor such as protease cleaves the transglycosylase, thereby providing the corisin peptide that induces apoptosis in lung epithelial cells.

### Reference Result 4

### Bacterial Secretion Factor that Cleaves Transglycosylase: Putative Serine Protease

In order to determine whether the transglycosylase cleavage factor is a protease or not, Pefabloc SC or diisopropyl fluorophosphate (DFP) as a serine protease inhibitor; E-64 as a cysteine protease inhibitor; or ethylenediaminetetraacetic acid (EDTA) as a chelating agent were added at various concentrations (0.1, 0.3, 1 mM) to a reaction mixture containing recombinant transglycosylase (2 mg/mL) and a peptidase fraction (>10kDa) prepared from Staphylococcus nepalensis culture supernatant, and this was incubated at 37°C for 0.5, 1, and 2 hours. As a result, after separation of the product on a protein gel by silver staining, it was found that the serine protease inhibitors Pefablock SC and DFP suppress transglycosylase degradation (FIG. 10a, b, c, and d). However, neither E-64 nor EDTA suppressed transglycosylase degradation (FIG. 10 e, f, g, and h). These results indicate that the putative serine protease secreted by Staphylococcus nepalensis degrades the corisin-containing transglycosylase thereof to form smaller peptides containing corisin.

### Materials and Method

Reference results, reagents used in the examples of the present Specification, and the like were obtained or prepared as follows.

### Reagent:

Human lung epithelial cell line A549 and hypersaline media (ATCC media 1097, 2168) were purchased from the American Type Culture Collection (Manassas, Virginia, (USA)), Dulbecco's Modified Eagle Medium (DMEM) was purchased from Sigma-Aldrich (St. Louis, Missouri (USA)), and fetal bovine serum (FBS) was purchased from Bio Whittaker (Walkersville, Maryland (USA)).

L-glutamine, penicillin, and streptomycin were purchased from Invitrogen (Carlsbad, CA).

A synthetic corisin and a corresponding synthetic scrambled peptide: NRVYNGPAASPVSEGMPIN (SEQ ID NO: 1) were prepared and provided by Peptide Institute Incorporation (Osaka City) and ThermoFisher Scientific (Waltham, MA).

Bleomycin (BLM) was purchased from Nippon Kayaku (Tokyo, Japan) and ALZET osmotic minipumps (model 2001) were purchased from Alza Corporation (Palo Alto, CA). Herein, a scrambled peptide is a peptide in which amino acid sequences have been disassembled, resulting in loss of function. Specifically, it is a peptide in which function has been lost due to disassembly of corisin amino acid sequences.

### Animal:

Female WT C57BL/6J mice were purchased from Japan SLC (Hamamatsu City, Japan) as laboratory animals, in which lung injury, pulmonary fibrosis, and acute exacerbation were induced (Reference Literature 29). Eight-to-nine week-old WT mice weighing 20 to 22 g were used for BLM-induced pulmonary fibrosis experiments. Furthermore, female and male transforming β1 (TGFβ1) TG mice having a C57BL/6J background in which naturally-progressing and lethal pulmonary fibrosis develops and WT littermates thereof were used (Non-Patent Literature 19, Reference Literature 31 and 61). The TG mice and WT littermates weighed 20 to 24 g and were 8 to 10 weeks old. The mice were bred in a specific pathogen-free environment at 21°C with a 12-hour light-dark cycle at the Mie University Experimental Animal Facility. Mouse cages were supplied with wood-wool nesting material and the mice were able to freely access water and food. Genotyping of the TG mice was performed by standard PCR analysis using DNA isolated from mouse tails and specific primer pairs (Non-Patent Literature 19).

### Ethical Statement:

The Recombinant DNA Experimental Safety Committee (approval no.: I-614 (henko 1); date: 12/15/2013; approval no.: I-708, date: 02/13/2019) and the Mie University Animal Research Committee approved the experimental protocols (approval no.: 25-20-hen 1-sai 1, date: 07/23/2015; approval number 29-23, date: 01/15/2019). All experimental procedures were performed in accordance with the internationally accepted laboratory animal care principles published by the National Institutes of Health (https://olaw.nih.gov/). The study followed ARRIVE guidelines for animal research and variables were measured blinded to treatment groups.

### Preparation of Peptidase Fraction:

A successively concentrated, 100 mL Staphylococcus nepalensis culture supernatant was prepared using a >50 kDa filter, and the obtained passage fraction was concentrated using a >10 kDa filter as illustrated in FIG. 7b. Then, a sample concentrated to >10 kDa was loaded onto a Sephacryl S-300 column, and several fractions (1 mL each) were separated. Products of the proteolytic activity of each fraction on the corisin-containing recombinant transglycosylase were separated by sodium dodecyl sulfate polyacrylamide gel electrophoresis and silver staining (FIG. 7c). Silver staining was performed using a Daiichi 2-D silver staining kit (Daiichi (Tokyo)). The reaction mixture contained 0.5 µL of 1× digestion buffer (20 mM TrisCl, 140 mM NaCl, pH 7.5), 2 mg/mL of recombinant transglycosylase, and 6 µL of each fraction. Next, a pool of fractions having high digestive activity (fractions 10 to 13 in FIG. 7c) was prepared and concentrated to 0.5 mL. Next, the concentrated sample was loaded onto a Sephacryl S-300 column and separated into several fractions, and absorbance was measured at 280 nm (FIG. 7d). The digestive activity of each fraction on recombinant transglycosylase 351 was then assessed as described above (FIG. 7d). Fractions having high proteolytic activity were used in the experiments.

### Recombinant Transglycosylase Degradation Assay:

Recombinant transglycosylase (12.5 µL; 2 µg/mL), 5 µL of 10× digestion buffer (TrisCl, 1.4 NaCl, pH 7.5), 25 µl of Staphylococcus nepalensis culture supernatant, and 7.5 µL of distilled water were added to 50 µL of the reaction mixture. The reaction mixture was incubated overnight at 37°C, and then 10 µL of the reaction mixture was subjected to sodium dodecyl sulfate-polyacrylamide gel electrophoresis (SDS-PAGE) to visualize the product using silver staining. In a separate experiment, several amounts of Staphylococcus nepalensis culture supernatant, an anticorisin monoclonal antibody, or an isotype antibody were added to the reaction mixture and run on SDS-PAGE for subsequent silver staining.

### Transglycosylase Degradation Inhibition Assay:

A reaction mixture of a digestive buffer containing recombinant transglycosylase (2 µg/mL); Staphylococcus nepalensis culture supernatant; and a serine protease inhibitor (Pefablock SC or diisopropyl fluorophosphate [DFP]), a cysteine protease inhibitor (E-64), or a chelating agent (ethylenediaminetetraacetic acid [EDTA]) at various concentrations (0.1, 0.3, 1 mM) was prepared. Next, the mixture was incubated for 0.5, 1, and 2 hours before performing SDS-PAGE and silver staining.

### Cell Culture:

Human A549 alveolar epithelial cells were cultured at 37°C in DMEM supplemented with 10% fetal bovine serum, 0.03% (w/v) L-glutamine, 100 IU/mL penicillin, and 100 µg/mL streptomycin in a 5% CO2 humidified atmosphere.

### Apoptosis Assay:

A549 cells (4×105 cells/well) were cultured to subconfluency in 12-well plates and serum-starved for 24 hours. Next, the cells were treated with corisin or a corisin-like peptide or a scrambled peptide based on the corisin sequences to assess apoptosis after 48 hours. Apoptosis was assessed using flow cytometry (Oxford, UK, BD Biosciences, FACScan) after fluorescein-labeled annexin V and propidium iodide (FITC Annexin V Apoptosis Detection Kit with PI, Bioelgend, San Diego, CA) staining. The percentage of cells positive for cleaved caspase-8, cleaved caspase-9, and cleaved caspase-3 was evaluated by flow cytometry using antibodies derived from Cell Signaling Technology (Beverly, Massachusetts (USA)).

### Transmission Electron Microscopy Observation of Apoptotic Cells

A549 cells (10×104 cells/mL) were plated on a collagen-coated 8-well chamber slide (BD Bioscience, San Jose, California) and cultured until semi-confluent. The cells were serum-starved for 6 hours and stimulated with a pro-apoptotic peptide (5 µM) for 16 hours. The cells were fixed for 2 hours at room temperature in a 0.1 M sodium cacodylate buffer (pH 7.4) using 2% fresh formaldehyde and 2.5% glutaraldehyde. After washing with a 0.1 M cacodylic acid buffer (pH 7.4), 1% OsO4 was fixed for 2 hours at 4°C in the same buffer. Samples were rinsed with distilled water, stained for 2 hours or overnight at room temperature with a 1% uranyl acetate aqueous solution, dehydrated with ethanol and propylene oxide, and embedded in Epon (Epon 812 resin, Nacalai). After removing the cells from the glass, ultrathin sections (94 nm) were cut, stained with uranyl acetate and Reynolds lead citrate, and observed under a transmission electron microscope (Tokyo, Japan, JEOL, JEM-1010).

### Assessment of Activity of Transglycosylase Degradation Product

A549 alveolar epithelial cells (2×105 cells/well) were cultured in 12-well plates until confluent. After overnight serum starvation, a reaction mixture containing 5 µM recombinant transglycosylase 351, 1/10 volume Staphylococcus nepalensis supernatant, and 5 µM anticorisin monoclonal antibody or isotype antibody was added to the cell cultures and incubated for 48 hours. Apoptosis was assessed as described above.

### Genomic DNA Sequencing and Genome Annotation

Genome sequencing was performed as in a previous report (Non-Patent Literature 19) using a combination of Oxford Nanopore sequencing and Illumina Miseq Nanopore sequencing. Simply stated, genomic DNA derived from each bacterial strain (400 ng) was converted into a nanopore library using Rapid Barcoding library kit SQK-RAD 004, and the resulting library was sequenced for 48 hours on a SpotON R 9.4.1 FLOMIN-MIN 106 flow cell using a GridION sequencer. Most of the readings were between 6 and 30 kb in length; however, a reading length of 94 kb was also obtained. Illumina Miseq sequencing was performed by preparing a shotgun genomic library using the Hyper Library construction kit from Kapa Biosystems (Roche). The library was quantified by qPCR and sequenced over 251 cycles on one MiSeq Nano flow cell. Fastq files were generated and separated with bcl2 fastq v 2.20 conversion software (Illumina).

Initial assembly of Oxford Nanopore data was performed using Canu (Reference Literature 62). Next, refining was performed using Nanopolish (Reference Literature 63) and Pilon (use of Illumina MiSeq reads) (Reference Literature 64). Finally, the genome was reoriented using Circlator (Reference Literature 65). Another hybrid genome assembly was performed using SPAdes (Reference Literature 66), and then the genome was reoriented using Circlator. Furthermore, hybrid genome assembly was performed using Unicycler (Reference Literature 67). The final hybrid genome assembly was prepared using Unicycler with the Canu assembly as the assembly backbone.

All assemblies were quality assessed using BUSCO (Reference Literature 68) and QUAST (Reference Literature 69) and compared to relevant reference genomes using MUMmer (Reference Literature 70). The genome assemblies were annotated (annotated) using the tool Prokka (Reference Literature 71), and after evaluation, the best overall BUSCO score was combined with the overall assembly metrics to determine the best overall assembly.

### Example 1

### Anticorisin Neutralizing Monoclonal Antibody Inhibition of Lung Cell Apoptosis Induced by Transglycosylase Degradation Products

### 1-1: Preparation of Monoclonal Antibody Against Pro-Apoptotic Peptide IVMPESSGNPNAVNPAGYR (SEQ ID NO: 1)

Based on the findings of the above reference results, it was expected that an anticorisin neutralizing antibody would inhibit the pro-apoptotic activity of transglycosylase degradation products derived from the proteolytic activity of the putative bacterial serine protease. In order to verify this prediction, first, a monoclonal antibody against pro-apoptotic peptide IVMPESSGNPNAVNPAGYR was prepared and characterized as an anticorisin neutralizing monoclonal antibody. Note that the pro-apopototic peptide IVMPESSGNPNAVNPAGYR is the corisin-like sequence of Staphylococcus nepalensis shown in FIG. 3 and FIG. 4, and this was prepared by the manufacturer (ThermoFisher Scientific).

The monoclonal antibody was prepared by Eurofins Genomics Inc. (Tokyo, Japan). Simply stated, a peptide used in immunization (NH2-C+IVMPESSGNPNAVNPAGYR-COOH) was synthesized and purified by high-performance liquid chromatography, and then bound by the maleimide method to keyhole limpet hemocyanin, a commonly used carrier protein (Reference Literature 72 and 73). In order to immunize the laboratory animals, an immunogen was subcutaneously injected once at weeks 0, 2, 4, and 6 into two Wistar rats, and then the same peptide was intravenously injected once at week 8 after the initial immunization. Blood was collected 5 weeks after the first immunization, and the antibody titer was measured by immunoassay. Splenocytes from one rat were used for cell fusion with myeloma cells (P3U1 and Sp2). Cell fusion was performed using a hypoxanthine-aminopterin-thymidine culture medium using a polyethylene glycol method (Reference Literature 74). A monoclonal antibody-producing hybridoma was cloned using a limiting dilution method (Reference Literature 74). The obtained hybridomas were stored in liquid nitrogen for subsequent use. Isotyping was performed using Rapid RAT Monoclonal Antibody Isotyping XpressCard purchased from Antagen Pharmaceuticals, Inc. (Boston, Massachusetts (USA)).

Various amounts of recombinant transglycosylase 351 were electrophoresed using polyacrylamide gel, and Western blotting was performed using a 1:1000 dilution of a supernatant of a hybridoma for producing each clone of the anticorisin monoclonal antibody. In particular, tissues or samples were washed twice with ice-cooled phosphate-buffered saline. Next, cell lysis was performed in a radioimmunoprecipitation assay (RIPA) buffer solution (10 mM Tris-Cl (pH 8.0), 1 mM EDTA, 1% Triton X-100, 0.1% sodium deoxycholate, 0.1% SDS, 140 mM NaCl, and 1 mM phenlymethylsulfonyl fluoride) supplemented with protease/phosphatase inhibitors (1 mM orthovanadate, 50 mM β-glycerophosphate, 10 mM sodium pyrophosphate, 5 µg/mL leupeptin, 2 µg/mL aprotinin, and 5 mM sodium fluoride). After centrifuging at 4°C for 10 minutes at 17,000×g, the protein concentration was measured using a Pierce BCA protein assay kit (Thermo Fisher Scientific, Inc., Waltham, Massachusetts). Samples containing equivalent amounts of protein were mixed with a Laemmli sample buffer solution and separated by sodium dodecyl sulfate-polyacrylamide gel electrophoresis (SDS-PAGE). Next, Western blotting was implemented using a nitrocellulose membrane and anti-cleaved caspase-3 or anti-β-actin antibody (Cell Signaling, Danvers, Massachusetts (USA)) (Reference Literature 61). The band intensity on the blots was quantitated using a public domain NIH Imaged program (wayne@codon.nih.gov; Wayne Rasband, NIH, Office of Research Services).

The recombinant transglycosylase 351 used herein was expressed as follows. In order to prepare the recombinant transglycosylase 351, a gene encoding the protein was synthesized using Escherichia coli (Escherichia coli)-optimized codons, terminal A was added and amplification was performed, and cloning was performed onto TA cloning vector-pGEM-T Easy (Promega, Madison, Wisconsin, (USA)). Next, the gene was removed, cloned into a modified pET28a vector, transformed into Escherichia coli BL21 DE3 cells, expressed as a 6-histidine tagged (His-tag) protein, and purified (Non-Patent Literature 19).

As described above, hybridoma clones 2A, 3Aa, 4A, 9A and 21A, which respectively produce immunoglobulins (Ig)IgG2bx, IgMκ, IgG2aκ, IgG2aκ, IgG2aκ, and IgG2bκ, were established (FIG. 11a). Isotyping of each monoclonal antibody was performed using the Rapid Monoclonal Antibody Isotyping Kit.

Western blotting implemented using an increased amount of recombinant transglycosylase revealed that all monoclonal antibody clones recognize and bind to a corisin-containing transglycosylase derived from S. nepalensis (FIG. 11b). Next, a corisin peptide was coated on a microplate and the binding of each clone of the anticorisin monoclonal antibody was evaluated using various diluents of a hybridoma supernatant. The monoclonal antibodies produced by each hybridoma clone differentially bound to the surface-coated corisin at various levels of dilution. In particular, clones 9A and 21A exhibited strong binding activity (FIG. 11c).

### 1-2: Epitope Mapping of Monoclonal Antibody Against Pro-Apoptotic Peptide IVMPESSGNPNAVNPAGYR (SEQ ID NO: 1)

Epitope mapping was performed to identify the binding site of the anticorisin monoclonal antibody in the corisin molecule. The binding of each monoclonal antibody to a microarray of small peptides having different lengths of corisin sequences was evaluated for this purpose. Using a hemagglutinin peptide and an anti-hemagglutinin antibody as controls, the integrity and assay quality in the peptide microarray were verified. Mapping was performed by PEPPerPRINT Incorporation (Heidelberg).

Specifically, the peptide IVMPESSGNPNAVNPAGYR was first extended at the C-terminus and the N-terminus using a neutral GSGSG linker to avoid forming a truncated peptide. The extended peptide sequence was translated into 5, 10, and 15 amino acid peptides with peptide-peptide overlaps of 4, 9, and 14 amino acids. The resulting peptide microarray contained 72 different linear peptides in duplicate (144 spots) and an additional hemagglutinin (YPYDVPDYAG, 17 spots) control peptide. Prestaining of a peptide microarray copy was performed in an incubation buffer (wash buffer, [phosphate-buffered saline, pH 7.4, 0.05% Tween 20, 10% Rockland blocking buffer, MB-070) using a secondary (goat anti-rat IgG [H+L] DyLight 680) antibody and a control (mouse monoclonal anti-HA [12CA5] DyLight 800) antibody, and background interactions with the 72 different peptides of the microarray were determined. Next, other peptide microarray copies were incubated with rat monoclonal antibodies 9A, 21A, 2A, and 4A at a concentration of 1 µg/mL in the incubation buffer solution, stained with secondary and reference antibodies, and then read at scanning intensities of 7/7 (middle spot/peripheral spot (upper and left edge spot)) using an LI-COR Odyssey imaging system. The additional hemagglutinin peptide constituting the peptide microarray was simultaneously stained as an internal quality control, and the quality of the assay and the integrity of the peptide microarray were confirmed (FIG. 12a, b).

Quantification of spot intensities and peptide annotation were based on a 24-bit grayscale tiff file at scanning intensities of 7/7, exhibiting a higher dynamic range than a 16-bit colorized tiff file. Microarray image analysis was performed using a PepSlide (registered trademark) analyzer. The results are described in supplemental microarray data. A software algorithm breaks down the fluorescence intensities of each spot into raw, foreground, and background signals to calculate the median of the average foreground intensities and inter-spot deviations of spot duplicates. Intensity maps were generated on the basis of the median value of the average foreground intensity, and an intensity color code highlighted the interactions in the peptide maps of the middle spots in relation to the high spot intensities and white in relation to the low spot intensities. The maximum deviation from spot to spot was 40%. Otherwise, the corresponding intensity value was set to zero. Furthermore, the average spot intensities of assays using rat antibodies against antigen sequences from the N-terminus to the C-terminus of peptide IVMPESSGNPNAVNPAGYR were plotted to visualize the total spot intensities and signal-to-noise ratios. In order to identify the epitopes of rat antibodies 9A, 21A, 2A, and 4A, intensity plots were correlated with the peptide and intensity maps, as well as visual inspection of microarrays. In this case, while it is unclear whether or not a defined amino acid contributes to antibody binding properties, the corresponding amino acid characters have been drawn in gray.

As a result of the above epitope mapping, only staining of the hemagglutinin peptide with the control antibody showed an established spot pattern having no background interaction at any scanning intensity level (FIG. 12a, b). Following the subsequent incubation buffer of rat monoclonal antibodies 9A (c, d), 21A (e, f), 2A (g, h), and 4A (i, j) at a concentration of 1 µg/ml during incubation and another peptide microarray copy, staining was performed using a secondary antibody and a control antibody, followed by reading at scanning intensities of 7/7 (middle spot/peripheral spot) using the LI-COR Odyssey Imaging System. Then, a moderate and overt antibody response to a medium-spot epitope-like spot pattern containing a consensus motif PESSGNP (SEQ ID NO: 68) for the 9A, 21A, and 2A monoclonal antibody clones was indicated (FIG. 12c, d, e, f, g, h), and containing a consensus motif NPAGY (SEQ ID NO: 69) for the 4A monoclonal antibody clone (FIG. 12i, j), and included a high signal-to-noise ratio (supplementary microarray data).

### 1-3: Sequencing of Variable Framework and Complementarity Determining Region of Anticorisin Monoclonal Antibody Clones

Variable regions of anticorisin monoclonal antibody clones 4A, 9A, and 2A were sequenced at Syd Labs, Incorporated (Hopkinton, Massachusetts (USA)). Using cDNA synthesized using total RNA derived from each hybridoma, 5' rapid amplification reaction of cDNA termini was performed. Positive bands obtained by PCR analysis were cloned and sequenced. The complementarity determining regions (CDRs) of each clone were sequenced. CDR regions were defined using IMGT/V-QUEST (http://www.imgt.org). Furthermore, sequences of the variable regions of the anticorisin monoclonal antibody clone 21A were determined in the same manner. CDR identification was performed on the basis of the Kabat system.

FIG. 13 shows the variable frameworks and complementarity determining regions of the monoclonal antibodies.

Details are as follows:
Anticorisin monoclonal antibody clone 4A
H chain (4A-HC.7331): variable region
evqvlesggglvqpgnslklscatsGFTFSTAWmywyrqfpekrlewvarIKAKSNSYATdytesvkgr ftisrddskgsiylrmnnlkeedtaiyycASTDAFYFSHSYwgqgvlvtvss (SEQ ID NO: 36)
H chain variable region CDR1 amino acid sequence: GFTFSTAW (SEQ ID NO: 37)
H chain variable region CDR2 amino acid sequence: IKAKSNSYAT (SEQ ID NO: 38)
H chain variable region CDR3 amino acid sequence: ASTDAFYFSHSY (SEQ ID NO: 39)
H chain (4A-HC.7331): full length 
MELCMMWIFLVAFLKGVQCevqvlesggglvqpgnslklscatsGFTFSTAWmywyrqfpekrl
ewvarIKAKSNSYATdytesvkgrftisrddskgsiylrmnnlkeedtaiyycASTDAFYFSHSYwgqgvl
vtvssAETTAPSVYPLAPGTALKSNSMVTLGCLVKGYFPEPVTVTWNSGALSSGVHT
FPAVLQSGLYTLTSSVTVPSST WSSQAVTCNVAH (SEQ ID NO: 79)
(However, sequence: MELCMMWIFLVAFLKGVQC (SEQ ID NO: 80) is a signal peptide, and
sequence:
   AETTAPSVYPLAPGTALKSNSMVTLGCLVKGYFPEPVTVTWNSGALSSGVHTFPA VLQSGLYTLTSSVTVPSSTWSSQAVTCNVAH (SEQ ID NO: 81) is a constant region)
L chain (4A-LC.7395): variable region 
divmtqtpssqavsagekvtmrcrssQSLLYSENKKNYlawyqqkpgrspklliyWASTgesgvpdrfig
sgsgtdftltissvqaedlavyycQQYYNFPLTfgsgtkleik (SEQ ID NO: 40)
L chain variable region CDR1 amino acid sequence: QSLLYSENKKNY (SEQ ID NO: 41)
L chain variable region CDR2 amino acid sequence: WAST (SEQ ID NO: 42)
L chain variable region CDR3 amino acid sequence: QQYYNFPLT (SEQ ID NO: 43)
L chain (4A-LC.7395): full length 
MESQTQVLMSLLLWVSGTCGdivmtqtpssqavsagekvtmrcrssQSLLYSENKKNYlaw
yqqkpgrspklliyWASTgesgvpdrfigsgsgtdftltissvqaedlavyycQQYYNFPLTfgsgtkleikRAD
AAPTVSIFPPSMEQLTSGGATVVCFVNNFYPRDISVKWKIDGSEQRDGVLDSVTDQ
DSK DSTYSMSSTLSLTKVEYERHNLYTCEVVHKTS (SEQ ID NO: 82)
(However, sequence: MESQTQVLMSLLLWVSGTCG (SEQ ID NO: 83) is a signal peptide, and
sequence: 
RADAAPTVSIFPPSMEQLTSGGATVVCFVNNFYPRDISVKWKIDGSEQRDGVLDSV
TDQDSKDSTYSMSSTLSLTKVEYERHNLYTCEVVHKTS (SEQ ID NO: 84)
 is a constant region)

### Anticorisin monoclonal antibody clone 9A

L chain (9A-LC.7402): variable region 
divmtqspssqavsagekvtmsckssQNLLYSEDKKNYlawyqqkpgqspklliyWAStresgvpdrfi

gsgsgtdftltvtsvqaedlavyycQQYYNFPRTfgggtklelk (SEQ ID NO: 44)
L chain variable region CDR1 amino acid sequence: QNLLYSEDKKNY (SEQ ID NO: 45)
L chain variable region CDR2 amino acid sequence: WAS (SEQ ID NO: 46)
L chain variable region CDR3 amino acid sequence: QQYYNFPRT (SEQ ID NO: 47)
L chain (9A-LC.7402): full length 
MESQTQVLMSLLLWVSGTCGdivmtqspssqavsagekvtmsckssQNLLYSEDKKNYla
wyqqkpgqspklliyWAStresgvpdrfigsgsgtdftltvtsvqaedlavyycQQYYNFPRTfgggtklelkRA
DAAPTVSIFPPSMEQLTSGGATVVCFVNNFYPRDISVKWKIDGSEQRDGVLDSVTD
QDSKDSTYSMSSTLSLTKVEYERHNLYTCEVVHKTS (SEQ ID NO: 85)
(However, sequence: MESQTQVLMSLLLWVSGTCG (SEQ ID NO: 86) is a signal peptide, and
sequence 
RADAAPTVSIFPPSMEQLTSGGATVVCFVNNFYPRDISVKWKIDGSEQRDGVLDSV
TDQDSKDSTYSMSSTLSLTKVEYERHNLYTCEVVHKTS (SEQ ID NO: 87)
 is a constant region)

### Anticorisin monoclonal antibody clone 2A

H chain (2A-HC.7352): variable region 
evklvesggglvqpgnsltlscvasGFTFTNYGmhwirqapkkglewiamIYYDSSKMsyadtvkgrfti

srdnskntlylemnslrsedtamyycAAEGFGTPFPYwgqgtlvtvss (SEQ ID NO: 48)
H chain variable region CDR1 amino acid sequence: GFTFTNYG (SEQ ID NO: 70)
H chain variable region CDR2 amino acid sequence: IYYDSSKM (SEQ ID NO: 49)
H chain variable region CDR3 amino acid sequence: AAEGFGTPFPY (SEQ ID NO: 50)
H chain (2A-HC.7352): full length 
MDFRLNLVFLVFILKGVWCevklvesggglvqpgnsltlscvasGFTFTNYGmhwirqapkkgle
wiamIYYDSSKMsyadtvkgrftisrdnskntlylemnslrsedtamyycAAEGFGTPFPYwgqgtlvtvss
AQTTAPSVYPLAPGCGDTTSSTVTLGCLVKGYFPEPVTVTWNSGALSSDVHTFPAV
LQSGLYTLTSSVTSSTWPSQTVTCNVAH (SEQ ID NO: 88)
(However, sequence: MDFRLNLVFLVFILKGVWC (SEQ ID NO: 89) is a signal peptide, and
sequence: 
AQTTAPSVYPLAPGCGDTTSSTVTLGCLVKGYFPEPVTVTWNSGALSSDVHTFPAV

LQSGLYTLTSSVTSSTWPSQTVTCNVAH (SEQ ID NO: 90)
 is a constant region)
L chain (2A-LC.7413): variable region 
divmtqtpssravsagekvtmsckssQSLLYSENEKNYlawyqqrpgqspklliyWAStresgvpdrfigt
gsgtdftltissvqaedlavyycQQYYHFPRTfgggtrlelk (SEQ ID NO: 51)
L chain variable region CDR1 amino acid sequence: QSLLYSENEKNY (SEQ ID NO: 52)
L chain variable region CDR2 amino acid sequence: WAS (SEQ ID NO: 53)
L chain variable region CDR3 amino acid sequence: QQYYHFPRT (SEQ ID NO: 54)
L chain (2A-LC.7413): full length 
MESQTQVLMSLLLWVSGSCGdivmtqtpssravsagekvtmscksssQSLLYSENEKNYlaw
yqqrpgqspklliyWAStresgvpdrfigtgsgtdftltissvqaedlavyycQQYYHFPRTfgggtrlelkRADA
APTVSIFPPSMEQLTSGGATVVCFVNNFYPRDISVKWKIDGSEQRDGVLDSVTDQD
SKDSTYSMSSTLSLTKVEYERHNLYTCEVVHKTS (SEQ ID NO: 91)
(However, sequence: MESQTQVLMSLLLWVSGSCG (SEQ ID NO: 92) is a signal peptide, and
sequence: 
RADAAPTVSIFPPSMEQLTSGGATVVCFVNNFYPRDISVKWKIDGSEQRDGVLDSV
TDQDSKDSTYSMSSTLSLTKVEYERHNLYTCEVVHKTS (SEQ ID NO: 93)
 is a constant region)

### Anticorisin monoclonal antibody clone 21A:

H chain (KK1410-2_VH_pep): variable region 
evklvesggglvqpgnsltlscgasgftftNYGMHwirqapkkglewigMIYYDSSKMSYADTVKGr

ftisrdnsknilylemnslrsedtamyycaaEGFGTPFPYwgqgtlvtvss (SEQ ID NO: 71)
H chain variable region CDR1 amino acid sequence: NYGMH (SEQ ID NO: 72)
H chain variable region CDR2 amino acid sequence: MIYYDSSKMSYADTVKG (SEQ ID NO: 73)
H chain variable region CDR3 amino acid sequence: EGFGTPFPY (SEQ ID NO: 74)
H chain (KK1410-2_VH_pep): full length 
MDFRLNLVFLVFILKGVWCevklvesggglvqpgnsltlscgasgftftNYGMHwirqapkkglew
igMIYYDSSKMSYADTVKGrftisrdnsknilylemnslrsedtamyycaaEGFGTPFPYwgqgtlvtvs
s (SEQ ID NO: 94)
(However, sequence: MDFRLNLVFLVFILKGVW (SEQ ID NO: 95) is a signal peptide)
L chain (KK1410-2_VL_pep): variable region 
divmtqtpssqavsagekvtmscKSSQSLLYREDTKNYLAwyqqrpgqspklliyWASTRESgvp
drfigsgsgtdftltissvqaedlavyycQQYYHFPRTfgggtrlelk (SEQ ID NO: 75)
L chain variable region CDR1 amino acid sequence: KSSQSLLYREDTKNYLA (SEQ ID NO: 76)
L chain variable region CDR2 amino acid sequence: WASTRES (SEQ ID NO: 77)
L chain variable region CDR3 amino acid sequence: QQYYHFPRT (SEQ ID NO: 78)
L chain (KK1410-2_VL_pep): full length 
MESQTQVLMSLLLWVSGTYGdivmtqtpssqavsagekvtmscKSSQSLLYREDTKNYL
AwyqqrpgqspklliyWASTRESgvpdrfigsgsgtdftltissvqaedlavyycQQYYHFPRTfgggtrlelk
(SEQ ID NO: 96)
(However, sequence: MESQTQVLMSLLLWVSGTYG (SEQ ID NO: 97) is a signal peptide)

### 1-4: Inhibition of Corisin Pro-Apoptotic Activity by Anticorisin Monoclonal Antibody Clones

Whether monoclonal antibodies (2A, 9A, and 21A) having high binding ability to corisin can inhibit the pro-apoptotic activity of corisin on cultured alveolar epithelial cells was evaluated.

Apoptosis inhibition assay was performed by culturing A549 alveolar epithelial cells to a subconfluent, serum starving overnight, and then pretreating anticorisin monoclonal antibody clones 2A, 9A, or 21A and continuing to culture for an additional 48 hours. The number of apoptotic cells was evaluated using flow cytometry as described above.

A549 alveolar epithelial cells were pretreated with each clone of the anticorisin monoclonal antibody (1:100 dilution of hybridoma supernatant) and then cultured in the presence of corisin or a scrambled peptide. A549 cells pretreated with rat anticorisin serum were used as a positive control and cells which had not undergone pretreatment were used as a negative control. Flow cytometry analysis revealed that only the clone 21A monoclonal antibody significantly neutralized corisin-induced apoptosis of alveolar epithelial cells in vitro (FIG. 14a, b).

Furthermore, an investigation was carried out as to whether the clone 21A neutralizing monoclonal antibody inhibits apoptotic activity of both Staphylococcus nepalensis and Staphylococcus haemolyticus. A549 alveolar epithelial cells were pretreated with anticorisin monoclonal antibody clone A21 hybridoma supernatant (1:100 dilution) and then cultured in the presence of Staphylococcus haemolyticus strain 12 culture supernatant. A549 cells pretreated with only monoclonal antibody A21 or isotype IgG were used as a negative control, and cells pretreated with Staphylococcus nepalensis supernatant were used as a positive control. As a result, the clone 21A neutralizing monoclonal antibody also significantly inhibited the apoptotic activity of both Staphylococcus nepalensis and Staphylococcus haemolyticus bacterial supernatant on A549 alveolar epithelial cells (FIG. 15a, b).

Therefore, in subsequent experiments, the clone 21A monoclonal antibody was used to evaluate whether anticorisin monoclonal antibodies can inhibit the pro-apoptotic activity of Staphylococcus nepalensis corisin-containing transglycosylase degradation products. A reaction mixture containing recombinant transglycosylase (2 mg/mL), a peptidase fraction (>10 kDa) derived from Staphylococcus nepalensis culture supernatant, and 20 µg/mL of anticorisin monoclonal antibody clone A21 or an isotype antibody was incubated for 2 hours, and then added to an A549 cell culture. After 48 hours of culture, apoptosis was evaluated by flow cytometry analysis. Cells treated with corisin and an anticorisin antibody or an isotype antibody were used as a positive control, and cells treated with an anticorisin antibody or an isotype antibody alone were used as a negative control. As a result, flow cytometry analysis showed that anticorisin monoclonal antibody clone A21 significantly inhibited the apoptotic activity of transglycosylase degradation products on alveolar epithelial cells compared to the isotype antibody control (FIG. 16a, b). Of note, however, gel electrophoresis and silver staining revealed that anticorisin neutralizing monoclonal antibody clone 21A was unable to inhibit proteolysis of the recombinant transglycosylase (FIG. 16c). Overall, these observations show that bacterial secretion of the putative serine protease causes transglycosylase proteolysis, transglycosylase proteolysis releases corisin to induce apoptosis in lung epithelial cells, and that the binding of an anticorisin neutralizing monoclonal antibody to transglycosylase does not interfere with transglycosylase degradation.

### Example 2

### Inhibitory Effect of the Anticorisin Neutralizing Antibody on Corisin-Induced AE in Pulmonary Fibrosis

It has been shown previously that corisin induces pulmonary fibrosis AE in TGFβ1 TG mice. Based on this finding, an examination was conducted as to whether an anticorisin neutralizing antibody suppresses AE of fibrotic diseases. First, changes in circulating levels of anticorisin monoclonal antibody clone 21A after intraperitoneal administration were evaluated and the half-life thereof was calculated. Five TGFβ1 transgenic mice (TGFβ1 TG mice) with pulmonary fibrosis caused by lung-specific overexpression of a gene encoding full-length human TGFβ1 were injected intraperitoneally with 20 mg/kg of anticorisin antibody clone A21. Blood was collected after 3, 6, 24, 48 168, 336, and 504 hours. After centrifugation, plasma was separated and the levels of anticorisin antibodies were measured using enzyme immunoassays as described in Materials and Method. As a result, the average half-life in plasma of the anticorisin neutralizing monoclonal antibody was 3.8 ±2.5 days (FIG. 17).

Next, TGFβ1 TG mice with pulmonary fibrosis were then assigned to two groups having corresponding computed tomography (CT) scan scores (FIG. 18a, b). CT examinations were performed as follows.

Lung CT was performed using a LaTheta LCT-200 micro CT purchased from Hitachi Aloka Medical (Tokyo, Japan). Mice were anesthetized by inhalation of isoflurane and then placed in the prone position to collect data (Non-Patent Literature 19, Reference Literature 61). Seven to nine specialists in pulmonary radiology, blinded to treatment groups, scored the CT findings on the TGFβ1 TG mice with pulmonary fibrosis based on previously reported and validated criteria as follows. Score 1, Normal lung findings; score 2, interim findings; score 3, mild pulmonary fibrosis; score 4, interim findings; score 5, moderate pulmonary fibrosis; score 6, interim findings; and score 7, progressive pulmonary fibrosis (Non-Patent Literature 19). The same criteria were used to score pulmonary inflammation and fibrosis in a BLM-induced pulmonary fibrosis mouse model (FIG. 21b).

A group of fibrosis-free TGFβ1 TG mice administered intratracheal corisin served as a control (FIG. 18a, b). Anticorisin monoclonal antibody clone 21A was administered to one group having pulmonary fibrosis, and an unrelated isotype antibody was injected intraperitoneally once every three days in the two weeks prior to the intratracheal instillation of corisin in order to induce AE in another group having pulmonary fibrosis (FIG. 19a). That is, the anticorisin monoclonal antibody (mAtb) was injected intraperitoneally into a group of TGF β1-TG mice (n=6) with pulmonary fibrosis, and an isotype antibody (Atb) was administered to another group (n=6) with pulmonary fibrosis five times, two days prior. Intratracheal Instillation of Corisin Only corisin was administered intratracheally to a TGFβ1-TG mouse group (n=5) without fibrosis. For comparison, CT was performed before and after intratracheal instillation of corisin or physiological saline.

As a result, the control TGFβ1 TG mice without fibrosis which were treated with physiological saline, an isotype antibody, or the anticorisin antibody showed no change (FIG. 18b). The TGFβ1 TG mice administered corisin and treated with an isotype antibody showed significantly worse CT findings (FIG. 19c), whereas mice treated with the anticorisin monoclonal antibody showed significant improvement in pulmonary fibrosis CT scores (FIG. 19d).

Next, whether or not the monoclonal anticorisin antibody suppresses acute exacerbation (AE) of pulmonary fibrosis in TGFβ1 TG mice was evaluated.

TGFβ1 TG mice were randomly assigned to two groups with matching grades of pulmonary fibrosis and one group without pulmonary fibrosis. The anticorisin monoclonal antibody was injected intraperitoneally into a group of TGFβ1-TG mice (n=6) with pulmonary fibrosis, and the isotype antibody was administered to another group (n=6) with pulmonary fibrosis five times, two days prior. Corisin was instilled intratracheally. Only corisin was administered intratracheally to the TGFβ1-TG mouse group (n=5) without fibrosis. Following euthanasia by anesthetic overdose, bronchoalveolar lavage fluid (BALF) was collected from each group of mice, the BALF fluid was centrifuged, and the pellets were used to evaluate total cell counts and cell fractions between treatment groups.

As a result, TGFβ1 TG mice treated with the anticorisin monoclonal antibody had significantly reduced total cell and lymphocyte counts in BALF compared to control and isotype antibody-treated mice (FIG. 20a, b). Lung injury markers such as surfactant protein-D (SP-D), MUC5B, matrix metalloproteinase-1 (MMP-1), decorin, and the like, as well as bronchoalveolar lavage fluid (BALF) levels of SP-D, MUC5B, and MUC-1 were significantly reduced in anticorisin monoclonal antibody-treated mice compared to control and isotype antibody-treated mice (FIG. 20c). Hydroxyproline content and collagen deposition in the lungs were significantly lower in mice treated with the anticorisin monoclonal antibody than in mice treated with the isotype antibody (FIG. 20c, d, e). The grade of pulmonary fibrosis significantly correlated with pulmonary inflammatory cell count (FIG. 20f). These observations indicate that corisin is a potential therapeutic target for AE in pulmonary fibrosis.

### Example 3

### AE-Inducing Action of Corisin in Bleomycin-Induced Pulmonary Fibrosis

Bleomycin (BLM)-induced pulmonary fibrosis in mice or rats is the most characterized and commonly used preclinical model for IPF studies (Reference Literature 28). It is unclear whether corisin exacerbates disease in BLM-induced pulmonary fibrosis. To address this question, an experimental model was developed in wild-type (WT) mice by infusing BLM via osmotic minipumps and administering corisin via intratracheal instillation (FIG. 21a). Bleomycin (BLM) or physiological saline (SAL) was infused from osmotic minipumps implanted subcutaneously in the backs of mice according to the experiment schedule for inducing acute exacerbation in mice with bleomycin-induced pulmonary fibrosis shown in FIG. 21a. Computed tomography (CT) scans were performed before and after intratracheal administration of corisin or a scrambled peptide before mouse euthanasia. Mice administered SAL via osmotic minipumps and intratracheal corisin or a scrambled peptide were used as a control. Mice were euthanized 22 days after BLM or SAL pump implantation.

After confirming pulmonary fibrosis onset by chest CT, autologous CT score criteria (FIG. 21b) were used to assign mice into two groups with concordant CT scores.

Wild-type (WT) mice were administered bleomycin (BLM) by osmotic minipump on day 0 and intratracheally instilled (75 µL) with corisin or a scrambled peptide (300 µg/mouse) on day 20 post-pump implantation. CTs were performed as described in Example 2. As a result, the CT scores of mice with pulmonary fibrosis worsened significantly after intratracheal corisin administration (FIG. 22a, b, c). Next, wild-type (WT) mice were administered physiological saline (SAL) by osmotic minipump on day 0 and intratracheally instilled (75 µL) with corisin or a scrambled peptide (300 µg/mouse) on day 20 post-pump implantation. As a result, CT scores did not change in mice with pulmonary fibrosis which were intratracheally instilled with a scramble peptide or mice without pulmonary fibrosis which received either intratracheal corisin or a scrambled peptide (FIG. 22d, e, f).

Next, inflammatory cells and the like in mice with BLM-induced pulmonary fibrosis which had been administered intratracheal corisin were examined. Wild-type (WT) mice were administered bleomycin (BLM) or physiological saline (SAL) by osmotic minipump on day 0 and intratracheally instilled (75 µL) with corisin or a scrambled peptide (300 µg/mouse) on day 20 post-pump implantation. BALF total cell counts were determined using a NucleoCounter and cell fraction counts were conducted after Giemsa staining using WindRoof image software. Levels of serum amyloid P component (SAP), MUC-1, monocyte chemoattractant protein-1 (MCP-1), periostin, collagen I, and osteopontin were measured by enzyme immunoassay according to the manufacturer's instructions. As a result, the total number of lymphocytes in BALF increased significantly in the mice with BLM-induced pulmonary fibrosis which had been administered intratracheal corisin compared to the control mice which had been administered the intratracheal scrambled peptide. Additionally, the circulation level of serum amyloid P component increased, and the levels of MUC-1, monocyte chemoattractant protein-1 (MCP-1), periostin, collagen I, and osteopontin also increased (FIG. 23a, b, c).

Next, caspase-3 cleavage was evaluated by Western blotting and quantified. Caspase-3 cleavage was significantly increased in the mice administered intratracheal corisin compared their counterparts, the mice which had been administered the scrambled peptide (FIG. 23d, e). Collagen deposition was evaluated using Masson's trichrome staining and quantified using WindRoof imaging software. Pulmonary tissue content of hydroxyproline was measured by colorimetric analysis using a commercially available kit in accordance with the manufacturer's instructions. As a result, collagen deposition and hydroxyproline content in the lungs were also increased in the mice administered corisin compared to the mice administered the scrambled peptide (FIG. 23f, g, h). Mice administered physiological saline via osmotic minipump and treated with corisin or the scrambled showed no significant changes. These findings indicate that corisin also exacerbates disease in BLM-induced pulmonary fibrosis.

### Example 4

### Suppressive Effect of the Anticorisin Monoclonal Antibody on BLM-induced Pulmonary Fibrosis

Since corisin aggravates BLM-induced pulmonary fibrosis, it was expected that this lethal peptide would play a role in this experimental model. In order to verify this prediction, mice were infused with BLM via osmotic minipump and treated with the anticorisin neutralizing monoclonal antibody or an isotype antibody via an intraperitoneal route three times weekly for three weeks (FIG. 24a). Wild-type (WT) mice were administered bleomycin (BLM) by osmotic minipump and treated with the anticorisin monoclonal antibody (WT/BLM/anticorisin) or isotype antibody (WT/BLM/isotype) via an intraperitoneal route three times weekly for three weeks. WT mice administered physiological saline (SAL) by osmotic minipump and treated with the anticorisin monoclonal antibody (WT/SAL/anticorisin) or isotype antibody (WT/SAL/isotype) via an intraperitoneal route three times weekly for three weeks were used as control mice. Computed tomography (CT) scans were taken one day prior to euthanasia of the mice, and nine experts with no knowledge of the treatment groups recorded the CT findings. The mice were euthanized on day 21 after BLM or physiological saline (SAL) pump implantation. The mice administered physiological saline (SAL) via osmotic minipump were control mice prepared to exclude secondary effects of the antibodies.

As a result, the mice with BLM-induced pulmonary fibrosis which had been treated with the anticorisin monoclonal antibody showed a significant decrease in CT fibrosis scores compared with the corresponding mice treated with the isotype antibody (FIG. 24b, c). There were no CT changes in the mice treated with the anticorisin monoclonal antibody or the isotype antibody after physiological saline infusion.

Next, BALF total cell counts were determined using a NucleoCounter and cell fraction counts were conducted after Giemsa staining using WindRoof image software. Evaluation of inflammatory cells in BALF showed that the total number of all cell types, as well as the total number of lymphocytes and neutrophils, were significantly reduced in the mice with BLM-induced pulmonary fibrosis which had been treated with the anticorisin monoclonal antibody compared to isotype antibody-treated mice (FIG. 24d, e).

In addition, acute tissue damage, parenchymal cell apoptosis, and tissue fibrosis in the lungs of mice with pulmonary fibrosis treated with the anticorisin monoclonal antibody were examined. Wild-type (WT) mice were administered bleomycin (BLM) by osmotic minipump and treated with the anticorisin monoclonal antibody (WT/BLM/anticorisin) or isotype antibody (WT/BLM/isotype) via an intraperitoneal route three times weekly for three weeks. WT mice administered physiological saline (SAL) by osmotic minipump and treated with the anticorisin monoclonal antibody (WT/SAL/anticorisin) or isotype antibody (WT/SAL/isotype) via an intraperitoneal route three times weekly for three weeks were used as control mice. Levels of osteopontin, MUC-1, and MUC5B were measured by enzyme immunoassay using a commercially available kit. DNA fragmentation was evaluated by staining with terminal deoxynucleotide transferase dUTP nick-end labeling (TUNEL). Grades of pulmonary fibrosis were scored blind by seven experts using the Ashcroft score. Collagen deposition in the lungs was evaluated using Masson's trichrome staining and quantified using WindRoof image software. Pulmonary tissue content of hydroxyproline was measured by colorimetric analysis using a commercially available kit in accordance with the manufacturer's instructions.

As a result, the mice with BLM-induced pulmonary fibrosis which had been treated with the anticorisin monoclonal antibody showed low plasma and BALF levels of osteopontin, MUC-1, and MUC5B, a reduction in the number of apoptotic lung epithelial cells, and a decrease in Ashcroft score and collagen deposition and hydroxyproline in the lungs (FIG. 25a, b, c, d, e, f, g, h) compared to the mice which had been administered the isotype antibody. Overall, these findings indicate that corisin also plays a role in the onset of BLM-induced pulmonary fibrosis.

### Example 5

### Amelioration Effect of the Anticorisin Monoclonal Antibody on BLM-induced Lung Injury Pulmonary Fibrosis when Administered During the Acute Phase

Lung injury induced by BLM subcutaneously administered via an osmotic minipump is characterized by acute phase pneumonia, which peaks on day 11, followed by chronic phase pulmonary fibrosis (Reference Literature 29). An investigation was carried out as to whether the anticorisin monoclonal antibody ameliorates pulmonary fibrosis by inhibiting BLM-induced acute stage lung injury in the early acute phase.

Bleomycin (BLM) was infused via osmotic minipumps subcutaneously implanted in the backs of mice. On days 2, 4, 6, 9, and 11 post-infusion, the mice were treated via an intraperitoneal route with an isotype antibody (WT/BLM/isotype) or the anticorisin monoclonal antibody (WT/BLM/anticorisin) at a dose of 20 mg/kg. Mice were not treated during the chronic phase from day 12 until euthanasia on day 22. Blood sampling was performed on day 9 and computed tomography (CT) scans were taken on day 9 prior to euthanasia of the mice. The mice were euthanized on day 22 after BLM pump implantation. CT lung opacity was measured in each group using WindRoof image software. Blood was sampled on day 9 after implantation of the BLM pumps. Total cell numbers in the blood were determined using a NucleoCounter and cell fractions were counted after Giemsa staining. WindRoof imaging software was used.

As a result, CT findings for lung injury and the number of neutrophils in the blood were significantly improved on day 9 in the mice treated with the anticorisin monoclonal antibody (FIG. 26b, c, d, e) compared to the controls treated with the isotype antibody, IgG2.

Next, wild-type (WT) mice were administered bleomycin (BLM) by osmotic minipump and treated with the anticorisin monoclonal antibody (WT/BLM/anticorisin) or isotype antibody (WT/BLM/isotype) via an intraperitoneal route on days 2, 4, 6, 9, and 11 following bleomycin infusion via pump. CTs were performed on day 21 after BLM pump implantation (chronic phase). Furthermore, bronchoalveolar lavage fluid (BALF) was collected 22 days after BLM pump implantation. BALF total cell counts were determined using a NucleoCounter and differential cell counts were conducted after Giemsa staining using WindRoof image software.

As a result, the mice treated with the anticorisin monoclonal antibody showed significant improvement in CT fibrosis scores and lymphocyte counts in BALF during the chronic phase of the disease (on day 22 after commencement of BLM infusion) compared to the mice which received the control antibody (FIG. 27a, b, c, d).

Lung injury, pulmonary parenchymal apoptosis, and collagen deposition on day 22 were also examined in the mice with bleomycin-induced pulmonary fibrosis treated with the anticorisin monoclonal antibody during the acute phase of the disease. Wild-type (WT) mice were administered bleomycin (BLM) by osmotic minipump and treated with the anticorisin monoclonal antibody (WT/BLM/anticorisin) or isotype antibody (WT/BLM/isotype) via an intraperitoneal route on days 2, 4, 6, 9, and 11 following BLM infusion via pump. Bronchoalveolar lavage fluid (BALF) and plasma were collected 22 days after BLM pump implantation. Levels of MUC-1, surfactant protein C (SP-C), SP-D, collagen I, periostin, osteopontin, and total TGFβ1 were measured using a commercially available enzyme immunoassay kit according to the manufacturer's instructions (FIG. 28a). Western blotting of cleaved caspase-3 and β-actin was also performed (FIG. 28b, c). Collagen deposition in the lungs was quantified using Masson's trichrome-stained photographs and WindRoof image software (FIG. 28f, g). The amount of hydroxyproline contained in the lungs was measured by colorimetric analysis using a commercially available kit in accordance with the manufacturer's instructions (FIG. 28h).

As a result, plasma levels of MUC-1, SP-C, SP-D, collagen I, periostin, and osteopontin, and MUC-1 BALF levels and total TGFβ1 on day 22 were significantly lower in the mice treated with the anticorisin monoclonal antibody than in the mice treated with the isotype antibody (FIG. 28a). Compared with the mice treated with the isotype antibody, the mice treated with the anticorisin monoclonal antibody had significantly lower Ashcroft scores, collagen deposition, and hydroxyproline contents, and decreased caspase-3 cleavage in the lungs (FIG. 28b, c, d, e, f, g). These observations indicate that acute phase corisin-related acute lung injury is an important determinant of pulmonary fibrosis in the chronic phase of the disease.

### Example 6

### Prolongation Effect of the Anticorisin Monoclonal Antibody on the Survival Rate of Mice with Pulmonary Fibrosis AE

Computed tomography (CT) scans were performed on human transforming growth factor β1 (TGFβ1) transgenic (TG) mice. Seven experts blindly recorded the grade of pulmonary fibrosis, and the average value of the seven expert scores for each individual mouse was taken as the CT score of the mouse. TGFβ1 TG mice with pulmonary fibrosis were divided into two groups with matching CT fibrosis scores and administered bleomycin (BLM) via osmotic minipumps. One of these two groups was intraperitoneally injected with the anticorisin monoclonal antibody (TGFβ1-TG/BLM/anticorisin), and the other group was intraperitoneally injected with the isotype antibody (TGFβ1-TG/BLM/isotype). The groups were treated with the anticorisin monoclonal antibody or the isotype antibody three times a week for three weeks. TGFβ1 TG mice without pulmonary fibrosis infused with physiological saline via osmotic minipump and treated with the anticorisin monoclonal antibody (TGFβ1-TG/SAL/anticorisin) or isotype antibody (TGFβ1-TG/SAL/isotype) were used as control mice (FIG. 29a, b). Survival rates in mice treated in this manner are shown in FIG. 29c.

As a result, the TGFβ1 TG mice with pulmonary fibrosis and BLM-induced AE treated with the anticorisin monoclonal antibody exhibited significantly longer survival rates than the mice treated with the isotype antibody (FIG. 29c). As expected, there was no change in survival rates in the control group administered physiological saline via osmotic minipump.

### Example 7

### Suppression of Lipopolysaccharide-Induced Acute Lung Injury by the Anticorisin Monoclonal Antibody.

Based on the above results, an investigation was carried out as to whether corisin also affects the onset of acute lung injury caused by other substances such as a lipopolysaccharide (LPS).

Wild-type (WT) mice were treated with an isotype antibody or an anticorisin monoclonal antibody at a dose of 20 mg/kg once a day every other day for a total of three times via an intraperitoneal route. Mice were given an intratracheal (IT) drip with a high dose (150 µg) of a lipopolysaccharide (LPS) or physiological saline (SAL) two days after the final treatment with the antibody and were euthanized two days after LPS infusion. Mice administered intratracheal physiological saline (SAL) and treated with the isotype antibody (WT/SAL/isotype) or the anticorisin antibody (WT/SAL/anticorisin) were used as control mice (FIG. 30a). Bronchoalveolar lavage fluid (BALF) was collected from mice euthanized on day 2 after intratracheal LPS instillation. The obtained BALF cells were counted using a NucleoCounter, and cell fractions were Giemsa stained and counted (FIG. 30b, c). Furthermore, the levels of lactate dehydrogenase A (LDHA), surfactant protein D (SP-D), matrix metalloproteinase-1 (MMP-1), MUC-1, and tumor necrosis factor α (TNF-α) were measured using a commercially available immunoassay kit in accordance with the manufacturer's instructions (FIG. 30d).

Furthermore, an investigation was carried out as to whether treatment with an anticorisin monoclonal antibody improves CT findings in mice with severe lipopolysaccharide-induced acute lung injury. Wild-type (WT) mice were treated with an isotype antibody (WT/LPS/isotype) or an anticorisin antibody (WT/LPS/anticorisin) at a dose of 20 mg/kg via an intraperitoneal route once a day every other day for one week before being euthanized. Mice were given an intratracheal (IT) drip with a high dose (150 µg) of a lipopolysaccharide (LPS) or physiological saline. Mice administered intratracheal physiological saline (SAL) and treated with the isotype antibody (WT/SAL/isotype) or the anticorisin antibody (WT/Sal/anticorisin) were used as control mice. Computed tomography (CT) scans were performed one day after intratracheal instillation of the lipopolysaccharide (LPS) (FIG. 31a). CT opacity from the apex to base regions of the lungs were quantified using WindRoof image software as described in Materials and Method (FIG. 31b).

As a result, the mice treated with the anticorisin monoclonal antibody showed significantly reduced lung CT opacity (FIG. 31a, b), reduced counts of neutrophils infiltrating the lungs, and significantly reduced plasma and BALF levels of lactate dehydrogenase A (LDHA), SP-D, and MMP-1 and BALF levels of MUC-1 and tumor necrosis factor-α (TNF-α) compared to the mice pretreated with the isotype control. This indicates that corisin is involved in the acute inflammatory response to LPS (FIG. 30c, d).

Next, an investigation was carried out as to whether the anticorisin monoclonal alleviates moderate lipopolysaccharide-induced acute lung injury. WT mice were treated with an isotype antibody or an anticorisin monoclonal antibody at a dose of 20 mg/kg once a day every other day for a total of three times via an intraperitoneal route. Mice were given IT instillations of a low dose (75 µg) of LPS 2 days after the final treatment with the antibody and were euthanized two days after LPS instillation. BALF was then collected. BALF cells were counted using a NucleoCounter and stained with Giemsa for cell fraction counting as described in Materials and Method (FIG. 32b, c). BALF and plasma levels of surfactant protein D (SP-D), monocyte chemoattractant protein-1 (MCP-1), TNFα, lactate dehydrogenase A (LDHA), MUC-5B, and matrix metalloproteinase-1 (MMP-1) were measured using a commercially available immunoassay kit in accordance with the manufacturer's instructions (FIG. 32d).

As a result, when a smaller amount of intratracheal LPS was used in this manner to conduct another experiment to induce a milder degree of acute lung injury, the mice pretreated with the anticorisin monoclonal antibody had significantly reduced counts of neutrophils infiltrating the lungs, significantly reduced plasma and BALF levels of SP-D, monocyte chemoattractant protein-1 (MCP-1), and TNFα, and significantly reduced BALF levels of LDHA and plasma levels of MUC5B and MMP-1 compared to the control mice pretreated with the isotype (FIG. 32a, b, c, d). Overall, these observations also further support the involvement of corisin in the onset of acute lung injury.

### Example 8

### Plasma Levels of Corisin as a Potential Biomarker for Pulmonary Fibrosis

The incidence and frequency of AE in IPF patients predicts the acceleration of pulmonary fibrosis and undesirable clinical consequences (Non-Patent Literature 1). It was predicted that circulating corisin may constitute a biomarker for pulmonary fibrosis. In order to verify this prediction, an enzyme immunoassay using the anticorisin monoclonal antibody was designed.

### 8-1: Measurement of Corisin

An enzyme immunoassay method using the anticorisin monoclonal antibody clone 9A as a capture antibody and a biotinylated anticorisin monoclonal antibody as a detection antibody was developed. Simply stated, the capture antibody was coated overnight on a 96-well plate at 4°C in phosphate-buffered saline at a final concentration of 2 µg/mL. After blocking with 1% bovine serum albumin in phosphate-buffered saline and adequately washing with phosphate, buffered saline in Tween, various reference concentrations of corisin, and plasma samples were added to the wells and incubated overnight at 4°C. The wells were then washed before adding horseradish peroxidase-conjugated streptavidin (R&D Systems) to phosphate-buffered saline. After adequate washing and incubation, a substrate solution was added for color development and absorbance was read at 450 nm. Values were extrapolated from a standard curve drawn using several concentrations of corisin.

### 8-2: Evaluation of Corisin as a Potential Biomarker for Pulmonary Fibrosis

Plasma levels of corisin in wild-type (WT) mice and TGFβ1 transgenic mice (TG) with pulmonary fibrosis caused by lung-specific overexpression of a gene encoding full-length human TGFβ1 were measured to evaluate correlation with fibrosis markers (FIG. 33a). Specifically, transforming growth factor β1 (TGFβ1) transgenic (TG) mice were genotyped, computed tomography (CT) scans were performed, and the findings regarding the extent of pulmonary fibrosis were recorded. The mice were euthanized for blood and lung tissue sampling. Ashcroft scores for pulmonary fibrosis, and plasma corisin and pulmonary hydroxyproline measurements were taken as described in Materials and Method.

As a result, as expected, the computed tomography (CT) scores for pulmonary fibrosis, the Ashcroft scores for fibrosis, and the pulmonary hydroxyproline content in the TGFβ1 TG mice were significantly increased compared to the WT mice (FIG. 32b; FIG. 34a, b). The TGFβ1 TG mice had significantly elevated plasma concentrations of corisin compared to the WT mice, and there was significant correlation between plasma concentrations of corisin and CT fibrosis scores, Ashcroft scores, and pulmonary hydroxyproline content. This supports the potential application of circulating corisin as a biomarker for disease progression in pulmonary fibrosis (FIG. 31b).

### Example 9

### Preparation of Vaccine

The amino acid sequence at the corisin position was examined for several transglycosylases of the same family as the corisin-containing transglycosylase of Staphyloccocus nepalensis (that is, S. nepalensis strain CNDG protein 00351), as shown in the alignment of FIG. 4. It is very clear that certain species of bacteria have alterations in amino acids in specific locations. A Weissella confusa (confusa) corisin peptide showed two amino acid alterations, Asp14Ser (or N14S) and Ala16Asp (or A16N). All nomenclature of spontaneous mutations/alterations is based on the S. nepalensis corisin number. That is, position 1 is isoleucine (I) and position 19 is arginine (R). As illustrated in FIG. 3, (3) a W. confusa peptide (Shem_00350 or 1b, 7b, or 12b) was synthesized (accession number WP_112464134.1), and the apoptotic activity thereof was compared to a Staphylococcus haemolyticus corisin peptide having the same sequence except for the two alterations indicated in the W. conf peptide (that is, N14S and A16N-peptide accession number WP_112464134.1). As shown in FIG. 3 (3), it is very clear that these mutations dramatically reduced the apoptotic activity of the W. confusa peptide (25% of the activity of Shem_00350 or 1b, 7b, or 12b). Another example of a mutation that reduces the apoptosis-inducing ability of the corisin peptide is Mycobacterium abscessus corisin peptide 1 (Absc-1 in the Myco alignment or Genbank accession number SKT 99287.1). The Myco abc-1 peptide has three amino acid alterations and one amino acid deletion compared to the S. haemolyticus corisin peptide, as shown in the alignment (4) of FIG. 4. Data concerning the apoptosis-inducing ability illustrated in FIG. 3 (3) shows that the alteration and deletion of amino acids brought about a much lower induction ability of the Myabs-1 peptide (approximately 30% of the activity of hemolytic streptococcal peptide-Shem 00350 or 1b, 7b, or 12b). The opposite effect was observed in the original corisins; that is, the Staphyloccocus nepalensis strain CNDG peptide (or CNDG-00351 in the alignment) and the Staphylococcus haemolyticus peptide (Shem_00350 or 1b, 7b, or 12b). Here, there was a single amino acid alteration in the Staphyloccocus nepalensis peptide compared to the Staphyloccocus haemolyticus peptide (that is, Gly 7 Ser or G7S in the S. nepalensis peptide). Unlike the previous examples, the apoptosis-inducting activity was always slightly higher compared to the Staphyloccucus haemolyticus peptide.

Based on the above analysis, amino acid mutations were made in corisin, and the following three peptides were synthesized.
Corisin N14S: IVMPESSGNPNAVSPAGYR (SEQ ID NO: 55)
Corisin P15A: IVMPESSGNPNAVNAAGYR (SEQ ID NO: 56)
Corisin A16N: IVMPESSGNPNAVNPNGYR (SEQ ID NO: 57)

After synthesis, the activity thereof was compared with the parent (peptide) sequence (IVMPESSGNPNAVNPAGYR) to determine whether activity was decreased or not. It was predicted that the corisin peptide would become inactive when one amino acid was altered. The obtained results are shown in FIG. 35. As predicted, corisin N14S and P15A were found to be as devoid of apoptotic activity, equivalent to a non-functional scrambled peptide.

Next, amino acid changes will be made (since antibody binding is thought to occur on the N-terminus side half, the focus is on the C-terminus side half). Currently, alterations to one amino acid are being investigated, but based on these results, apoptosis inductivity of the corisin peptide should be completely eliminated by combining two to three amino acids. A dead (or attenuated) peptide may be tested in a mouse model via either intramuscular injection or intranasal (inhalation) administration in order to determine the ability thereof to protect mouse patients from a corisin-releasing bacterium or from administration of a corisin peptide.

### Example 10

### Additional Activity of Corisin

Caki-2 cell line derived from proximal tubular epithelium, HaCaT keratinocyte cell line derived from adult skin, ARPE-19 retinal pigment epithelial cell line, Caco-2 cell line derived from colon cancer, and HIEC-6 cell line derived from normal human small intestinal epithelial cells were obtained from the American Type Culture Collection (American Type Culture Collection; Manassas, VA). RPMI 1640 culture medium was purchased from Sigma-Aldrich (St. Louis, Missouri) and fetal bovine serum (FBS) was purchased from Bio Whittaker (Bio Whittaker; Walkersville, Maryland). L-glutamine, penicillin, and streptomycin were obtained from Invitrogen (Carlsbad, California). Caki-2 cells, Caco-2 cells, and ihPOD cells were cultured in RPMI 1640 culture medium, HaCaT cells in DMEM culture medium, ARPE-19 cells in DMEM/F12 culture medium, and HIEC-6 cells in DMEM culture medium.

Each cell line was cultured until subconfluent to examine caspase-3 cleavage by Western blotting. Next, the cells were washed and cultured overnight in a culture medium that did not contain FCS. Cells were stimulated with 10 µg/mL of a scrambled peptide, corisin, or corisin-like peptide, cultured for 24 hours, and then Western blotting was performed.

The obtained results are shown in FIG. 39. Examination of caspase-3 activity showed that cleavage of caspase-3 was significantly increased in all cells treated with corisin and the corisin-like peptide compared to cells treated with the scrambled peptide, indicating activation of apoptotic pathways. That is, FIG. 39 shows that corisin induces apoptosis in large intestinal mucosal epithelial cells, small intestinal mucosal epithelial cells, keratinocytes of the skin, retinal epithelial cells, kidney podocytes, and ureteral epithelial cells. Based on these results, the anticorisin monoclonal antibody of the present invention can be expected to have a therapeutic effect on not only pulmonary fibrosis, but also diseases caused by these cells, such as ulcerative colitis, Crohn's disease, retinopathy, nephritis and renal fibrosis, chronic dermatitis, scleroderma, and cirrhosis of the liver.

### Example 11

### Comparison of Serum Corisin Concentrations

An enzyme immunoassay method was developed using a rabbit polyclonal anti-transglycosylase polyclonal antibody and a biotinylated anticorisin 9A monoclonal antibody. Corisin levels in serum were compared in healthy subjects, patients with stable idiopathic pulmonary fibrosis (IPF), and IPF patients with acute exacerbation.

The obtained results are shown in FIG. 40. FIG. 40 shows that the corisin concentration in serum was significantly higher in the stable IPF patients compared to the healthy subjects, and was also significantly higher in the IPF patients with acute exacerbation than in the stable IPF patients. Based on these results, the usefulness of corisin measurement as a biomarker for IPF patients became evident.

### Example 12

### Native Corisin Exhibits Stronger Apoptotic Activity Than Synthetic Corisin

Previous experiments showed that synthetic corisin has apoptotic activity at a concentration of 10 µg/mL. An investigation was carried out as to how much activity native corisin secreted by bacteria has compared to synthetic corisin. First, the apoptotic activity of a bacterial culture supernatant was examined. Ten-fold (1/10) diluted Staphylococcus nepalensis culture supernatant was added to a culture medium of A59 alveolar epithelial cells in the presence or absence of the anticorisin monoclonal antibody (clone A21) (20 µg/mL). After 48 hours of culture, apoptosis of the A549 cells was investigated by flow cytometry analysis.

The obtained results are shown in FIG. 41a, b, and c. FIG. 41a shows that apoptosis of the A549 cells by 10-fold diluted bacterial supernatant is significantly and almost completely suppressed by the anticorisin monoclonal antibody.

Next, the concentration of native corisin in an undiluted bacterial supernatant was measured using the enzyme immunoassay method.

The obtained results are shown in FIG. 41b and c. FIG. 41b and c show that the mean concentration of native corisin in an undiluted culture supernatant of Staphylococcus nepalensis was 1042 ±174.6 pg/mL and the mean concentration of native corisin in undiluted culture supernatant of Staphylococcus haemolyticus was 802.4 ±57.7 pg/mL.

Based on the above results, because the bacterial supernatant was used at a dilution of 1:10 in the experiment to induce apoptosis in A549 cells, it became evident that native corisin has strong biological activity at a concentration of approximately 100 pg/mL. Synthetic corisin exhibits apoptotic activity at a concentration of 10 µg/mL, but native corisin exhibits apoptotic activity at a concentration of approximately 100 pg/mL, as illustrated in FIG. 41a, b, and c. Therefore, it was found that native corisin has apoptotic activity 100,000 times that of synthetic corisin (10 µg/100 pg = 100,000).

### Example 13

### The Anticorisin Monoclonal Antibody (Clone A21) Also Suppresses the Apoptotic Activity of Native Corisin in Human Bodily Fluid (Bronchoalveolar Lavage Fluid)

Experiments similar to those above were performed to examine pro-apoptotic activity of native corisin present in the bronchoalveolar lavage fluid (BALF) of patients with IPF. BALF collected from IPF patients with acute exacerbation (n = 14) and healthy subjects (n = 5) was added to a culture medium of A59 alveolar epithelial cells in the presence of the anticorisin monoclonal antibody (clone A21) or a control IgG (20 µg/mL), and apoptosis was examined by flow cytometry after 48 hours.

FIG. 41d shows the results obtained by each IPF patient or each healthy subject. FIG. 41e shows the results obtained for all IPF patients or all healthy subjects. FIG. 41e shows that the anticorisin monoclonal antibody of the present invention significantly suppressed the apoptotic activity of BALF in IPF patients. On the other hand, FIG. 41f shows the concentration (650.9 ±218.3 pg/mL) of native corisin in the BALF of the IPF patients (n = 14) and the concentration (458.4 ±60.7 pg/mL) of native corisin in the BALF of the healthy subjects (n = 5). A 1:2 dilution of BALF was used in the experiment to induce apoptosis in A549 cells. Therefore, it was found that native corisin in human bodily fluids also has potent biological activity.

Furthermore, FIG. 41g shows the half-life of corisin in a test tube and in cell culture supernatant. In order to ascertain how quickly corisin is metabolized in a cell culture system, the half-life of corisin was investigated. A549 alveolar epithelial cells were cultured in DMEM to which 10% fetal bovine serum had been added until confluent. Next, corisin was inoculated into a cell culture medium at a final concentration of 10 micrograms/mL, and cell supernatant was collected after 0, 1, 3, 6, 12, and 24 hours. The concentration of corisin was measured by an enzyme immunoassay method, and the half-life of corisin was examined. The results revealed that, as shown in FIG. 41g, the half-life of (synthetic) corisin in the solution is less than 1 hour in a human alveolar cell culture system. It can be seen from these results that the concentration of (native) corisin in the serum of IPF patients increases at the onset of acute exacerbation, but the concentration quickly decreases due to its short half-life. Therefore, serum corisin measurements should be performed immediately after the onset of acute exacerbation.

The following are the reference literature cited in the present Specification.

### Reference Literature

20. Argemi, X., Hansmann, Y., Prola, K. & Prevost, G. Coagulase-Negative Staphylococci Pathogenomics. Int JMol Sci20, doi:10.3390/ijms20051215 (2019).
21. Czekaj, T., Ciszewski, M. & Szewczyk, E. M. Staphylococcus haemolyticus - an emerging threat in the twilight of the antibiotics age. Microbiology (Reading) 161, 2061-2068, doi:10.1099/mic.0.000178 (2015).
22. Yin, Y. et al. A hybrid sub-lineage of Listeria monocytogenes comprising hypervirulent isolates. Nat Commun 10, 4283, doi:10.1038/s41467-019-12072-1 (2019).
23. Johansen, M. D., Herrmann, J. L. & Kremer, L. Non-tuberculous mycobacteria and the rise of Mycobacterium abscessus. Nat Rev Microbiol 18, 392-407, doi:10.1038/s41579-020-0331-1 (2020).
24. Fusco, V. et al. The genus Weissella: taxonomy, ecology and biotechnological potential. Front Microbiol 6, 155, doi:10.3389/fmicb.2015.00155 (2015).
25. Bedoui, S., Herold, M. J. & Strasser, A. Emerging connectivity of programmed cell death pathways and its physiological implications. Nat Rev Mol Cell Biol 21, 678-695, doi:10.1038/s41580-020-0270-8 (2020).
26. Batandier, C., Leverve, X. & Fontaine, E. Opening of the mitochondrial permeability transition pore induces reactive oxygen species production at the level of the respiratory chain complex I. J Biol Chem 279, 17197-17204, doi:10.1074/jbc.M310329200 (2004).
27. Du, C., Fang, M., Li, Y., Li, L. & Wang, X. Smac, a mitochondrial protein that promotes cytochrome c-dependent caspase activation by eliminating IAP inhibition. Cell 102, 33-42, doi:10.1016/s0092-8674(00)00008-8 (2000).
28. Jenkins, R. G. et al. An Official American Thoracic Society Workshop Report: Use of Animal Models for the Preclinical Assessment of Potential Therapies for Pulmonary Fibrosis. Am J Respir Cell Mol Biol 56, 667-679, doi:10.1165/rcmb.2017-0096ST (2017).
29. Urawa, M. et al. Protein S is protective in pulmonary fibrosis. / Thromb Haemost 14, 1588-1599, doi:10.1111/jth.l3362 (2016).
30. Drakopanagiotakis, F., Xifteri, A., Polychronopoulos, V. & Bouros, D. Apoptosis in lung injury and fibrosis. Eur Respir J32, 1631-1638, doi:10.1183/09031936.00176807 (2008).
31. D'Alessandro-Gabazza, C. N. et al. Development and preclinical efficacy of novel transforming growth factor-betal short interfering RNAs for pulmonary fibrosis. Am J Respir Cell Mol Biol46, 397-406, doi:10.1165/rcmb.2011-01580C (2012).
32. Upagupta, C., Shimbori, C., Alsilmi, R. & Kolb, M. Matrix abnormalities in pulmonary fibrosis. Eur Respir Rev 27, doi:10.1183/16000617.0033-2018 (2018).
33. Ryerson, C. J., Cottin, V., Brown, K. K. & Collard, H. R. Acute exacerbation of idiopathic pulmonary fibrosis: shifting the paradigm. Eur Respir J 46, 512-520, doi:10.1183/13993003.00419-2015 (2015).
34. Jeon, K. et al. Prognostic factors and causes of death in Korean patients with idiopathic pulmonary fibrosis. RespirMedlOO, 451-457, doi:10.1016/j.rmed.2005.06.013 (2006).
35. Kondoh, Y. et al. Risk factors of acute exacerbation of idiopathic pulmonary fibrosis. Sarcoidosis Vase Diffuse Lung Dis 27, 103-110 (2010).
36. Raghu, G. et al. Diagnosis of Idiopathic Pulmonary Fibrosis. An Official ATS/ERS/JRS/ALAT Clinical Practice Guideline. Am J Respir Crit Care Med 198, e44-e68, doi:10.1164/rccm.201807-1255ST (2018).
37. Kreuter, M. & Maher, T. M. Treatment of Acute Exacerbation of Idiopathic Pulmonary Fibrosis. A Call to Arms. Am J Respir Crit Care Med 201, 1030-1032, doi:10.1164/rccm.202001 -0057ED (2020).
38. Collard, H. R. et al. Suspected acute exacerbation of idiopathic pulmonary fibrosis as an outcome measure in clinical trials. Respir Res 14, 73, doi:10.1186/1465-9921-14-73 (2013).
39. Barbas-Filho, J. V. et al. Evidence of type II pneumocyte apoptosis in the pathogenesis of idiopathic pulmonary fibrosis (IFP)/usual interstitial pneumonia (UIP). J Clin Pathol54, 132-138, doi:10.1136/jcp.54.2.132 (2001).
40. Sauler, M., Bazan, I. S. & Lee, P. J. Cell Death in the Lung: The Apoptosis-Necroptosis Axis. Annu Rev Physiol 81, 375-402, doi:10.1146/annurev-physiol-020518-114320 (2019).
41. Uhal, B. D. etal. Fibroblasts isolated after fibrotic lung injury induce apoptosis of alveolar epithelial cells in vitro. Am J Physiol 269, L819-828, doi:10.1152/ajplung.l995.269.6.L819 (1995).
42. Bellani, G. et al. Epidemiology, Patterns of Care, and Mortality for Patients With Acute Respiratory Distress Syndrome in Intensive Care Units in 50 Countries. JAMA 315, 788-800, doi:10.1001/jama.2016.0291 (2016).
43. Force, A. D. T. et al. Acute respiratory distress syndrome: the Berlin Definition. JAMA 307, 2526-2533, doi:10.1001/jama.2012.5669 (2012).
44. Marchioni, A. et al. Acute exacerbation of idiopathic pulmonary fibrosis: lessons learned from acute respiratory distress syndrome? Crit Care 22, 80, doi:10.1186/sl3054-018-2002-4 (2018).
45. Matthay, M. A. et al. Acute respiratory distress syndrome. Nat Rev Dis Primers 5, 18, doi:10.1038/s41572-019-0069-0 (2019).
46. Ware, L. B. & Matthay, M. A. The acute respiratory distress syndrome. NEnglJ Med342, 1334-1349, doi:10.1056/NEJM200005043421806 (2000).
47. Domscheit, H., Hegeman, M. A., Carvalho, N. & Spieth, P. M. Molecular Dynamics of Lipopolysaccharide-Induced Lung Injury in Rodents. Front Physiol 11, 36, doi:10.3389/fphys.2020.00036 (2020).
48. Menezes, S. L. et al. Pulmonary and extrapulmonary acute lung injury: inflammatory and ultrastructural analyses. J Appl Physiol (1985) 98, 1777-1783, doi:10.1152/japplphysiol.01182.2004 (2005).
49. Chiba, H., Otsuka, M. & Takahashi, H. Significance of molecular biomarkers in idiopathic pulmonary fibrosis: A mini review. Respir Investig 56, 384-391, doi:10.1016/j.resinv.2018.06.001 (2018).
50. Collard, H. R. et al. Plasma biomarker profiles in acute exacerbation of idiopathic pulmonary fibrosis. Am J Physiol Lung Cell Mol Physiol 299, L3-7, doi:10.1152/ajplung.90637.2008 (2010).
51. Moodley, Y. P. et al. Analysis by proteomics reveals unique circulatory proteins in idiopathic pulmonary fibrosis. Respirology 24, 1111-1114, doi:10.1111/resp.l3668 (2019).
52. O'Dwyer, D. N. et al. The peripheral blood proteome signature of idiopathic pulmonary fibrosis is distinct from normal and is associated with novel immunological processes. Sci Repl, 46560, doi:10.1038/srep46560 (2017).
53. Rosas, I. O. etal. MMP1 and MMP7 as potential peripheral blood biomarkers in idiopathic pulmonary fibrosis. PLoS Med5, e93, doi:10.1371/journal.pmed.0050093 (2008).
54. Todd, J. L. et al. Peripheral blood proteomic profiling of idiopathic pulmonary fibrosis biomarkers in the multicentre IPF-PRO Registry. Respir Res 20, 227, doi:10.1186/sl2931-019-1190-z (2019).
55. White, E. S. et al. Plasma Surfactant Protein-D, Matrix Metalloproteinase-7, and Osteopontin Index Distinguishes Idiopathic Pulmonary Fibrosis from Other Idiopathic Interstitial Pneumonias. Am J Respir Crit Care Med 194, 1242-1251, doi:10.1164/rccm.201505-08620C (2016).
56. Yang, I. V. et al. The peripheral blood transcriptome identifies the presence and extent of disease in idiopathic pulmonary fibrosis. PLoS One 7, e37708, doi:10.1371/journal.pone.0037708 (2012).
57. Maranville, J. C. & Di Rienzo, A. Combining genetic and nongenetic biomarkers to realize the promise of pharmacogenomics for inflammatory diseases. Pharmacogenomics 15, 1931-1940, doi:10.2217/pgs.l4.129 (2014).
58. Wang, J. et al. [Research advances in the mechanism of pulmonary fibrosis induced by coronavirus disease 2019 and the corresponding therapeutic measures]. Zhonghua Shao ShangZaZhi36, 691-697, doi:10.3760/cma.j.cn501120-20200307-00132 (2020).
59. Das, KM et al. Follow-up chest radiographic findings in patients with MERS-CoV after recovery. Indian J Radiol Imaging 27, 342-349, doi:10.4103/ijri.IJRI_469_16 (2017).
60. George, P. M., Wells, A. U. & Jenkins, R. G. Pulmonary fibrosis and COVID-19: the potential role for antifibrotic therapy. LancetRespirMed8, 807-815, doi:10.1016/S2213-2600(20)30225-3 (2020).
61. Fujiwara, K. et al. Inhibition of Cell Apoptosis and Amelioration of Pulmonary Fibrosis by Thrombomodulin. Am JPathoim, 2312-2322, doi:10.1016/j.ajpath.2017.06.013 (2017).
62. Koren, S. et al. Canu: scalable and accurate long-read assembly via adaptive k-mer weighting and repeat separation. Genome Res 21, 722-736, doi:10.1101/gr.215087.116 (2017).
63. Loman, N. J., Quick, J. & Simpson, J. T. A complete bacterial genome assembled de novo using only nanopore sequencing data. Nat Methods 12, 733-735, doi:10.1038/nmeth.3444 (2015).
64. Walker, B.J. et al. Pilon: an integrated tool for comprehensive microbial variant detection and genome assembly improvement. PLoS One 9, el 12963, doi:10.1371/journal.pone.0112963 (2014).
65. Hunt, M. et al. Circlator: automated circularization of genome assemblies using long sequencing reads. Genome Biol 16, 294, doi:10.1186/sl3059-015-0849-0 (2015).
66. Bankevich, A. et al. SPAdes: a new genome assembly algorithm and its applications to single-cell sequencing. JComputBiol 19, 455-477, doi:10.1089/cmb.2012.0021 (2012).
67. Wick, R. R., Judd, L. M., Gorrie, C. L. & Holt, K. E. Unicycler: Resolving bacterial genome assemblies from short and long sequencing reads. PLoS Comput Biol 13, el005595, doi:10.1371/journal.pcbi. 1005595 (2017).
68. Waterhouse, R. M. etal. BUSCO applications from quality assessments to gene prediction and phylogenomics. MolBiolEvol35, 543-548, doi:10.1093/molbev/msx319 (2017).
69. Gurevich, A., Saveliev, V., Vyahhi, N. & Tesler, G. QUAST: quality assessment tool for genome assemblies. Bioinformatics 29, 1072-1075, doi:10.1093/bioinformatics/btt086 (2013).
70. Delcher, A. L., Salzberg, S. L. & Phillippy, A. M. Using MUMmer to identify similar regions in large sequence sets. Curr Protoc Bioinformatics Chapter 10, Unit 10 13, doi:10.1002/0471250953.bil003s00 (2003).
71. Seemann, T. Prokka: rapid prokaryotic genome annotation. Bioinformatics 30, 2068-2069, doi:10.1093/bioinformatics/btul53 (2014).
72. Francis, M. B. & Carrico, I. S. New frontiers in protein bioconjugation. Curr Opin Chem BiolU, 771-773, doi:10.1016/j.cbpa.2010.11.006 (2010).
73. Koniev, O. & Wagner, A. Developments and recent advancements in the field of endogenous amino acid selective bond forming reactions for bioconjugation. Chem Soc Rev44, 5495-5551, doi:10.1039/c5cs00048c (2015).
74. Macardle, P. J. & Bailey, S. in Cell Biology: A Laboratory Handbooksol. 1 (ed J.E. Celis) Ch. 57, 475-490 (Elsevier, 2006).
75. Tomaru, A. et al. Oligonucleotide-targeting periostin ameliorates pulmonary fibrosis. Gene TherlA, 706-716, doi: 10.1038/gt.2017.80 (2017).
76. Yasui, H. et al. Intratracheal administration of activated protein C inhibits bleomycin-induced lung fibrosis in the mouse. Am J Respir Crit Care Med 163, 1660-1668, doi:10.1164/ajrccm. 163.7.9911068 (2001).
77. Ashcroft, T., Simpson, J. M. & Timbrell, V. Simple method of estimating severity of pulmonary fibrosis on a numerical scale. J Clin Pathol 41, 467-470, doi:10.1136/jcp.41.4.467 (1988).

### [Sequence List]

C:¥Users¥common¥Desktop¥676827¥676827.txt

## Claims

1. An isolated monoclonal antibody molecule or a derivative thereof that specifically binds to corisin.

2. The monoclonal antibody molecule or a derivative thereof according to claim 1, wherein corisin is IVMPESSGNPNAVNPAGYR (SEQ ID NO: 1).

3. The monoclonal antibody molecule or a derivative thereof according to claim 1 or claim 2, wherein PESSGNP (SEQ ID NO: 68) or NPAGY (SEQ ID NO: 69) is an epitope.

4. The monoclonal antibody molecule or a derivative thereof according to claim 1 or claim 2, comprising:
(IA)
(i) an H chain variable region comprising an H chain variable region CDR1 amino acid sequence of SEQ ID NO: 37, an H chain variable region CDR2 amino acid sequence of SEQ ID NO: 38, and an H chain variable region CDR3 amino acid sequence of SEQ ID NO: 39; and
(ii) an L chain variable region comprising an L chain variable region CDR1 amino acid sequence of SEQ ID NO: 41, an L chain variable region CDR2 amino acid sequence of SEQ ID NO: 42, and an L chain variable region CDR3 amino acid sequence of SEQ ID NO: 43;
(IIA)
(i) an H chain variable region comprising an H chain variable region CDR1 amino acid sequence of SEQ ID NO: 70, an H chain variable region CDR2 amino acid sequence of SEQ ID NO: 49, and an H chain variable region CDR3 amino acid sequence of SEQ ID NO: 50; and
(ii) an L chain variable region including an L chain variable region CDR1 amino acid sequence of SEQ ID NO: 52, an L chain variable region CDR2 amino acid sequence of SEQ ID NO: 53, and an L chain variable region CDR3 amino acid sequence of SEQ ID NO: 54;or
(IVA)
(i) an H chain variable region comprising an H chain variable region CDR1 amino acid sequence of SEQ ID NO: 72, an H chain variable region CDR2 amino acid sequence of SEQ ID NO: 73, and an H chain variable region CDR3 amino acid sequence of SEQ ID NO: 74; and
(ii) an L chain variable region comprising an L chain variable region CDR1 amino acid sequence of SEQ ID NO: 76, an L chain variable region CDR2 amino acid sequence of SEQ ID NO: 77, and an L chain variable region CDR3 amino acid sequence of SEQ ID NO: 78.

5. The monoclonal antibody molecule or a derivative thereof according to any one of claims 1 to 3, comprising:
(IB) an H chain variable region comprising an H chain variable region amino acid sequence of SEQ ID NO: 36 or an amino acid sequence that is at least 80% identical thereto, or an H chain variable region comprising at least one framework (FW) region including an amino acid sequence having a substitution, deletion, insertion, or addition of one or several amino acid residues in comparison to the H chain variable region amino acid sequence of SEQ ID NO: 36;
(IIB) an H chain variable region comprising an H chain variable region amino acid sequence of SEQ ID NO: 48 or an amino acid sequence that is at least 80% identical thereto, or an H chain variable region comprising at least one framework (FW) region including an amino acid sequence having a substitution, deletion, insertion, or addition of one or several amino acid residues in comparison to the H chain variable region amino acid sequence of SEQ ID NO: 48; or
(IVB) an H chain variable region comprising an H chain variable region amino acid sequence of SEQ ID NO: 71 or an amino acid sequence that is at least 80% identical thereto, or an H chain variable region comprising at least one framework (FW) region including an amino acid sequence having a substitution, deletion, insertion, or addition of one or several amino acid residues in comparison to the H chain variable region amino acid sequence of SEQ ID NO: 71.

6. The monoclonal antibody molecule or a derivative thereof according to any one of claims 1 to 3, comprising:
(IC) an L chain variable region comprising an L chain variable region amino acid sequence of SEQ ID NO: 40 or an amino acid sequence that is at least 80% identical thereto, or an L chain variable region comprising at least one framework region including an amino acid sequence having a substitution, deletion, insertion, or addition of one or several amino acid residues in comparison to the L chain variable region amino acid sequence of SEQ ID NO: 40;
(IIC) an L chain variable region including an L chain variable region amino acid sequence of SEQ ID NO: 51 or an amino acid sequence that is at least 80% identical thereto, or an L chain variable region including at least one framework region including an amino acid sequence having a substitution, deletion, insertion, or addition of one or several amino acid residues in comparison to the L chain variable region amino acid sequence of SEQ ID NO: 51;
(IIIC) an L chain variable region comprising an L chain variable region amino acid sequence of SEQ ID NO: 44 or an amino acid sequence that is at least 80% identical thereto, or an L chain variable region comprising at least one framework region including an amino acid sequence having a substitution, deletion, insertion, or addition of one or several amino acid residues in comparison to the L chain variable region amino acid sequence of SEQ ID NO: 44; or
(IVC) an L chain variable region comprising an L chain variable region amino acid sequence of SEQ ID NO: 75 or an amino acid sequence that is at least 80% identical thereto, or an L chain variable region comprising at least one framework region including an amino acid sequence having a substitution, deletion, insertion, or addition of one or several amino acid residues in comparison to the L chain variable region amino acid sequence of SEQ ID NO: 75.

7. The monoclonal antibody molecule or a derivative thereof according to any one of claims 1 to 3, comprising any H chain variable region according to claim 5 and any L chain variable region according to claim 6.

8. The monoclonal antibody molecule or a derivative thereof according to claim 7, comprising:
the H chain variable region of (IB) according to claim 5 and the L chain variable region of (IC) according to claim 6;
the H chain variable region of (IIB) according to claim 5 and the L chain variable region of (IIC) according to claim 6; or the H chain variable region of (IVB) according to claim 5 and the L chain variable region of (IVC) according to claim 6.

9. The monoclonal antibody molecule or a derivative thereof according to claim 8, wherein: the H chain variable region of (IB) according to claim 5 is the H chain variable region amino acid sequence of SEQ ID NO: 36, and the L chain variable region of (IC) according to claim 6 is the L chain variable region amino acid sequence of SEQ ID NO: 40; orthe H chain variable region of (IIB) according to claim 5 is the H chain variable region amino acid sequence of SEQ ID NO: 48, and the L chain variable region of (IIC) according to claim 6 is the L chain variable region amino acid sequence of SEQ ID NO: 51; orthe H chain variable region of (IVB) according to claim 5 is the H chain variable region amino acid sequence of SEQ ID NO: 71, and the L chain variable region of (IVC) according to claim 6 is the L chain variable region amino acid sequence of SEQ ID NO: 75.

10. The monoclonal antibody molecule or a derivative thereof according to claim 8, wherein:
the H chain variable region of (IB) according to claim 5 is an H chain variable region comprising an amino acid sequence that is at least 80% identical to the H chain variable region amino acid sequence of SEQ ID NO: 36, and the L chain variable region of (IC) according to claim 6 is an L chain variable region comprising an amino acid sequence that is at least 80% identical to the L chain variable region amino acid sequence of SEQ ID NO: 40; or
the H chain variable region of (IIB) according to claim 5 is an H chain variable region comprising an amino acid sequence that is at least 80% identical to the H chain variable region amino acid sequence of SEQ ID NO: 48, and the L chain variable region of (IIC) according to claim 6 is an L chain variable region comprising an amino acid sequence that is at least 80% identical to the L chain variable region amino acid sequence of SEQ ID NO: 51; or
the H chain variable region of (IVB) according to claim 5 is an H chain variable region comprising an amino acid sequence that is at least 80% identical to the H chain variable region amino acid sequence of SEQ ID NO: 71, and the L chain variable region of (IVC) according to claim 6 is an H chain variable region comprising an amino acid sequence that is at least 80% identical to the H chain variable region amino acid sequence of SEQ ID NO: 75.

11. The monoclonal antibody molecule or a derivative thereof according to claim 8, wherein:
the H chain variable region of (IB) according to claim 5 is an H chain variable region comprising at least one framework (FW) region including an amino acid sequence having a substitution, deletion, insertion, or addition of one or several amino acid residues in comparison to the H chain variable region amino acid sequence of SEQ ID NO: 36, and the L chain variable region of (IC) according to claim 6 is an L chain variable region comprising at least one framework (FW) region including an amino acid sequence having a substitution, deletion, insertion, or addition of one or several amino acid residues in comparison to the L chain variable region amino acid sequence of SEQ ID NO: 40; orthe H chain variable region of (IIB) according to claim 5 is an H chain variable region comprising at least one framework (FW) region including an amino acid sequence having a substitution, deletion, insertion, or addition of one or several amino acid residues in comparison to the H chain variable region amino acid sequence of SEQ ID NO: 48, and the L chain variable region of (IIC) according to claim 6 is an L chain variable region comprising at least one framework (FW) region including an amino acid sequence having a substitution, deletion, insertion, or addition of one or several amino acid residues in comparison to the L chain variable region amino acid sequence of SEQ ID NO: 51; or
the H chain variable region of (IVB) according to claim 5 is an H chain variable region comprising at least one framework (FW) region including an amino acid sequence having a substitution, deletion, insertion, or addition of one or several amino acid residues in comparison to the H chain variable region amino acid sequence of SEQ ID NO: 71, and the L chain variable region of (IVC) according to claim 6 is an L chain variable region comprising at least one framework (FW) region including an amino acid sequence having a substitution, deletion, insertion, or addition of one or several amino acid residues in comparison to the L chain variable region amino acid sequence of SEQ ID NO: 75.

12. A derivative of the antibody molecule according to any of claims 1 to 11, wherein the derivative is Fab, F(ab')₂, Fv, or a single chain Fv fragment (scFv).

13. The antibody molecule or a derivative thereof according to any one of claims 1 to 12, wherein the antibody molecule or a derivative thereof neutralizes and/or inhibits corisin-induced apoptosis.

14. A pharmaceutical composition for treating or preventing acute lung injury or fibrosis, wherein the pharmaceutical composition contains an effective dose of the monoclonal antibody molecule or a derivative thereof according to any of claims 1 to 13.

15. The pharmaceutical composition according to claim 14, wherein the acute lung injury is fatal pneumonia that causes acute respiratory distress syndrome (ARDS), reduces lung function, and requires respiratory care.

16. The pharmaceutical composition according to claim 14, wherein the fibrosis is selected from a group composed of idiopathic pulmonary fibrosis (IPF), liver cirrhosis, renal fibrosis, cystic fibrosis, myelofibrosis, and fibrous disease of the breast.

17. A vaccine composition for preventing acute lung injury or fibrosis, comprising as an active ingredient a corisin mutant having one or several mutations in the sequence of corisin having the amino acid sequence: IVMPESSGNPNAVNPAGYR (SEQ ID NO: 1), wherein the mutant does not exhibit apoptotic activity.

18. Use of corisin as a biomarker to determine the stage of progression of acute lung injury or fibrosis.
